# EUROPEAN PATENT SPECIFICATION

(11) **EP 1 499 733 B1**
(45) Date of publication and mention of the grant of the patent: **10.03.2010**
(21) Application number: 03724363.1
(22) Date of filing: 22.04.2003
(51) Int. Cl.: C12N 15/80

(54) **METHODS FOR PREPARING VARIANTS OF A DNA SEQUENCE IN FILAMENTOUS FUNGI**
VERFAHREN ZUR HERSTELLUNG VON VARIANTEN EINER DNA-SEQUENZ IN FILAMENTÖSEN PILZEN
PROCEDES DE PREPARATION DE VARIANTS D'UNE SEQUENCE D'ADN DANS DES CHAMPIGNONS FILAMENTEUX

(30) Priority: 22.04.2002 US 374688 P
(43) Date of publication of application: 26.01.2005
(73) Proprietor: Novozymes, Inc., Davis, CA 95616 (US)
(72) Inventor: BRODY, Howard, Davis, CA 95616 (US); OTANI, Suzanne, Elk Grove, CA 95758 (US); CHERRY, Joel, Davis, CA 95616 (US)
(74) Representative: Kofoed, Gertrud Sonne
(86) International application number: PCT/US2003/013577
(87) International publication number: WO 2003/089648

(56) References cited:
- WO-A-00/55346
- WO-A-97/07205
- GEMS D H ET AL: "CO-TRANSFORMATION WITH AUTONOMOUSLY-REPLICATING HELPER PLASMIDS FACILITES GENE CLONING FROM AN ASPERGILLUS NIDULANS GENE LIBRARY" CURRENT GENETICS, NEW YORK, NY, US, vol. 24, 1993, pages 520-524, XP000864503 ISSN: 0172-8083
- VERDOES J C ET AL: "CHARACTERISATION OF AN EFFICIENT GENE CLONING STRATEGY FOR ASPERGILLUS NIGER BASED ON AN AUTONOMOUSLY REPLICATING PLASMID: CLONING OF THE NICB GENE OF A. NIGER" GENE, ELSEVIER BIOMEDICAL PRESS. AMSTERDAM, NL, vol. 146, 1994, pages 159-165, XP001207145 ISSN: 0378-1119
- POMPON D ET AL: "PROTEIN ENGINEERING BY CDNA RECOMBINATION IN YEASTS: SHUFFLING OF MAMMALIAN CYTOCHROME P-450 FUNCTIONS" GENE, ELSEVIER BIOMEDICAL PRESS. AMSTERDAM, NL, vol. 83, no. 1, 15 November 1989 (1989-11-15), pages 15-24, XP000054542 ISSN: 0378-1119
- KOSTRUB C.F. ET AL.: 'Use of gap repair in fission yeast to obtain novel alleles of specific genes' NUCLEIC ACIDS RESEARCH vol. 26, no. 10, September 1998, pages 4783 - 4784, XP002968563
- VAN HEEMST D. ET AL.: 'Cloning, sequencing, disruption and phenotypic analysis of uvsC, an aspergillus nidulans homologue of yeast RAD51' vol. 254, January 1997, pages 654 - 664, XP002968564

### SEQUENCE LISTING

<110> Novozymes Biotech, Inc.
<120> Method For Preparing Polypeptide variants
<130> 10301.204-WO
<150> US 60/374,688
   <151> 2002-04-22
<160> 58
<170> PatentIn version 3.2
<210> 1
   <211> 2032
   <212> DNA
   <213> A. oryzae
<400> 1
   ttctcggact ggatgaagca agatgcaacg aagcaagctc atgatctgac attagggccc     60
   ctctcatgtt tccttgcatt attgttccaa tatagcgttg cgtaattgta ggcttacctc    120
   ttaaggcagc agcctgatgt gcctggaaca cgtgacctcg tgacagcctc gacgcgtcca    180
   gaacctcaac aaattgttta tcgccgtaga gtcatacgcc attttgccac atcgaccgac    240
   ttacgaattt taaataagac ttctattgtt tccaaactgg tgactacaaa gcagcctttg    300
   gggtattcgt ccgataagaa acaatcctag cgaagaattc atccacagtt acaatagaac    360
   gcgtcctgtc gcgtcgagta ggctgtgtgc aagaccagta gcagttgctt gattactctt    420
   ggggctggct agggagacac tttacttgcc tacctgattg aaaatgacgg cggatatgga    480
   tactcagaat gaatacgatg atagtggact tcccgggcct ggagcgccca cgccactttc    540
   agctttagaa gtgaggaatc tctactgtcc acacaataca atattacaag cttgatttag    600
   tagtaaaagc ccctttcagt gacttgtact gaccattgac tcgatagggt gttgcgggat    660
   taacgggaag agatatcaaa ttgtttgtcg atgccggcta tcacactgtc gaatcaattg    720
   cgtatacgta cgtctttcct cttgaatgtt taaacgaact tgctgcagca ccaaacttgg    780
   agagcttaag gagatgctga cattggcatt ggtggtacat catagaccga aacgtttact    840
   ggaacaaatc aaaggtatat cggagcagaa ggccaccaag gttttggttg aaggttagtc    900
   acctcaactc atggagctca accgtctgta aggattcgtg ctgattatac ctgataaata    960
   gctgccaagc ttgtgccaat gggtttcacg actgcaacag aaatgcatgc acgtcgaagt   1020
   gagctcatat cgatcacgac aggatccaag caactagata ctctcctagg cggtggtata   1080
   gaaacgggat ctattaccga gatattcgga gaattcagga caggtaaaag tcaaatttgc   1140
   catacgcttg cagtgacttg ccagctgcca ttcgacatgg gtggtgggga agggaagtgt   1200
   ctttatattg atactgaagg gacatttcga ccggtccgtc tgttggcagt tgctcaaaga   1260
   tacggacttg ttggcgaaga ggtactcgat aatgtggcct atgcccgcgc ttataactcg   1320
   gatcaccagc tccagctgct gaaccaggcg tctcaaatga tgtgcgaaac tcgtttctca   1380
   cttcttgtcg tcgactctgc tacagcgcta tatcggacag attttaacgg ccgtggtgaa   1440
   ctatcgactc gacaaacaca tctcgctaaa ttcatgcgta ccttgcagcg cttggcggat   1500
   gaatttggta ttgccgtcgt catcaccaac caggtcgtcg cccaggtcga cggcggtccg   1560
   agtgcaatgt tcaacccaga ccccaagaag ccaatcggtg gaaacattat cgcacacgcc   1620
   agcacgacca ggctgagtct gaaaaagggg agaggagaga cccgagtgtg caagatctat   1680
   gacagtccct gtctgcccga gagtgactgt ctttttgcta tcaatgaaga tggtattggg   1740
   gatcctagcc ccaaggactt ggaaaacaac tgaggagcga tgaagctgta ttaattactt   1800
   acgataccac gatcggtata tgattttact tggtttgttc tttagtacat attgtttagt   1860
   atcttgattt tgatagcata cggtgtttgt ggtattgtgc tagattttat gtgctaattg   1920
   agataaaagt tgatcaataa aaaaagaact atgacttgta tatacaaaga acgtatggtc   1980
   ttctaataat atctatttcg aacgatttgc tcttctgtcc ttccatcaaa tt           2032
<210> 2
   <211> 315
   <212> PRT
   <213> A. oryzae
<400> 2
   Met Thr Ala Asp Met Asp Thr Gln Asn Glu Tyr Asp Asp Ser Gly Leu
   1               5                   10                  15
   Pro Gly Pro Gly Ala Pro Thr Pro Leu Ser Ala Leu Glu Gly Val Ala
               20                  25                  30
   Gly Leu Thr Gly Arg Asp Ile Lys Leu Phe Val Asp Ala Gly Tyr His
           35                  40                  45
   Thr Val Glu Ser Ile Ala Tyr Thr Pro Lys Arg Leu Leu Glu Gln Ile
       50                  55                  60
   Lys Gly Ile Ser Glu Gln Lys Ala Thr Lys Val Leu Val Glu Ala Ala
   65                  70                  75                  80
   Lys Leu Val Pro Met Gly Phe Thr Thr Ala Thr Glu Met His Ala Arg
                   85                  90                  95
   Arg Ser Glu Leu Ile Ser Ile Thr Thr Gly Ser Lys Gln Leu Asp Thr
   100 105 110
   Leu Leu Gly Gly Gly Ile Glu Thr Gly Ser Ile Thr Glu Ile Phe Gly
   115 120 125
   Glu Phe Arg Thr Gly Lys Ser Gln Ile Cys His Thr Leu Ala Val Thr
   130 135 140
   Cys Gln Leu Pro Phe Asp Met Gly Gly Gly Glu Gly Lys Cys Leu Tyr
   145 150 155 160
   Ile Asp Thr Glu Gly Thr Phe Arg Pro Val Arg Leu Leu Ala Val Ala
   165 170 175
   Gln Arg Tyr Gly Leu Val Gly Glu Glu Val Leu Asp Asn Val Ala Tyr
   180 185 190
   Ala Arg Ala Tyr Asn Ser Asp His Gln Leu Gln Leu Leu Asn Gln Ala
   195 200 205
   Ser Gln Met Met Cys Glu Thr Arg Phe Ser Leu Leu Val Val Asp Ser
   210 215 220
   Ala Glu Phe Gly Ile Ala Val Val Ile Thr Asn Gln Val Val Ala Gln
   225 230 235 240
   Val Asp Gly Gly Pro Ser Ala Met Phe Asn Pro Asp Pro Lys Lys Pro
   245 250 255
   Ile Gly Gly Asn Ile Ile Ala His Ala Ser Thr Thr Arg Leu Ser Leu
   260 265 270
   Lys Lys Gly Arg Gly Glu Thr Arg Val Cys Lys Ile Tyr Asp Ser Pro
   275 280 285
   Cys Leu Pro Glu Ser Asp Cys Leu Phe Ala Ile Asn Glu Asp Gly Ile
   290 295 300
   Gly Asp Pro Ser Pro Lys Asp Leu Glu Asn Asn
   305 310 315
<210> 3
   <211> 2500
   <212> DNA
   <213> A. oryzae
<400> 3
   gtggtatcgc gactgtcgga ttccgtcacg cgtcgagcag ctcagttgac ctctgtctat 60
   cttcattacc ccccactgtg tacgccacgc gcatcggtgt ataccaatga taccctttct 120
   taactagtgt aacatttata tatcttttaa taatgcccgc gtgagccacc ccagtgtgct 180
   acgctaccgc tgctattaat cccgatctaa gctaacgcgt cttagtgttg gcgaccaaca 240
   tcgaggcaat ccgacatgca tcatgatgcc caacacgaca gacactacat cagcaaaccc 300
   ctttgaggaa cgtcctcgcc gcatgaatga gtatacagct cgggagatcg ccacactgca 360
   agcacggctc gataagaaat taggccccga atacatctcc tctaggccag gcgccgcggg 420
   acagagagtc cattatctgg ctgcagacaa atgcattaac ctagccaacg aggtctttgg 480
   tttcaatggg tggtcaagtt cgatacaaca aattcagatc gatttcgtat gttctattga 540
   taggagcata tctatgttgc gtgtgcccga gatcggacag ctgataaatc cctcgttgta 600
   acaggttgac gagagcccaa atacggggaa gattagcttg ggcttgtcag ttgtagtgag 660
   ggtgactcta aaggatggga cctaccatga ggtatacttt tgcgtgaatg atatgctccg 720
   atgtgcccaa acgctaacca ctttggacga cgttaggata tcgggtacgg ccacattgag 780
   aactgtaaag ggaaagctgc ggctttcgaa aaagcaaaga aagaaggaac cacagatgcc 840
   ctaaagcgta cattgaggaa cttcggcaac gtcctgggca attgcattta tgataaggat 900
   tacgtatcga aagtgacgaa agtgaagaca gcgcctgtat gtgtttctac ggcatattac 960
   tcgactagac tcaggtacta acatgctccc aggcaagatg ggacgtggat gaccttcacc 1020
   gacaccctga tttcgcaccc atcaagaaag aaccagttca acagaagccg atgcaggagg 1080
   atgatgatct ccctcctcgc ccgactgatg cgggaaagaa caacagtaac tcagccgata 1140
   ctgcctttga tgctgatgga gagttcggaa gtacgtaacg ataaatcaag ctaccttgca 1200
   tgcatttcac taacgatcgc aggtgattta tttgacgaag cggactttgg agtcgccgca 1260
   actggaaacc cagatgaaat agtaatagac ccagataccc aaagatttca gcagccacca 1320
   acacctctga accgtcaaaa tggcccagcc ccgtacaggg gccctcaaca gcataacccc 1380
   ttagccgctg caagacccca ttccgccatt gccacaccat ccaaaccaga aagaccgccg 1440
   aaccaggcag ctgccgctag acagatacca cctcctgctc tgaatggcag accaaaccct 1500
   gctgcacccg cccacaaccc gcaacacaac cttccaagcg gaagaatacc accagctcaa 1560
   ccaagaccta atcaagacac agccatgccc ggtgcaagtg gtcagatgcc catcaaacgg 1620
   gaacaagttc ctaatcccaa cgaccccgga acccaggaca tgctcccacc aggaagctca 1680
   ccgatgccat ctgcctcatt cttctcagct cgagcagtcg atctcctacg tgacaaccca 1740
   caagcaaacg cagccccggc attcgacccc catgcagaaa gcccatccat ccgcaagaca 1800
   gctggcgtcg accacagtaa gagcgtcccg atttccaaac ccatgcttgc cagcgtatcc 1860
   cccgccgcca acaatacccg tgacttcgtc aacccttctc aagatatgca tcggaaaatc   1920
   ggcgctccta gcggaatagg cagtcccatg aatcgaggcc agacaacctc atcttaccgc   1980
   ccattaacaa gaccgaacat cgaccccaag aatgctgtga atactacagc tgcaaaccgg   2040
   ggcgtcgggc cacaaaatct aaatgggaaa cgacctcccc taagtgatgt gactaatgca   2100
   tccactttag gcggcagcgg gcctgctccc attggtggtg cgatagaccc taagaggccg   2160
   aaaatcaacg acgggcctct tccacaccaa cagcaacaac atccgcaata ggactcctag   2220
   cggatttagt atagttacac ggcaacataa ataatagcca tgcttacggg gagatcgtcc   2280
   actcattgca tcgttagagg tcatctcatc ggtagttcaa gacatggcgt tcaggattgg   2340
   ggtacgggta aaggagctcc gggttaaatg taatgaatgt tcttgatgaa tgttattttt   2400
   gttatattct cttcactcca gcttaaagca tccaagacgt gcctccttag gcagttgtgt   2460
   gactggattg tctacggaca ctacacagct tgtactatac                         2500
<210> 4
   <211> 565
   <212> PRT
   <213> A. oryzae
<400> 4
   Met Met Pro Asn Thr Thr Asp Thr Thr Ser Ala Asn Pro Phe Glu Glu
   1               5                   10                  15
   Arg Pro Arg Arg Met Asn Glu Tyr Thr Ala Arg Glu Ile Ala Thr Leu
               20                  25                  30
   Gln Ala Arg Leu Asp Lys Lys Leu Gly Pro Glu Tyr Ile Ser Ser Arg
           35                  40                  45
   Pro Gly Ala Ala Gly Gln Arg Val His Tyr Leu Ala Ala Asp Lys Cys
       50                  55                  60
   Ile Asn Leu Ala Asn Glu Val Phe Gly Phe Asn Gly Trp Ser Ser Ser
   65                  70                  75                  80
   Ile Gln Gln Ile Gln Ile Asp Phe Val Asp Glu Ser Pro Asn Thr Gly
                   85                  90                  95
   Lys Ile Ser Leu Gly Leu Ser Val Val Val Arg Val Thr Leu Lys Asp
               100                 105                 110
   Gly Thr Tyr His Glu Asp Ile Gly Tyr Gly His Ile Glu Asn Cys Lys
           115                 120                 125
   Gly Lys Ala Ala Ala Phe Glu Lys Ala Lys Lys Glu Gly Thr Thr Asp
       130                 135                 140
   Ala Leu Lys Arg Thr Leu Arg Asn Phe Gly Asn Val Leu Gly Asn Cys
   145                 150                 155                 160
   Ile Tyr Asp Lys Asp Tyr Val Ser Lys Val Thr Lys Val Lys Thr Ala
                   165                 170                 175
   Pro Ala Arg Trp Asp Val Asp Asp Leu His Arg His Pro Asp Phe Ala
               180                 185                 190
   Pro Ile Lys Lys Glu Pro Val Gln Gln Lys Pro Met Gln Glu Asp Asp
           195                 200                 205
   Asp Leu Pro Pro Arg Pro Thr Asp Ala Gly Lys Asn Asn Ser Asn Ser
       210                 215                 220
   Ala Asp Thr Ala Phe Asp Ala Asp Gly Glu Phe Gly Ser Asp Leu Phe
   225                 230                 235                 240
   Asp Glu Ala Asp Phe Gly Val Ala Ala Thr Gly Asn Pro Asp Glu Ile
                   245                 250                 255
   Val Ile Asp Pro Asp Thr Gln Arg Phe Gln Gln Pro Pro Thr Pro Leu
               260                 265                 270
   Asn Arg Gln Asn Gly Pro Ala Pro Tyr Arg Gly Pro Gln Gln His Asn
           275                 280                 285
   Pro Leu Ala Ala Ala Arg Pro His Ser Ala Ile Ala Thr Pro Ser Lys
       290                 295                 300
   Pro Glu Arg Pro Pro Asn Gln Ala Ala Ala Ala Arg Gln Ile Pro Pro
   305                 310                 315                 320
   Pro Ala Leu Asn Gly Arg Pro Asn Pro Ala Ala Pro Ala His Asn Pro
                   325                 330                 335
   Gln His Asn Leu Pro Ser Gly Arg Ile Pro Pro Ala Gln Pro Arg Pro
               340                 345                 350
   Asn Gln Asp Thr Ala Met Pro Gly Ala Ser Gly Gln Met Pro Ile Lys
           355                 360                 365
   Arg Glu Gln Val Pro Asn Pro Asn Asp Pro Gly Thr Gln Asp Met Leu
   370 375 380
   Pro Pro Gly Ser Ser Pro Met Pro Ser Ala Ser Phe Phe Ser Ala Arg
   385 390 395 400
   Ala Val Asp Leu Leu Arg Asp Asn Pro Gln Ala Asn Ala Ala Pro Ala
   405 410 415
   Phe Asp Pro His Ala Glu Ser Pro Ser Ile Arg Lys Thr Ala Gly Val
   420 425 430
   Asp His Ser Lys Ser Val Pro Ile Ser Lys Pro Met Leu Ala Ser Val
   435 440 445
   Ser Pro Ala Ala Asn Asn Thr Arg Asp Phe Val Asn Pro Ser Gln Asp
   450 455 460
   Met His Arg Lys Ile Gly Ala Pro Ser Gly Ile Gly Ser Pro Met Asn
   465 470 475 480
   Arg Gly Gln Thr Thr Ser Ser Tyr Arg Pro Leu Thr Arg Pro Asn Ile
   485 490 495
   Asp Pro Lys Asn Ala Val Asn Thr Thr Ala Ala Asn Arg Gly Val Gly
   500 505 510
   Pro Gln Asn Leu Asn Gly Lys Arg Pro Pro Leu Ser Asp Val Thr Asn
   515 520 525
   Ala Ser Thr Leu Gly Gly Ser Gly Pro Ala Pro Ile Gly Gly Ala Ile
   530 535 540
   Asp Pro Lys Arg Pro Lys Ile Asn Asp Gly Pro Leu Pro His Gln Gln
   545 550 555 560
   Gln Gln His Pro Gln
   565
<210> 5
   <211> 2981
   <212> DNA
   <213> A. oryzae
<220>
   <221> misc_feature
   <222> (2932)..(2932)
   <223> n= a,c,g, or t
<400> 5
   gacagcgtga tactttggtg tttagacggc cacagggaaa cgcgccaaga tgtggcaacg 60
   cgttgttcat gactctatgg aactgacatt gactgccagg catcagccca cctattactg 120
   cgtgaaatag aaaggctttc tagatagcac cgctaccttt aatgtaagga aaatattaat 180
   tctgttctct catgctataa atcgctaact tctcaaggta tcgaccacga ccgagtgtaa 240
   gggaagatgg cggaagcaca ccgtcatcca aaaacctcca aaccccgtcc tcaaagtcca 300
   ttgaccgcct atcaaaacca ttcaaatgcc ctggatctgc tacacccaca cggacatcgg 360
   ataaacctgc gaggaaacga agaaaggtga attatgcggg ggctgatgaa actgtggacg 420
   ataatagtga aaagccatac accaacgagg aacgtttagc actcgccacc agagatgtga 480
   acaggttccc tgtgttcaaa cctaaagata aagagacaac cttcaaacaa cgattcaaga 540
   tacctttaat caacaaggcg gttgacagct acaatggcgc tagggcggcg ccaaccttgg 600
   ggatgcgaca aggtgctaca tttgtcgtga aacctctaca tgatcctagc ggagaatttg 660
   cgatagtgct gtatgatccg actgtcgatg atgccgatga gaacagtgaa acgaagttgc 720
   cggaagatgg aaaacccgaa gaacaacaac ccaagttgga cgctcccctt gtacacaaga 780
   gcttagcaga catacttggt cttaagaaga aagttgaaac tggtccaagg gttccagtcg 840
   tgatagaccc aaggttggca aaggttctac gcccacatca aattgaaggt gtaaaggtaa 900
   catgactgtt ccaatcaatg ctctcctgcg atattagact aacgataact gttttctagt 960
   ttttataccg ctgcacaacc ggaatggtcg ataagaacgc acacggctgt ataatggcgg 1020
   atggaatggg actagggaaa acagtatggc cgacagaccc ttcaaaacac taaccccggc 1080
   tgacggcgat agcttcaatg catctcattg atgtggacat tgctcaagca gtctcctgag 1140
   gcaggcaaga cccttatcca gaagtgtatc attgcttgtc cttcaagttt ggttggcaac 1200
   tgggccaatg agctaggtag gtagtgcgcc ctggatgttt taacacctgc taacaaccac 1260
   agtgaaatgg ctaggtaaag atgccatcac tccttttgcg gtggatggca aagcttcgaa 1320
   gacagaactc acatctcaga tcaagcaatg ggctattgct tccggtcgcg ccgttgtgag 1380
   acctgtgctc attgtgtcct acgaaacgct caggatgtat gttgaagcat tgaaggatag 1440
   ccccataggg ctacttcttt gcgatgaagg tcatcggctt aaaaataagg atagtttaac 1500
   atggactgca ctcaacagtc tgaatgtgca acgtcgtgtt atcttgtcag gaacccctat 1560
   tcaaaatgat ctttcggaat atttcgccct gctcaacttc gccaacccag atttattagg 1620
   gtcgcagaat gaatttcgga aaaggttcga attgcctatc ctcagaggaa gggatgccgc 1680
   aggatcggac gaagacaaaa agaaaggcga tgaatgtcta gctgagctct caaccatcgt 1740
   caacaaattc attatccgcc gaacaaatga tatattgacg aaatacttgc cagtcaagta 1800
   tgagcatgtt gtcttttgca atttgtctca attccaactc gacctttata accacttcat 1860
   tcagagccca gaaattagga gcttgctcag gggcaaagga agccagccgc ttaaggcaat 1920
   tggccttttg aaaaagcttt gcaaccatcc tgatctactt aacctttcca ccgaccttcc 1980
   aggatgcgaa tttgcatttc cagaagatta cgtgccacct gaggcaagag ggcgtgaccg 2040
   cgatatcaag tcttggtact cggggaaaat gatggttttg gatcgaatgc tagcacgtat 2100
   acgccaggac acaaatgaca aaattgttct cattagtaat tacacccaga cacttgacct 2160
   gttcgaaaag ctatgcagat cgagggggta tggctcgttg agactggacg gtactatgaa 2220
   tgtgaataag cggcaaaagc tcgtcgacaa attcaacaac cctgacgggg aagaatttgt 2280
   atttctcctc agcagcaagg ccggtggatg tggcctcaat ctaataggcg ccaatcgtct 2340
   cgtgctgttt gacccagatt ggaacccagc tgctgatcaa caagcattgg cacgagtttg 2400
   gcgtgatggt cagaagaaag actgtttcgt gtaccgattt atcgcgaccg gctcaattga 2460
   ggagaagatc ttccaacggc agtctcataa gcaatcattg tcctcatgcg ttgtggattc 2520
   agcggaagat gttgagcggc atttttcttt ggagtctctc cgcgaactat tccaattcaa 2580
   accggaaacc cgaagtgaca cacatgacac cttcaagtgc aagagatgca gaccggatgg 2640
   agcgcaattc atcaaggcgc aggctatgtt gtatggcgat accagcacct ggaatcactt 2700
   tgttaatgat ggcgagaagg gtgcccttag caagatccag gacctgctga tacgacagga 2760
   gaccggggag agagatgtgt ctgcggtatt ccagtatata agtcactgat ctaattctta 2820
   caagcgtcgt gttttacact gtctatatgt tcaaagcagt tgatgctacg gcaatcatga 2880
   gttggcacaa ttctgggctg tcagtgcagc tatttacatt tggtagctag gnatatcatg 2940
   cattcatgct tttgctatca taggctacat taggtctatg g 2981
<210> 6
   <211> 811
   <212> PRT
   <213> A. oryzae
<400> 6
   Met Tyr Arg Pro Arg Pro Ser Val Arg Glu Asp Gly Gly Ser Thr Pro
   1               5                   10                  15
   Ser Ser Lys Asn Leu Gln Thr Pro Ser Ser Lys Ser Ile Asp Arg Leu
               20                  25                  30
   Ser Lys Pro Phe Lys Cys Pro Gly Ser Ala Thr Pro Thr Arg Thr Ser
           35                  40                  45
   Asp Lys Pro Ala Arg Lys Arg Arg Lys Val Asn Tyr Ala Gly Ala Asp
   50 55 60
   Glu Thr Val Asp Asp Asn Ser Glu Lys Pro Tyr Thr Asn Glu Glu Arg
   65 70 75 80
   Leu Ala Leu Ala Thr Arg Asp Val Asn Arg Phe Pro Val Phe Lys Pro
   85 90 95
   Lys Asp Lys Glu Thr Thr Phe Lys Gln Arg Phe Lys Ile Pro Leu Ile
   100 105 110
   Asn Lys Ala Val Asp Ser Tyr Asn Gly Ala Arg Ala Ala Pro Thr Leu
   115 120 125
   Gly Met Arg Gln Gly Ala Thr Phe Val Val Lys Pro Leu His Asp Pro
   130 135 140
   Ser Gly Glu Phe Ala Ile Val Leu Tyr Asp Pro Thr Val Asp Asp Ala
   145 150 155 160
   Asp Glu Asn Ser Glu Thr Lys Leu Pro Glu Asp Gly Lys Pro Glu Glu
   165 170 175
   Gln Gln Pro Lys Leu Asp Ala Pro Leu Val His Lys Ser Leu Ala Asp
   180 185 190
   Ile Leu Gly Leu Lys Lys Lys Val Glu Thr Gly Pro Arg Val Pro Val
   195 200 205
   Val Ile Asp Pro Arg Leu Ala Lys Val Leu Arg Pro His Gln Ile Glu
   210 215 220
   Gly Val Lys Phe Leu Tyr Arg Cys Thr Thr Gly Met Val Asp Lys Asn
   225 230 235 240
   Ala His Gly Cys Ile Met Ala Asp Gly Met Gly Leu Gly Lys Thr Leu
   245 250 255
   Gln Cys Ile Ser Leu Met Trp Thr Leu Leu Lys Gln Ser Pro Glu Ala
   260 265 270
   Gly Lys Thr Leu Ile Gln Lys Cys Ile Ile Ala Cys Pro Ser Ser Leu
   275 280 285
   Val Gly Asn Trp Ala Asn Glu Leu Val Lys Trp Leu Gly Lys Asp Ala
   290 295 300
   Ile Thr Pro Phe Ala Val Asp Gly Lys Ala Ser Lys Thr Glu Leu Thr
   305 310 315 320
   Ser Gln Ile Lys Gln Trp Ala Ile Ala Ser Gly Arg Ala Val Val Arg
   325 330 335
   Pro Val Leu Ile Val Ser Tyr Glu Thr Leu Arg Met Tyr Val Glu Ala
   340 345 350
   Leu Lys Asp Ser Pro Ile Gly Leu Leu Leu Cys Asp Glu Gly His Arg
   355 360 365
   Leu Lys Asn Lys Asp Ser Leu Thr Trp Thr Ala Leu Asn Ser Leu Asn
   370 375 380
   Val Gln Arg Arg Val Ile Leu Ser Gly Thr Pro Ile Gln Asn Asp Leu
   385 390 395 400
   Ser Glu Tyr Phe Ala Leu Leu Asn Phe Ala Asn Pro Asp Leu Leu Gly
   405 410 415
   Ser Gln Asn Glu Phe Arg Lys Arg Phe Glu Leu Pro Ile Leu Arg Gly
   420 425 430
   Arg Asp Ala Ala Gly Ser Asp Glu Asp Lys Lys Lys Gly Asp Glu Cys
   435 440 445
   Leu Ala Glu Leu Ser Thr Ile Val Asn Lys Phe Ile Ile Arg Arg Thr
   450 455 460
   Asn Asp Ile Leu Thr Lys Tyr Leu Pro Val Lys Tyr Glu His Val Val
   465 470 475 480
   Phe Cys Asn Leu Ser Gln Phe Gln Leu Asp Leu Tyr Asn His Phe Ile
   485 490 495
   Gln Ser Pro Glu Ile Arg Ser Leu Leu Arg Gly Lys Gly Ser Gln Pro
   500 505 510
   Leu Lys Ala Ile Gly Leu Leu Lys Lys Leu Cys Asn His Pro Asp Leu
   515 520 525
   Leu Asn Leu Ser Thr Asp Leu Pro Gly Cys Glu Phe Ala Phe Pro Glu
       530                 535                 540
   Asp Tyr Val Pro Pro Glu Ala Arg Gly Arg Asp Arg Asp Ile Lys Ser
   545                 550                 555                 560
   Trp Tyr Ser Gly Lys Met Met Val Leu Asp Arg Met Leu Ala Arg Ile
                   565                 570                 575
   Arg Gln Asp Thr Asn Asp Lys Ile Val Leu Ile Ser Asn Tyr Thr Gln
               580                 585                 590
   Thr Leu Asp Leu Phe Glu Lys Leu Cys Arg Ser Arg Gly Tyr Gly Ser
           595                 600                 605
   Leu Arg Leu Asp Gly Thr Met Asn Val Asn Lys Arg Gln Lys Leu Val
       610                 615                 620
   Asp Lys Phe Asn Asn Pro Asp Gly Glu Glu Phe Val Phe Leu Leu Ser
   625                 630                 635                 640
   Ser Lys Ala Gly Gly Cys Gly Leu Asn Leu Ile Gly Ala Asn Arg Leu
                   645                 650                 655
   Val Leu Phe Asp Pro Asp Trp Asn Pro Ala Ala Asp Gln Gln Ala Leu
               660                 665                 670
   Ala Arg Val Trp Arg Asp Gly Gln Lys Lys Asp Cys Phe Val Tyr Arg
           675                 680                 685
   Phe Ile Ala Thr Gly Ser Ile Glu Glu Lys Ile Phe Gln Arg Gln Ser
       690                 695                 700
   His Lys Gln Ser Leu Ser Ser Cys Val Val Asp Ser Ala Glu Asp Val
   705                 710                 715                 720
   Glu Arg His Phe Ser Leu Glu Ser Leu Arg Glu Leu Phe Gln Phe Lys
                   725                 730                 735
   Pro Glu Thr Arg Ser Asp Thr His Asp Thr Phe Lys Cys Lys Arg Cys
               740                 745                 750
   Arg Pro Asp Gly Ala Gln Phe Ile Lys Ala Gln Ala Met Leu Tyr Gly
           755                 760                 765
   Asp Thr Ser Thr Trp Asn His Phe Val Asn Asp Gly Glu Lys Gly Ala
   770 775 780
   Leu Ser Lys Ile Gln Asp Leu Leu Ile Arg Gln Glu Thr Gly Glu Arg
   785 790 795 800
   Asp Val Ser Ala Val Phe Gln Tyr Ile Ser His
   805 810
<210> 7
   <211> 20
   <212> DNA
   <213> A. oryzae
<220>
   <221> misc_feature
   <222> (3)..(3)
   <223> y= c or t
<220>
   <221> misc_feature
   <222> (6)..(6)
   <223> y= c or t
<220>
   <221> misc_feature
   <222> (9)..(9)
   <223> n= inosine
<220>
   <221> misc_feature
   <222> (12)..(12)
   <223> n= inosine
<220>
   <221> misc_feature
   <222> (15)..(15)
   <223> y= c or t
<220>
   <221> misc_feature
   <222> (18)..(18)
   <223> n= a,c,g or t
<220>
   <221> misc_feature
   <222> (19)..(19)
   <223> m= a or c
<400> 7
   gayaaygtng cntaygcnmg 20
<210> 8
   <211> 20
   <212> DNA
   <213> A. oryzae
<220>
   <221> misc_feature
   <222> (3)..(3)
   <223> n= inosine
<220>
   <221> misc_feature
   <222> (6)..(6)
   <223> r= a or g
<220>
   <221> misc_feature
   <222> (9)..(9)
   <223> n= a,c, g or t
<220>
   <221> misc_feature
   <222> (12)..(12)
   <223> r= a or g
<220>
   <221> misc_feature
   <222> (15)..(15)
   <223> r= a or g
<400> 8
   ttnggrtcng grttraacat 20
<210> 9
   <211> 20
   <212> DNA
   <213> A. oryzae
<220>
   <221> misc_feature
   <222> (3)..(3)
   <223> y= c or t
<220>
   <221> misc_feature
   <222> (6)..(6)
   <223> n= inosine
<220>
   <221> misc_feature
   <222> (9)..(9)
   <223> n= inosine
<220>
   <221> misc_feature
   <222> (12)..(12)
   <223> n= a,c,g or t
<220>
   <221> misc_feature
   <222> (is)..(15)
   <223> y= c or t
<220>
   <221> misc_feature
   <222> (18)..(18)
   <223> r= a or g
<400> 9
   acytgngcna cnacytgrtt 20
<210> 10
   <211> 29
   <212> DNA
   <213> A. oryzae
<220>
   <221> misc_feature
   <222> (20)..(20)
   <223> y= c or t
<220>
   <221> misc_feature
   <222> (23)..(23)
   <223> y= c or t
<220>
   <221> misc_feature
   <222> (26)..(26)
   <223> n= a,c,g or t
<400> 10
   cgaacgaagt cttcggttty aayggntgg 29
<210> 11
   <211> 30
   <212> DNA
   <213> A. oryzae
<220>
   <221> misc_feature
   <222> (19)..(19)
   <223> n= a,c,g, or t
<220>
   <221> misc_feature
   <222> (22)..(22)
   <223> y= c or t
<220>
   <221> misc_feature
   <222> (25)..(25)
   <223> y= c or t
<220>
   <221> misc_feature
   <222> (28)..(28)
   <223> y= c or t
<400> 11
   cttcatgccg tcggtagtnc cytcyttytt 30
<210> 12
   <211> 42
   <212> DNA
   <213> A. oryzae
<400> 12
   tatcaattct taattaagga tccaagcttg tttaaacaat tc 42
<210> 13
   <211> 40
   <212> DNA
   <213> A. oryzae
<400> 13
   agttaacaat taattcctag gttcgaacaa atttgttaac 40
<210> 14
   <211> 33
   <212> DNA
   <213> A. oryzae
<400> 14
   gatacatgtt atggagatgt tctatcacac aag 33
<210> 15
   <211> 29
   <212> DNA
   <213> A. oryzae
<400> 15
   caggatcctg cagtattgac tactatggt 29
<210> 16
   <211> 32
   <212> DNA
   <213> A. oryzae
<400> 16
   ctgtttaaac tgcagggagg aactgaaaaa gg 32
<210> 17
   <211> 31
   <212> DNA
   <213> A. oryzae
<400> 17
   gttaagcttg cgaaacgcaa ataatgtgtt g 31
<210> 18
   <211> 35
   <212> DNA
   <213> A. oryzae
<400> 18
   gttacatgtc tccagaacga cgcccggcgg acatc 35
<210> 19
   <211> 28
   <212> DNA
   <213> A. oryzae
<400> 19
   tgaagcttca gatctcggtg acgggcag 28
<210> 20
   <211> 33
   <212> DNA
   <213> A. oryzae
<400> 20
   ggttaattaa ccggcaggga aggccaatga aag 33
<210> 21
   <211> 39
   <212> DNA
   <213> A. oryzae
<400> 21
   ccacgcgtat ttaaatgtcc gggatggata gcactgtgg 39
<210> 22
   <211> 37
   <212> DNA
   <213> A. oryzae
<400> 22
   ggacgcgtgc ggccgcgtac caggagtacg tcgcagg 37
<210> 23
   <211> 28
   <212> DNA
   <213> A. oryzae
<400> 23
   ggagatctgc agctgtgtac caatagac 28
<210> 24
   <211> 25
   <212> DNA
   <213> A. oryzae
<400> 24
   catttaaatg atgacggcgg atatg 25
<210> 25
   <211> 27
   <212> DNA
   <213> A. oryzae
<400> 25
   gttaattaat cagttgtttt ccaagtc 27
<210> 26
   <211> 27
   <212> DNA
   <213> A. oryzae
<400> 26
   agcggccgct cagttgtttt ccaagtc 27
<210> 27
   <211> 28
   <212> DNA
   <213> A. oryzae
<400> 27
   atttaaatga tgcccaacac gacagaca 28
<210> 28
   <211> 28
   <212> DNA
   <213> A. oryzae
<400> 28
   ttaattaact attgcggatg ttgttgct 28
<210> 29
   <211> 27
   <212> DNA
   <213> A. oryzae
<400> 29
   gcggccgcct attgcggatg ttgttgc 27
<210> 30
   <211> 19
   <212> DNA
   <213> A. oryzae
<220>
   <221> misc_feature
   <222> (3)..(3)
   <223> y= c or t
<220>
   <221> misc_feature
   <222> (6)..(6)
   <223> n= inosine
<220>
   <221> misc_feature
   <222> (9)..(9)
   <223> y= c or t
<220>
   <221> misc_feature
   <222> (15)..(15)
   <223> y= c or t
<220>
   <221> misc feature
   <222> (18)..(18)
   <223> n= a,c,g or t
<400> 30
   gayccngayt ggaayccng 19
<210> 31
   <211> 20
   <212> DNA
   <213> A. oryzae
<220>
   <221> misc_feature
   <222> (3)..(3)
   <223> y= c or t
<220>
   <221> misc_feature
   <222> (6)..(6)
   <223> y= c or t
<220>
   <221> misc_feature
   <222> (9)..(9)
   <223> n= inosine
<220>
   <221> misc_feature
   <222> (12)..(12)
   <223> r= a or g
<220>
   <221> misc_feature
   <222> (15)..(15)
   <223> n= a,c,g, or t
<220>
   <221> misc_feature
   <222> (17)..(17)
   <223> k= g or t
<400> 31
   ttyttytgnc crtcnckcca 20
<210> 32
   <211> 20
   <212> DNA
   <213> A. oryzae
<220>
   <221> misc_feature
   <222> (3)..(3)
   <223> y= c or t
<220>
   <221> misc_feature
   <222> (6)..(6)
   <223> y= c or t
<220>
   <221> misc_feature
   <222> (9)..(9)
   <223> n= inosine
<220>
   <221> misc_feature
   <222> (12)..(12)
   <223> r= a or g
<220>
   <221> misc_feature
   <222> (15)..(15)
   <223> n= a,c,g, or t
<220>
   <221> misc_feature
   <222> (16)..(16)
   <223> y= c or t
<220>
   <221> misc_feature
   <222> (18)..(18)
   <223> n= a,c,g, or t
<400> 32
   aaytayacnc aracnytnga 20
<210> 33
   <211> 19
   <212> DNA
   <213> A. oryzae
<220>
   <221> misc_feature
   <222> (3)..(3)
   <223> n= inosine
<220>
   <221> misc_feature
   <222> (6)..(6)
   <223> y= c or t
<220>
   <221> misc_feature
   <222> (9)..(9)
   <223> y= c or t
<220>
   <221> misc_feature
   <222> (12)..(12)
   <223> d= a,g, or t
<220>
   <221> misc_feature
   <222> (15)..(15)
   <223> n= a,c,g, or t
<220>
   <221> misc_feature
   <222> (18)..(18)
   <223> n= a,c,g, or t
<400> 33
   atnttytcyt cdatngtnc 19
<210> 34
   <211> 19
   <212> DNA
   <213> A. oryzae
<400> 34
   aatgcttgtt gatcagcag 19
<210> 35
   <211> 33
   <212> DNA
   <213> A. oryzae
<400> 35
   gcaagcgcgc gcaatacatg gtgttttgat cat 33
<210> 36
   <211> 69
   <212> DNA
   <213> A. oryzae
<400> 36
   gcctctagat ctcccgggcg cgccggcaca tgtaccaggt cttaagctcg agctcggtca 60
   ccggtggcc 69
<210> 37
   <211> 30
   <212> DNA
   <213> A. oryzae
<400> 37
   gaatgacttg gttgacgcgt caccagtcac 30
<210> 38
   <211> 25
   <212> DNA
   <213> A. oryzae
<400> 38
   cttattagta ggttggtact tcgag 25
<210> 39
   <211> 37
   <212> DNA
   <213> A. oryzae
<400> 39
   gtccccagag tagtgtcact atgtcgaggc agttaag 37
<210> 40
   <211> 64
   <212> DNA
   <213> A. oryzae
<400> 40
   gtatgtccct tgacaatgcg atgtatcaca tgatataatt actagcaagg gaagccgtgc 60
   ttgg 64
<210> 41
   <211> 59
   <212> DNA
   <213> A. oryzae
<400> 41
   cctctagatc tcgagctcgg tcaccggtgg cctccgcggc cgctggatcc ccagttgtg 59
<210> 42
   <211> 33
   <212> DNA
   <213> A. oryzae
<400> 42
   gcaagcgcgc gcaatacatg gtgttttgat cat 33
<210> 43
   <211> 31
   <212> DNA
   <213> A. oryzae
<400> 43
   ttgaattgaa aatagattga tttaaaactt c 31
<210> 44
   <211> 25
   <212> DNA
   <213> A. oryzae
<400> 44
   ttgcatgcgt aatcatggtc atagc 25
<210> 45
   <211> 26
   <212> DNA
   <213> A. oryzae
<400> 45
   ttgaattcat gggtaataac tgatat 26
<210> 46
   <211> 32
   <212> DNA
   <213> A. oryzae
<400> 46
   aaatcaatct attttcaatt caattcatca tt 32
<210> 47
   <211> 45
   <212> DNA
   <213> A. oryzae
<400> 47
   ggatgctgtt gactccggaa atttaacggt ttggtcttgc atccc 45
<210> 48
   <211> 44
   <212> DNA
   <213> A. oryzae
<400> 48
   ggtattgtcc tgcagacggc aatttaacgg cttctgcgaa tcgc 44
<210> 49
   <211> 26
   <212> DNA
   <213> A. oryzae
<400> 49
   tctgtgaggc ctatggatct cagaac 26
<210> 50
   <211> 27
   <212> DNA
   <213> A. oryzae
<400> 50
   gatgctgcat gcacaactgc acctcag 27
<210> 51
   <211> 30
   <212> DNA
   <213> A. oryzae
<400> 51
   atcggtttta tgtcttccaa gtcgcaattg 30
<210> 52
   <211> 33
   <212> DNA
   <213> A. oryzae
<400> 52
   cttggaagac ataaaaccga tggaggggta gcg 33
<210> 53
   <211> 26
   <212> DNA
   <213> A. oryzae
<400> 53
   tctgtgaggc ctatggatct cagaac 26
<210> 54
   <211> 27
   <212> DNA
   <213> A. oryzae
<400> 54
   gatgctgcat gcacaactgc acctcag 27
<210> 55
   <211> 33
   <212> DNA
   <213> A. oryzae
<400> 55
   cgcggatcca ccatgcgtac cgccaagttc gcc 33
<210> 56
   <211> 30
   <212> DNA
   <213> A. oryzae
<400> 56
   gccccgggtt acaggcactg agagtaccag 30
<210> 57
   <211> 23
   <212> DNA
   <213> A. oryzae
<400> 57
   cgatctcgca gtcccgattc gcc 23
<210> 58
   <211> 23
   <212> DNA
   <213> A. oryzae
<400> 58
   tccgggagct gcatgtgtca gag 23

## Description

### Field of the Invention

The present invention relates to a method for preparing variants of a nucleic acid sequence by *in vivo* recombination.

### Description of the Related Art

The advantages of producing biologically active polypeptides by cloning naturally occurring DNA sequences from microorganisms and expressing the DNA sequences in suitable host cells using recombinant DNA technology are well known in the art.

Novel polypeptide variants and mutants, particularly enzymes with improved properties such as specific activity, substrate specificity, pH-optimum, and temperature stability have been obtained by site-directed mutagenesis (see U.S. Patent No. 4,518,584) and random mutagenesis (see, U.S. Patent No. 4,894,331 and WO 93/01285). Site-directed mutagenesis results in substitution, deletion or insertion of specific amino acid residues, which have been chosen either on the basis of their type or on the basis of their location in the secondary or tertiary structure of the mature enzyme.

Since site-directed mutagenesis and random mutagenesis are cumbersome and time consuming methodologies, several alternative methods for the rapid preparation of modified polypeptides have been developed.

Weber et al., 1983, Nucleic Acids Research 11: 5661-5661, disclose a method for modifying genes by *in vivo* recombination between two homologous genes. A linear DNA sequence comprising a plasmid flanked to a DNA sequence encoding alpha-1 human interferon in the 5'-end and a DNA sequence encoding alpha-2 human interferon in the 3'-end is constructed and transfected into a *recA* positive strain of *E. coli*. Recombinants were identified and isolated using a resistance marker.

Pompon el al., 1989, Gene 83: 15-24, describe a method for shuffling gene domains of mammalian cytochrome P-450 by *in vivo* recombination of partially homologous sequences in *Saccharomyces cerevisiae* by transforming *Saccharomyces cerevisiae* with a linearized plasmid with filled-in ends, and a DNA fragment being partially homologous to the ends of said plasmid.

Stemmer, 1994, Proc. Natl. Acad. Sci. USA, 91: 10747-10751, and Stemmer, 1994, Nature 370: 389- 391, disclose methods for shuffling homologous DNA sequences by an *in vitro* PCR method. One cycle of shuffling consists of digesting a pool of homologous genes with DNase I. The resulting small fragments are reassembled into full-length genes. Positive recombinant genes containing shuffled DNA sequences are selected from a DNA library based on their improved function. Positive recombinants can be used as the starting material for (an)other shuffling round(s).

U.S. Patent No. 5,093,257 describes a method for producing hybrid polypeptides by *in vivo* recombination. Hybrid DNA sequences are produced by forming a circular plasmid comprising a replication sequence, a first DNA sequence encoding the amino-terminal portion of the hybrid polypeptide, a second DNA sequence encoding the carboxy-terminal portion of said hybrid polypeptide. The circular plasmid is transformed into a rec-positive microorganism in which the circular plasmid is amplified. This results in recombination of the circular plasmid mediated by the naturally occurring recombination mechanism of the rec-positive microorganism, which include prokaryotes such as *Bacillus* and *E. coli*, and eukaryotes such as *Saccharomyces cerevisiae*.

WO 00/24883 discloses methods of constructing and screening a library of polynucleotide sequences of interest in filamentous fungi by use of an episomal replicating AMA1-based plasmid vector.

Despite the availability of the above methods, there remains a need in the art for *in vivo* recombination methods for preparing variants of a DNA sequence in filamentous fungi.

The object of the present invention is to provide an improved method for preparing variants of a DNA sequence by *in vivo* recombination in filamentous fungi.

### Summary of the Invention

The present invention relates to methods for preparing variants of a nucleotide sequence in a filamentous fungal host, comprising:
(a) introducing into a population of filamentous fungal host cells:
   (1) one or more circular plasmids comprising a DNA sequence and a plasmid replicator mediating autonomous replication, wherein the one or more circularized plasmids are linearized by digestion of the DNA sequence and removal of a portion of the DNA sequence; and
   (2) a library of DNA fragments comprising one or more mutations of the DNA sequence, wherein the fragments comprise at least two regions, one or more first regions which are homologous to the 5' region or the 3' region of the gap in the linearized DNA sequence and/or plasmid sequence and one or more second regions which are homologous to the 5' region or the 3' region of the DNA fragments of the library;
      wherein the linearized plasmids and the DNA fragments recombine by *in vivo* homologous recombination to produce a plurality of autonomously replicating plasmids comprising one or more variants of the DNA sequence; and
      wherein the population of filamentous fungal host cells comprise a heterologous nucleic acid sequence encoding a recombination protein, which participates in the *in vivo* homologous recombination, selected from the group consisting of: (A) a nucleic acid sequence having at least 70% identity with SEQ ID NO:1, SEQ ID NO:3 or SEQ ID NO:5; and (B) a nucleic acid sequence which hybridizes under medium stringency conditions with (i) SEQ ID N0:1, SEQ ID NO:3 or SEQ ID NO:5, (ii) the cDNA sequence contained in SEQ ID NO:1, SEQ ID NO:3 or SEQ ID NO:5, or (iii) a complementary strand of (i) or (ii);
(b) cultivating the population of recombinant filamentous fungal cells in a medium suitable for growth; and
(c) screening the population of recombinant filamentous fungal cells for variants of the DNA sequence contained on one or more autonomously replicating circularized plasmids.

### Brief Description of the Figures

Figures 1A and B show the genomic DNA sequence and the deduced amino acid sequence of an *Aspergillus oryzae rdhA* gene and encoded recombination protein (SEQ ID NOS:1 and 2, respectively).
Figures 2A, B, and C show the shows the genomic DNA sequence and the deduced amino acid sequence of an *Aspergillus oryzae rdhB* gene and encoded recombination protein (SEQ ID NOS:3 and 4, respectively).

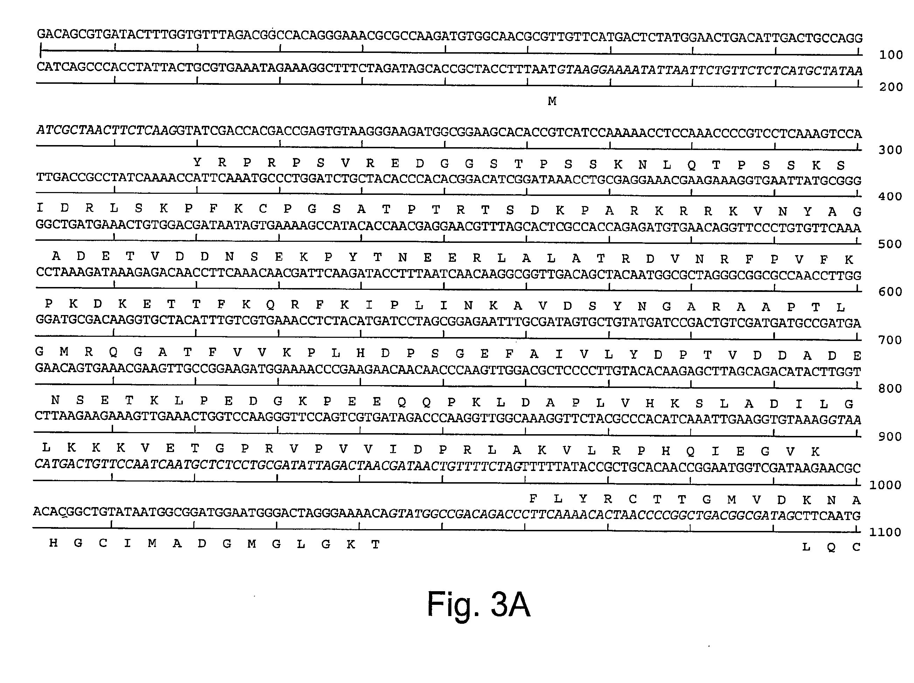

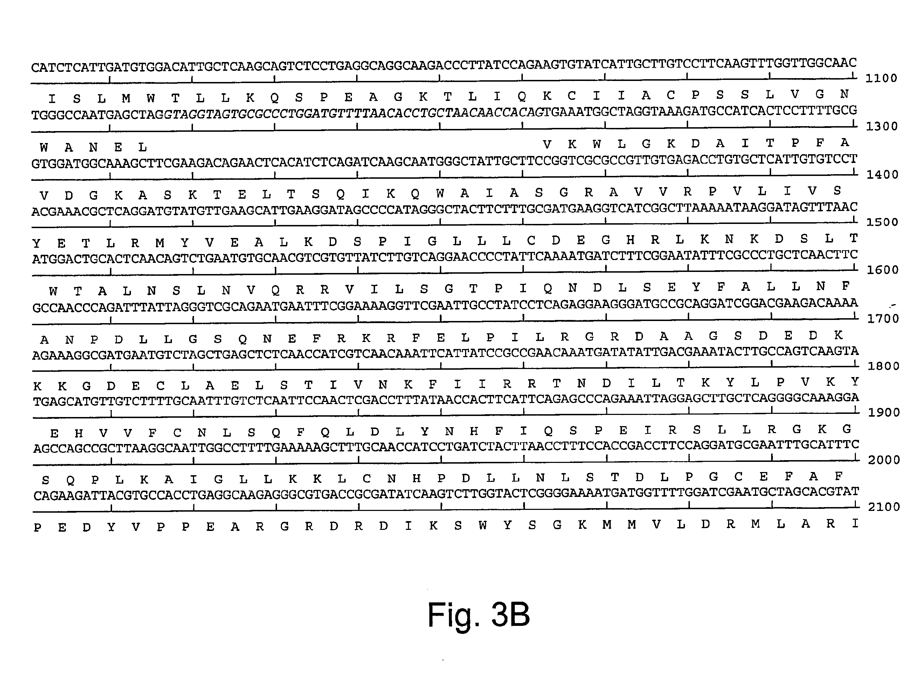

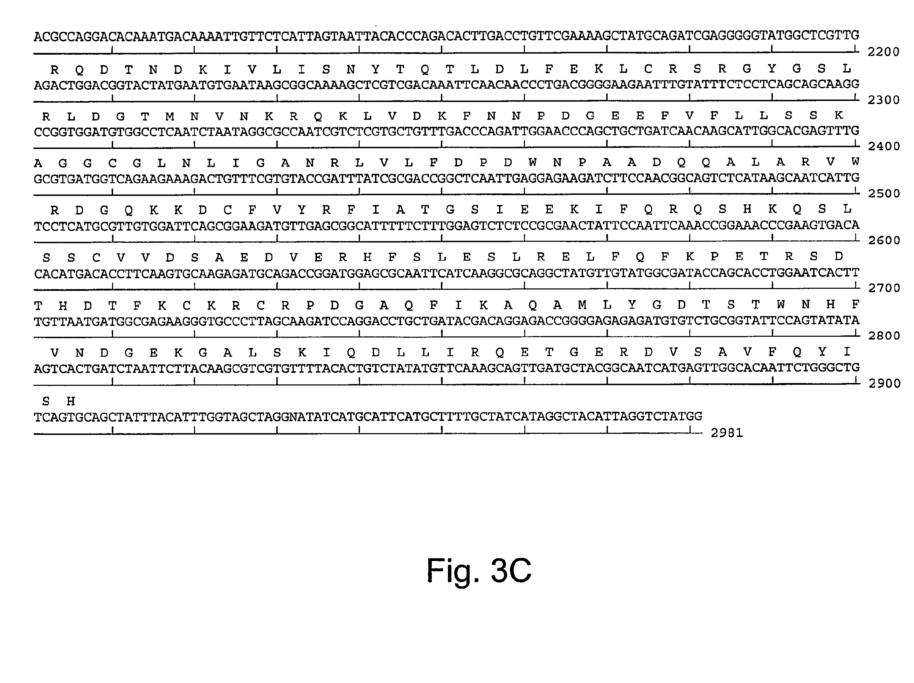

Figures 3A, B, and C show the shows the genomic DNA sequence and the deduced amino acid sequence of an *Aspergillus oryzae rdhD* gene and encoded recombination protein (SEQ ID NOS:5 and 6, respectively).
Figure 4 shows a restriction map of pPaHa3B.
Figure 5 shows a restriction map of pSMO145.
Figure 6 shows a restriction map of pToC202.
Figure 7 shows a restriction map of pSMO146.
Figure 8 shows a restriction map of pPH5.
Figure 9 shows a restriction map of pPH7.
Figure 10 shows a restriction map of pCW013.
Figure 11 shows the relative *Humicola insolens* cellobiohydralase activity of gap repaired transformants.

### Detailed Description of the Invention

The present invention relates to methods for preparing variants of a nucleotide sequence in a filamentous fungal host, comprising: (a) introducing into a population of filamentous fungal host cells: (1) one or more circular plasmids comprising a DNA sequence and a plasmid replicator mediating autonomous replication, wherein the one or more circularized plasmids are linearized by digestion of the DNA sequence and removal of a portion of the DNA sequence; and (2) a library of DNA fragments comprising one or more mutations of the DNA sequence, wherein the fragments comprise at least two regions, one or more first regions which are homologous to the 5' region or the 3' region of the gap in the linearized DNA sequence and/or plasmid sequence and one or more second regions which are homologous to the 5' region or the 3' region of the DNA fragments of the library; wherein the linearized plasmids and the DNA fragments recombine by *in vivo* homologous recombination to produce a plurality of autonomously replicating plasmids comprising one or more variants of the DNA sequence; and wherein the population of filamentous fungal host cells comprise a heterologous nucleic acid sequence encoding a recombination protein, which participates in the *in vivo* homologous recombination, selected from the group consisting of: (A) a nucleic acid sequence having at least 70% identity with SEQ ID NO:1, SEQ ID NO:3 or SEQ ID NO:5; and (B) a nucleic acid sequence which hybridizes under medium stringency conditions with (i) SEQ ID NO:1, SEQ ID NO:3 or SEQ ID NO:5, (ii) the cDNA sequence contained in SEQ ID NO:1, SEQ ID NO:3 or SEQ ID NO:5, or (iii) a complementary strand of (i) or (ii); (b) cultivating the population of recombinant filamentous fungal cells in a medium suitable for growth; and (c) screening the population of recombinant filamentous fungal cells for variants of the DNA sequence contained on one or more autonomously replicating circularized plasmids. This method, which we define here as gap repair, has a number of advantages over previously described methods.

An important advantage of the methods of the present invention is that they allow the shuffling of DNA fragments that are homologous with a DNA sequence of interest and the recovery of the resulting variants of the DNA sequence contained in autonomously replicating plasmids.

Another advantage of the methods of the present invention is that because of the efficient gap repair and high transformation frequency using the autonomous replicating plasmid, sufficient yields of gap repaired transformants can permit high throughput robotic screening similar to that performed in yeast.

A further advantage is that the present methods allow the construction of variant libraries *in vivo* in filamentous fungi. Previous methods for construction of variant libraries were dependent on the propagation of DNA in a host that allowed amplification of the said DNA, such as the propagation of plasmids containing bacterial replication sequences in *E. coli*, purification of the DNA, and transformation of the DNA into filamentous fungi. This method not only is much more labor intensive, but also is most typically accomplished by pooling of individual clones for plasmid purification. Such amplification, pooling, and transformation result in libraries in filamentous fungi that contain multiples of the original variants, increasing the screening required to ensure that all members of the original library are examined.

The present methods also allow the direct construction of autonomously replicating plasmids *in vivo* in filamentous fungi.

There is another important advantage of the methods of the present invention. In the yeast *Saccharomyces cerevisiae* the frequency of homologous recombination approaches 100%, allowing for very efficient gap repair as previously described by Pompon el al., 1989, Gene 83: 15-24. In contrast, the recombination frequency in many filamentous fungi, including *Aspergillus oryzae* and *Aspergillus niger*, usually varies between 0 and 5%, with most integration being random even when transformed with homologous DNA. For this reason, gap repair producing functional products is not expected in adequate numbers in filamentous fungi. Surprisingly, in the methods of the present invention, *in vivo* gap repair in *Aspergillus oryzae* indicate recombination resulting in functional products as a result of both perfect and imprecise homologous recombination within the overlap region shared between the gapped plasmid and linear DNA. The methods of the present invention may take advantage of this mode of recombination as over 90% functional recombination products can be obtained by having the recombination initiate within a non-functional region flanking the gap.

The term "shuffling" means recombination of nucleotide sequence(s) between two or more homologous DNA sequences resulting in recombines DNA sequences (*i*.*e*. DNA sequences having been subjected to a shuffling cycle) having a number of nucleotides exchanged, in comparison to the starting DNA sequences.

The term "recombination" is defined herein as the process wherein nucleic acids associate with each other in regions of homology, leading to interstrand DNA exchange between those sequences. For purposes of the present invention, homologous recombination is determined according to the procedures summarized by Paques and Haber, 1999, Microbiology and Molecular Biology Reviews 63: 349-404. The recombination may be homologous or non-homologous. "Homologous recombination" is defined herein as recombination in which no changes in the DNA sequences occurs within the regions of homology relative to the input DNA sequences. For perfect homologous recombination, the one or more regions should contain a sufficient number of nucleic acids, such as 100 to 1,500 base pairs, preferably 400 to 1,500 base pairs, and most preferably 800 to 1,500 base pairs, which are highly homologous with the corresponding nucleic acid sequence to enhance the probability of homologous recombination. "Non-homologous recombination" is defined herein as recombination where any mode of DNA repair incorporating strand exchange results in a DNA sequence different from any of the recombining sequences.

### DNA Sequences

In the methods of the present invention, the DNA sequence may be any DNA sequence. The DNA sequence preferably is selected from the group consisting of (a) a gene that encodes a polypeptide or an RNA; (b) a disrupted gene; (c) a partially deleted gene; (d) a regulatory control sequence; (e) a recombinantly manipulated version of a gene native or foreign to the filamentous fungal host cell; (f) a transposon; (g) a ribozyme; or (h) a portion of (a), (b), (c), (d), (e), (f) or (g).

The DNA sequences may be wild-type DNA sequences, DNA sequences encoding variants or mutants, or modifications thereof, such as extended or elongated DNA sequences, and may also be the outcome of DNA sequences having been subjected to one or more cycles of shuffling (*i.e*. variant DNA sequences) according to the methods of the invention or any other method known in the prior art.

In a preferred embodiment, the DNA sequence comprises a gene encoding a polypeptide or an RNA. The polypeptide or RNA encoded by the DNA sequence may be native or heterologous to the fungal host cell of interest.

The term "polypeptide" is not meant herein to refer to a specific length of the encoded product and, therefore, encompasses peptides, oligopeptides, and proteins. The term "heterologous polypeptide" is defined herein as a polypeptide that is not native to the filamentous fungal cell; a native polypeptide in which modifications have been made to alter the native sequence; or a native polypeptide whose expression is quantitatively altered as a result of a manipulation of the filamentous fungal cell by recombinant DNA techniques. For example, a native polypeptide may be recombinantly produced by, for example, placing a gene encoding the polypeptide under the control of a strong promoter.

In the methods of the present invention, the DNA sequences may be either wild-type, variant or modified DNA sequences, such as a DNA sequences coding for wild-type, variant or modified enzymes, respectively.

The polypeptide may be an antibody, hormone, enzyme, receptor, reporter, selectable marker, or protein having biological activity. In a preferred embodiment, the polypeptide is an oxidoreductase, transferase, hydrolase, lyase, isomerase, or ligase. In a more preferred embodiment, the polypeptide is an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, invertase, laccase, lipase, mannosidase, mutanase, oxidase, pectinolytic enzyme, peroxidase, phospholipase, phytase, polyphenoloxidase, proteolytic enzyme, ribonuclease, transglutaminase, or xylanase. In another preferred embodiment, the polypeptide is secreted extracellularly.

The hormone or protein having biological activity may be insulin, ACTH, glucagon, somatostatin, somatotropin, thymosin, parathyroid hormone, pigmentary hormones, somatomedin, erythropoietin, luteinizing hormone, chorionic gonadotropin, hypothalamic releasing factors, antidiuretic hormones, thyroid stimulating hormone, relaxin, interferon, thrombopoietin (TPO), or prolactin.

The DNA sequence encoding a polypeptide of interest may be obtained from any prokaryotic, eukaryotic, or other source, if suitable for expression in a filamentous fungal cell. The techniques used to isolate or clone a DNA sequence of interest are known in the art and include isolation from genomic DNA, preparation from cDNA, or a combination thereof, as described above. The DNA sequence may be of genomic, cDNA, RNA, semisynthetic, synthetic origin, or any combinations thereof.

In the methods of the present invention, the polypeptide may also include a fused or hybrid polypeptide in which another polypeptide is fused at the N-terminus or the C-terminus of the polypeptide or fragment thereof. A fused polypeptide is produced by fusing a nucleic acid sequence (or a portion thereof) encoding one polypeptide to a nucleic acid sequence (or a portion thereof) encoding another polypeptide. Techniques for producing fusion polypeptides are known in the art, and include, ligating the coding sequences encoding the polypeptides so that they are in frame and expression of the fused polypeptide is under control of the same promoter(s) and terminator. The hybrid polypeptide may comprise a combination of partial or complete polypeptide sequences obtained from at least two different polypeptides wherein one or more may be heterologous to the mutant fungal cell.

In another preferred embodiment, the DNA sequence comprises a disrupted gene. The gene may be disrupted with any nucleic acid sequence. In a preferred embodiment, the gene is disrupted with a selectable marker gene. In a more preferred embodiment, the gene is disrupted with a selectable marker gene selected from the group consisting of *amds* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), *sC* (sulfate adenyltransferase), and *trpC* (anthranilate synthase), as well as equivalents thereof. Preferred for use in an *Aspergillus* cell are the *amds* and *pyrG* genes of *Aspergillus nidulans* or *Aspergillus oryzae* and the *bar* gene of *Streptomyces hygroscopicus*. However, any selectable marker may be used if compatible with the filamentous fungal cell of choice.

In another preferred embodiment, the DNA sequence comprises a partially or fully deleted gene. Where the DNA sequence comprises a fully deleted gene, it will be understood that the nucleic acid sequence will contain regions upstream and downstream of the gene that are homologous with corresponding regions of the DNA fragments.

The DNA sequence comprising a disrupted or deleted gene may be constructed by using methods well known in the art, for example, insertions, disruptions, replacements, or deletions. The gene to be disrupted or deleted may be, for example, the coding region or a part thereof essential for activity, or the gene may contain a regulatory element required for expression of the coding region. An example of such a regulatory or control sequence may be a promoter sequence or a functional part thereof, i.e., a part which is sufficient for affecting expression of the nucleic acid sequence. Other control sequences for possible modification include, but are not limited to, a leader, polyadenylation sequence, propeptide sequence, signal sequence, transcription terminator, and transcriptional activator. See below for further discussion.

Disruption or deletion of the gene may be also accomplished by introduction, substitution, or removal of one or more nucleotides in the gene or a regulatory element required for the transcription or translation thereof. For example, nucleotides may be inserted or removed so as to result in the introduction of a stop codon, the removal of the start codon, or a change of the open reading frame.

An example of a convenient way to disrupt or delete a gene is based on techniques of gene replacement, gene deletion, or gene disruption. For example, in the gene disruption method, a nucleic acid sequence corresponding to the endogenous gene or gene fragment of interest is mutagenized *in vitro* to produce a defective nucleic acid sequence which is then transformed into the parent cell to produce a defective gene. By homologous recombination, the defective nucleic acid sequence replaces the endogenous gene or gene fragment. It may be desirable that the defective gene or gene fragment also encodes a marker, which may be used for selection of transformants in which the nucleic acid sequence has been modified or destroyed. The selectable marker gene may be used to achieve the disruption. The defective nucleic acid sequence may be a simple disruption of the endogenous sequence with a selectable marker gene. Alternatively, the defective nucleic acid sequence may contain an insertion or deletion of the endogenous sequence, or a portion thereof, in addition to the disruption with the selectable marker gene. Furthermore, the defective nucleic acid sequence may contain an insertion or deletion of the endogenous sequence, or a portion thereof, and the selectable marker gene is not involved in the modification but is used as a selectable marker for identifying transformants containing the defective gene.

In another preferred embodiment, the DNA sequence comprises a regulatory control sequence. The regulatory control sequence can be any control sequence, including, but not limited to, a promoter, signal sequence, leader, polyadenylation sequence, propeptide sequence, consensus translational initiator sequence, signal peptide sequence, and transcription terminator.

In another preferred embodiment, the DNA sequence comprises a recombinantly manipulated version of a gene native or foreign to the filamentous fungal cell.

In another preferred embodiment, the DNA sequence comprises a transposon. The term "transposon" is defined herein as mobile DNA sequence that can move from one site in a genome to another, or between different chromosomes (see *Plant Pathology* 534 (Gen CB 534) Fungal Genetics Spring 2001). There are two basic types of transposable elements in all organisms: (1) DNA sequences which move themselves to a new location as DNA (2) DNA sequences which move to a new location via an RNA intermediate. Transposons can: (1) inactivate genes (2) re-activate pseudogenes (genes which are unable to code for proteins) because they have promoter sequences 3) change expression of genes if they insert in regulatory regions. Transposons can promote rearrangements of the genome either directly or indirectly: (a) directly - transposition event may cause deletions or inversions (b) indirectly - transposons serve as substrates for recombination - "portable regions of homology" - provide sites for reciprocal recombination.

Examples of transposons include, but are not limited to, P elements, LINES, SINES, Ty1, gypsy, Fot1, hAT, Restless, Guest, elements, tn10, Tad-1, Afut-1, and the retrotransposons MAGGY Ty3 and Ty5.

### Plasmid Replicator

In the methods of the present invention, the plasmid replicator may be any plasmid replicator mediating autonomous replication which functions in a filamentous fungal cell. The term "plasmid replicator" is defined herein as a sequence that enables a plasmid or vector to replicate independent of chromosomal replication. Replicators often consist of sequences that do not represent authentic genomic replicators. Their mode of function in most cases are not understood. Often these plasmids occur spontaneously and are not recognized by mitotic mechanisms and are quickly lost lacking selective pressure.

Examples of a plasmid replicator useful in a filamentous fungal cell is AMA1 and ANS1 (Gems et al., 1991, Gene 98:61-67; Cullen et al., 1987, Nucleic Acids Research 15: 9163-9175; WO 00/24883). Isolation of the AMA1 gene and construction of plasmids or vectors comprising the gene can be accomplished according to the methods disclosed in WO 00/24883.

### Plasmids

The plasmid or plasmids may be any plasmid or vector that may conveniently be subjected to recombinant DNA procedures. The plasmid comprising the DNA sequence may be prepared by ligating the DNA sequence into a suitable plasmid, or by any other suitable method. The choice of plasmid will often depend on the filamentous fungal host cell into which it is to be introduced. In the methods of the present invention, the plasmid is an autonomously replicating plasmid, *i*.*e*. a plasmid which exists as an extrachromosomal entity, the replication of which is independent of chromosomal replication.

The linearizing of the plasmid(s) can be directed toward any site within the plasmid. The plamid(s) may be linearized by any suitable methods known in the art, for example, digestion with a restriction enzyme. The linearized ends of the plasmid may be filled-in with nucleotides as described in Pompon *et al.,* 1989, supra. However, it is preferred not to fill in the linearized ends as it might create a frameshift.

To facilitate the screening process, the plasmid is preferably an expression vector in which the DNA sequence in question is operably linked to additional segments required for transcription of the DNA. In general, the expression vector is derived from a plasmid, a cosmid or a bacteriophage, or may contain elements of any or all of these. For purposes of the present invention, the terms "plasmid" and "vector" are used interchangeably.

The DNA sequence will generally be operably linked to one or more regulatory control sequences which direct the expression of the coding sequence in a suitable host cell under conditions compatible with the control sequences. The term "expression" will be understood to include any step involved in the production of the polypeptide including, but not limited to, transcription, post-transcriptional modification, translation, post-translational modification, and secretion. The term "operably linked" indicates that the segments are arranged so that they function in concert for their intended purposes, *e.g.*, transcription initiates in a promoter and proceeds through the DNA sequence coding for the polypeptide in question.

The DNA sequence may be manipulated in a variety of ways to provide for expression of the polypeptide. Manipulation of the DNA sequence prior to its insertion into a plasmid or vector may be desirable or necessary depending on the DNA sequence, expression vector, and/or filamentous fungal host cell. The techniques for modifying nucleic acid sequences utilizing recombinant DNA methods are well known in the art.

The term "regulatory control sequences" is defined herein to include all components which are necessary or advantageous for the expression of a polypeptide of the present invention. Each control sequence may be native or foreign to the nucleic acid sequence encoding the polypeptide. Such control sequences include, but are not limited to, a leader, polyadenylation sequence, propeptide sequence, consensus translational initiator sequence of the present invention, signal peptide sequence, and transcription terminator. At a minimum, the control sequences include transcriptional and translational stop signals. The control sequences may be provided with linkers for the purpose of introducing specific restriction sites facilitating ligation of the control sequences with the coding region of the nucleic acid sequence encoding a polypeptide.

The control sequence may be an appropriate promoter sequence, a nucleic acid sequence which is recognized by a host cell for expression of the DNA sequence. The promoter sequence contains transcriptional control sequences which mediate the expression of the polypeptide. The promoter may be any nucleic acid sequence which shows transcriptional activity in the filamentous fungal host cell of choice including mutant, truncated, and hybrid promoters, and may be obtained from genes encoding extracellular or intracellular polypeptides either homologous or heterologous to the host cell.

Examples of suitable promoters for directing the transcription of the DNA sequence in a filamentous fungal host cell are promoters obtained from the genes for *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase *(glaA), Rhizomucor miehei* lipase, *Aspergillus oryzae* alkaline protease, *Aspergillus oryzae* triose phosphate isomerase, *Aspergillus nidulans* acetamidase, *Fusarium venenatum* amyloglucosidase, *Fusarium oxysporum* trypsin-like protease (WO 96/00787), as well as the NA2-tpi promoter (a hybrid of the promoters from the genes for *Aspergillus niger* neutral alpha-amylase and *Aspergillus oryzae* triose phosphate isomerase); and mutant, truncated, and hybrid promoters thereof.

The control sequence may be a suitable transcription terminator sequence, a sequence recognized by a host cell to terminate transcription. The terminator sequence is operably linked to the 3' terminus of the DNA sequence. Any terminator which is functional in the filamentous fungal host cell of choice may be used in the present invention.

Preferred terminators for filamentous fungal host cells are obtained from the genes for *Aspergillus oryzae* TAKA amylase, *Aspergillus* nigerglucoamylase, *Aspergillus nidulans* anthranilate synthase, *Aspergillus* nigeralpha-glucosidase, and *Fusarium oxysporum* trypsin-like protease.

The control sequence may also be a suitable leader sequence, a non-translated region of an mRNA which is important for translation by the filamentous fungal host cell. The leader sequence is operably linked to the 5'-terminus of the DNA sequence. Any leader sequence that is functional in the host cell of choice may be used in the present invention.

Preferred leaders for filamentous fungal host cells are obtained from the genes for *Aspergillus oryzae* TAKA amylase and *Aspergillus nidulans* triose phosphate isomerase.

The control sequence may also be a polyadenylation sequence, a sequence operably linked to the 3' terminus of the DNA sequence and which, when transcribed, is recognized by the host cell as a signal to add polyadenosine residues to transcribed mRNA. Any polyadenylation sequence which is functional in the filamentous fungal host cell of choice may be used in the present invention.

Preferred polyadenylation sequences for filamentous fungal host cells are obtained from the genes for *Aspergillus oryzae* TAKA amylase, *Aspergillus niger* glucoamylase, *Aspergillus nidulans* anthranilate synthase, *Fusarium oxysporum* trypsin-like protease, and *Aspergillus niger* alpha-glucosidase.

The control sequence may also be a signal peptide coding region that codes for an amino acid sequence linked to the amino terminus of a polypeptide and directs the encoded polypeptide into the cell's secretory pathway. The 5'-end of the coding sequence of the DNA sequence encoding a polypeptide may inherently contain a signal peptide coding region naturally linked in translation reading frame with the segment of the coding region which encodes the secreted polypeptide. Alternatively, the 5'-end of the coding sequence may contain a signal peptide coding region which is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the polypeptide. However, any signal peptide coding region which directs the expressed polypeptide into the secretory pathway of a filamentous fungal host cell of choice may be used in the present invention.

Effective signal peptide coding regions for filamentous fungal host cells are the signal peptide coding regions obtained from the genes for *Aspergillus oryzae* TAKA amylase, *Aspergillus niger* neutral amylase, *Aspergillus niger* glucoamylase, *Rhizomucor miehei* aspartic proteinase, *Humicola insolens* cellulase, and *Humicola lanuginosa* lipase.

The control sequence may also be a propeptide coding region that codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for *Rhizomucor miehei* aspartic proteinase and *Myceliophthora thermophila* laccase (WO 95/33836).

Where both signal peptide and propeptide regions are present at the amino terminus of a polypeptide, the propeptide region is positioned next to the amino terminus of a polypeptide and the signal peptide region is positioned next to the amino terminus of the propeptide region.

It may also be desirable to add regulatory sequences which allow the regulation of the expression of the DNA sequence relative to the growth of the host cell. Examples of regulatory systems are those which cause the expression of the gene to be turned on or off in response to a chemical or physical stimulus, including the presence of a regulatory compound. In filamentous fungi, the TAKA alpha-amylase promoter, *Aspergillus niger* glucoamylase promoter, and *Aspergillus oryzae* glucoamylase promoter may be used as regulatory sequences. Other examples of regulatory sequences are those which allow for gene amplification. In eukaryotic systems, these include the dihydrofolate reductase gene which is amplified in the presence of methotrexate, and the metallothionein genes which are amplified with heavy metals. In these cases, the nucleic acid sequence encoding the polypeptide would be operably linked with the regulatory sequence.

### DNA Fragments

The library of DNA fragments to be randomly combined (or "shuffled") with homologous regions in the linearized plasmid(s) by *in vivo* recombination may be prepared by any suitable method. For instance, the DNA fragment may be prepared by PCR amplification (polymerase chain reaction) of a plasmid or plasmid comprising the DNA sequence, using specific primers, for instance as described in U.S. Patent No. 4,683,202 or Saiki et al., 1988, Science 239: 487-491. The DNA fragment may also be isolated from a plasmid or plasmid comprising the desired DNA sequence by digestion with restriction enzymes, followed by isolation using, for example, electrophoresis.

The DNA fragment may alternatively be prepared synthetically by established standard methods, *e.g.* the phosphoamidite method described by Beaucage and Caruthers, 1981, Tetrahedron Letters 22: 1859-1869, or the method described by Matthes et al., (1984), EMBO Journal 3: 801-805. According to the phosphoamidite method, oligonucleotides are synthesized, for example, in an automatic DNA synthesizer, purified, annealed, ligated, and cloned into suitable plasmids.

Furthermore, the DNA fragment may be of mixed synthetic and genomic, mixed synthetic and cDNA or mixed genomic and cDNA origin prepared by ligating fragments of synthetic, genomic or cDNA origin (as appropriate), the fragments corresponding to various parts of the entire DNA sequence, in accordance with standard techniques.

The library of DNA fragments comprise one or more mutations of the DNA sequence,
wherein the fragments comprise at least two regions, one or more regions which are homologous to the 5' region or the 3' region of the gap in the linearized DNA sequence and/or plasmid sequence and one or more second regions which are homologous to the 5' region or the 3' region of the DNA fragments of the library.

The regions of the DNA fragment may be any sequence that is homologous with the DNA sequence and/or plasmid sequence.

In a preferred embodiment, the two or more regions of the DNA fragment are a 5' region and/or a 3' region that flank (a) a gene that encodes a polypeptide or an RNA; (b) a gene disrupted with a third nucleic acid sequence; (c) a partially deleted gene; (d) a regulatory control sequence; (e) a recombinantly manipulated version of a gene native or foreign to the filamentous fungal host cell; (f) a transposon; (g) a ribozyme; or (h) a portion of (a), (b), (c), (d), (e), (f) or (g).

In another preferred embodiment, the two or more regions of the DNA fragment are a 5' region and/or a 3' region of (a) a gene that encodes a polypeptide or an RNA; (b) a gene disrupted with a third nucleic acid sequence; (c) a partially deleted gene; (d) a regulatory control sequence; (e) a recombinantly manipulated version of a gene native or foreign to the filamentous fungal host cell; (f) a transposon; (g) a ribozyme; or (h) a portion of (a), (b), (c), (d), (e), (f) or (g).

In another preferred embodiment, the one or more regions of the DNA fragment that are homologous to the DNA sequence are part of a gene native or foreign to the filamentous fungal host cell.

In a preferred embodiment of the present invention, the DNA fragment fragments are prepared under conditions resulting in a low, medium or high random mutagenesis frequency. To obtain low mutagenesis frequency the DNA sequence(s) (comprising the DNA fragment(s)) may be prepared by a standard PCR amplification method (US 4,683,202 or Saiki et al., 1988, Science 239: 487-491). A medium or high mutagenesis frequency may be obtained by performing the PCR amplification under conditions which reduce the fidelity of replicaton by the thermostable polymerase and increase the misincorporation of nucleotides, for instance as described by Deshler, 1992, GATA 9: 103-106; Leung et al., 1989, BioTechniques 1: 11-15.

The PCR amplification (*i*.*e*. according to this embodiment also DNA fragment mutation) may be combined with a mutagenesis step using a suitable physical or chemical mutagenizing agent, *e.g.*, one which induces transitions, transversions, inversions, scrambling, deletions, and/or insertions.

In a preferred embodiment, the DNA fragment(s) to be shuffled preferably have a length of from about 30 bp to 8 kb, more preferably about 40 bp to 6 kb, even more preferably about 80 bp to 4 kb, and most preferably about 100 bp to 2 kb, to be able to interact optimally with the linearized plasmid.

### Filamentous Fungal Host Cells

The filamentous fungal host cell, into which the mixture of plasmid/fragment DNA sequences are to be introduced, may be any filamentous fungal cell useful in the methods of the present invention. A "recombination filamentous fungal cell" is defined herein as a cell capable of mediating shuffling of a number of homologous DNA sequences.

"Filamentous fungi" include all filamentous forms of the subdivision Eumycota and Oomycota (as defined by Hawksworth *et al.,* 1995, *supra*). The filamentous fungi are characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

In a preferred embodiment, the filamentous fungal host cell is an *Acremonium, Aspergillus, Fusarium, Humicola, Mucor, Myceliophthora, Neurospora, Penicillium, Thielavia, Tolypocladium,* or *Trichoderma* cell.

In a more preferred embodiment, the filamentous fungal host cell is an *Aspergillus awamori, Aspergillus foetidus, Aspergillus japonicus, Aspergillus nidulans, Aspergillus niger* or *Aspergillus oryzae* cell. In another most preferred embodiment, the filamentous fungal host cell is a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell. In another most preferred embodiment, the filamentous fungal host cell is a *Humicola insolens, Humicola lanuginosa, Mucor miehei, Myceliophthora thermophila, Neurospora crassa, Penicillium purpurogenum, Thielavia terrestris, Trichoderma harzianum, Trichoderma koningii, Trichoderma longibrachiatum, Trichoderma reesei,* or *Trichoderma viride* cell.

In another most preferred embodiment, the *Aspergillus* cell is an *Aspergillus oryzae* cell.

In another most preferred embodiment, the *Aspergillus* cell is an *Aspergillus niger* cell.

In another most preferred embodiment, the *Fusarium venenatum* cell is *Fusarium venenatum* A3/5, which was originally deposited as *Fusarium graminearum* ATCC 20334 and recently reclassified as *Fusarium venenatum* by Yoder and Christianson, 1998, Fungal Genetics and Biology23: 62-80 and O'Donnell et al., 1998, Fungal Genetics and Biology 23: 57-67; as well as taxonomic equivalents of *Fusarium venenatum* regardless of the species name by which they are currently known. In another preferred embodiment, the *Fusarium venenatum* cell is a morphological mutant of *Fusarium venenatum* A3/5 or *Fusarium venenatum* ATCC 20334, as disclosed in WO 97/26330.

Fungal cells may be transformed by a process involving protoplast formation, transformation of the protoplasts, and regeneration of the cell wall in a manner known *per* se. Suitable procedures for transformation of *Aspergillus* host cells are described in EP 238 023 and Yelton et al., 1984, Proceedings of the National Academy of Sciences USA 81: 1470-1474. Suitable methods for transforming *Fusarium* species are described by Malardier et al., 1989, Gene 78: 147-156 and WO 96/00787.

### In vivo Recombination

The methods of the present invention result in a high level of mixing of homologous genes or variants. A large number of variants or homologous genes can be mixed in one transformation. The mixing of improved variants or wild type genes followed by screening increases multi-fold the number of further improved variants compared to doing only random mutagenesis (for review see Kuchner, K. and Arnold, F.H. 1997. Directed evolution of enzyme catalysts. TIBTech 15:523-530). Random mutagenesis introduces mutations into a target DNA sequence, creating deleterious mutations much more frequently than beneficial ones. In iterative rounds of such mutagenesis, deleterious mutations accumulate more rapidly than beneficial ones, effectively masking the identification of beneficial mutations during screening. The random recombination between two or more homologous DNA sequences that contain multiple single nucleotide changes in their DNA sequences potentially allows all those nucleotide changes contained in one variant to be separated from one another and to be randomly mixed with any mutations present on other variants. This shuffling of mutations allows a means by which mutations from different parent sequences can be combined with each other randomly, The result of utilizing this method is an increased probability of combining nucleotide changes in a single DNA sequence.

Recombination of multiple overlapping fragments is possible with a high efficiency increasing the mixing of variants or homologous genes using the *in vivo* recombination method. An overlap as small as 30 bp is sufficient for recombination which may be utilized for very easy domain shuffling of even distantly related genes. In domain shuffling, larger blocks of non-homologous DNA are randomly assorted by means of stretches of homology at their termini.

In methods of the present invention, the term "positive polypeptide variants" means resulting polypeptide variants possessing a functional property or properties which have been improved in comparison to the polypeptides producible from the corresponding input DNA sequences. Examples, of such improved properties can be as different as *e.g.* biological activity, enzyme washing performance, antibiotic resistance etc. If the improved functional property of the polypeptide is not sufficiently good after one cycle of shuffling, the variant DNA sequence may be subjected to another cycle *ad infinitum*.

In a preferred embodiment, at least one shuffling cycle is a backcrossing cycle with the initially used DNA fragment or fragments, which may be the wild-type DNA fragment. This eliminates non-essential mutations. Non-essential mutations may also be eliminated by using wild-type DNA fragments as the initially used input DNA material.

However, the method of the present invention will in most cases lead to the replacement of a considerable number of amino acid and may in certain cases even alter the structure of one or more polypeptide domains (*i*.*e*. a folded unit of polypeptide structure).

According to the present invention more than two DNA sequences are shuffled at the same time. Actually any number of different DNA fragments and homologous polypeptides comprised in suitable plasmids may be shuffled at the same time. This is advantageous as a vast number of quite different variants can be made rapidly without an abundance of iterative procedures.

When recombining many fragments from the same region, multiple overlapping of the fragments will increase mixing by itself, but it is also important to have a relative high random mixing in overlapping regions in order to mix closely located variants/differences.

An overlap as small as 30 bp between two fragments is sufficient to obtain a very efficient recombination. Therefore, overlapping in the range from 30 to 5000 bp, preferably from 30 bp to 500 bp, especially 30 bp to 100 bp is suitable in the methods of the present invention.

In this embodiment of the present invention, preferably 2 or more overlapping fragments, more preferably 2 to 50 overlapping fragments, and most preferably 2 to 10 overlapping fragments may advantageously be used as DNA fragments in a shuffling cycle.

Besides increasing the mixing of genes, this is a very useful method for domain shuffling by creating small overlaps between DNA fragments from different domains and screen for the best combination. For example, in the case of three DNA fragments the overlapping regions may be as follows: the first end of the first fragment overlaps the first end of the linearized plasmid, the first end of the second fragment overlaps the second end of the first fragment, and the second end of the second fragment overlaps the first end of the third fragment, the first end of the third fragment overlaps (as stated above) the second end of the second fragment, and the second end of the third fragment overlaps the second end of the linearized plasmid.

It is understood that when using two or more DNA fragments as starting material, it is preferred to have continuous overlaps between the ends of the plasmid and the DNA fragments.

Even though it is preferred to shuffle homologous DNA sequences in the form of DNA fragment(s) and linearized plasmid(s), it is also possible to shuffle two or more linearized plasmids comprising homologous DNA sequences encoding polypeptides. However, in such a case it is important to linearize the plasmids at different sites.

In a further embodiment of the invention two or more linearized plasmids and one or more homologous DNA fragments are used as the starting material to be shuffled. The ratio between the linearized plasmid(s) and homologous DNA fragment(s) preferably lie in the range from 20:1 to 1:50, preferable from 2:1 to 1:10 (mol plasmid:mol fragments) with the specific concentrations being from 1 pM to 10 M of the DNA.

The linearized plasmids may be gapped in such a way that the overlap between the fragments is deleted in the plasmid. The repair of the gap in the plasmid then requires that the fragments recombine with one another in addition to recombining with the ends of the gapped plasmid in order to reconstitute a circular, autonomously replicating plasmid. In a preferred embodiment, the linearization of the plasmid or vector creates a sufficient gap in the coding sequence of the DNA sequence to force the homologous recombination of the DNA fragments with the corresponding regions of the DNA sequence. As mentioned earlier, gap repair producing functional products is not expected in adequate numbers in filamentous fungi. However, under the methods of the present invention, *in vivo* gap repair in *Aspergillus oryzae* indicates recombination resulting in functional products as a result of both perfect and imprecise homologous recombination within the overlap region shared between the gapped plasmid and linear DNA. Under this mode of recombination, over 90% functional recombination products can be obtained by having the recombination initiate within a non-functional, non-target region flanking the gap. Incorporation into a self-replicating plasmid increases the transformation frequency up to 4 orders of magnitude permitting organisms with inefficient rates of recombination, to achieve sufficient enough transformation for high throughput screening.

### Methods of Cultivation

In the methods of the present invention, the recombinant filamentous fungal host cells are cultivated in a nutrient medium suitable for growth of the cell or the production of the polypeptide variants of interest using methods known in the art. For example, the filamentous fungal cell may be cultivated by shake flask cultivation, and small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.,* in catalogues of the American Type Culture Collection). If the polypeptide is secreted into the nutrient medium, the polypeptide can be recovered directly from the medium. If the polypeptide is not secreted, it can be recovered from cell lysates.

The polypeptide variants may be detected using methods well known in the art that are specific for the polypeptides. These detection methods may include use of specific antibodies, formation of an enzyme product, or disappearance of an enzyme substrate. For example, an enzyme assay may be used to determine the activity of the polypeptide as described herein.

The resulting polypeptide variants may be recovered by methods known in the art. For example, the polypeptide may be recovered from the nutrient medium by conventional procedures including, but not limited to, centrifugation, filtration, extraction, spray-drying, evaporation, or precipitation.

The polypeptide variants may be purified by a variety of procedures known in the art including, but not limited to, chromatography (*e.g.*, ion exchange, affinity, hydrophobic, chromatofocusing, and size exclusion), electrophoretic procedures (*e.g.*, preparative isoelectric focusing), differential solubility (*e.g.*, ammonium sulfate precipitation), SDS-PAGE, or extraction (see, *e*.*g*., Protein Purification, J.-C. Janson and Lars Ryden, editors, VCH Publishers, New York, 1989).

### Screening of Nucleic Acid Variants

The screening method to be used for identifying positive variants depend on the desired improved property of the polypeptide variant or variant of the DNA sequence in question. The improved property of interest can be, but is not limited to, thermostability, thermolability, protease-resistance, pH optimum, pH stability, altered substrate specificity, and increased promoter activity.

The resulting variant DNA sequences (*i*.*e*. shuffled DNA sequences), will have a number of nucleotide(s) exchanged, which results in replacement of at least one amino acid within the corresponding polypeptide variant, when compared with the parent polypeptide. It is to be understood that silent mutations are also contemplated (*i*.*e*. nucleotide exchange which does not result in changes in the amino acid sequence).

If, for instance, the polypeptide in question is an enzyme and the desired improved functional property is the wash performance, the screening may conveniently be performed by use of a filter assay based on the following principle: The recombination host cell is incubated on a suitable medium and under suitable conditions for the enzyme to be secreted, the medium being provided with a double filter comprising a first protein-binding filter and on top of that a second filter exhibiting a low protein binding capability. The recombination host cell is located on the second filter. Subsequent to the incubation, the first filter comprising the enzyme secreted from the recombination host cell is separated from the second filter comprising said cells. The first filter is subjected to screening for the desired enzymatic activity and the corresponding microbial colonies present on the second filter are identified.

The filter used for binding the enzymatic activity may be any protein binding filter *e.g.* nylon or nitrocellulose. The top filter carrying the colonies of the expression organism may be any filter that has no or low affinity for binding proteins *e.g.* cellulose acetate. The filter may be pre-treated with any of the conditions to be used for screening or may be treated during the detection of enzymatic activity.

The enzymatic activity may be detected by a dye, fluorescence, precipitation, pH indicator, IR-absorbance or any other known technique for detection of enzymatic activity.

The detecting compound may be immobilized by any immobilizing agent *e.g.* agarose, agar, gelatine, polyacrylamide, starch, filter paper, cloth; or any combination of immobilizing agents.

In the case of variants of a DNA sequence, the variant sequences can be subjected to PCR, isolated, and sequenced using conventional methods to ascertain the nature of the changes in the sequence. Alternately, a desired change in a DNA sequence may be screened for any cell phenotype that it alters, such as plasmid copy number, protein expression level, level of antibiotic resistance, cell wall properties such as resistance to organic solvents or detergents, increased RNA stability, catalytic nucleic acid activity, nucleic acid binding to metals, chromatography supports, glass, etc.

In the case of promoter variants, the variant sequences can be fused to reporter genes such as GFP or GUS. The variants can then be screened using fluorescence or or any other known technique for detection of enzymatic activity.

### Genes Encoding Recombination Proteins

In the methods of the present invention, the filamentous fungal cell comprises a heterologous gene encoding a recombination protein. The gene encoding the recombination protein may be any isolated nucleic acid sequence encoding a recombination protein. The term "heterologous gene" is defined herein as a gene that encodes a recombination protein that is not native to the filamentous fungal cell; a native gene in which modifications have been made to alter the native sequence; or a native gene whose expression is quantitatively altered as a result of a manipulation by recombinant DNA techniques. For example, a native recombination protein may be recombinantly produced by, for example, placing a gene encoding the recombination protein under the control of a strong promoter.

The recombination protein promotes the recombination of the two or more regions of the DNA fragments with the corresponding homologous region in the DNA sequence to incorporate the DNA fragments therein by homologous recombination. In the methods of the present invention, any region that is homologous with the DNA sequence may be used.

In a first embodiment, the genes encode recombination proteins that have an amino acid sequence which has a degree of identity to SEQ ID NO:2, SEQ ID NO:4, or SEQ ID NO:6 of at least about 70%, preferably at least 75%, preferably at least about 80%, more preferably at least about 85%, even more preferably at least about 90%, most preferably at least about 95%, and even most preferably at least about 97% (hereinafter "homologous polypeptides"). In a preferred embodiment, the homologous recombination polypeptides have an amino acid sequence which differs by five amino acids, preferably by four amino acids, more preferably by three amino acids, even more preferably by two amino acids, and most preferably by one amino acid from SEQ ID NO:2, SEQ ID NO:4, or SEQ ID NO:6. For purposes of the present invention, the degree of identity between two amino acid sequences is determined by the Clustal method (Higgins, 1989, CABIOS 5: 151-153) using the LASERGENE™ MEGALIGN™ software (DNASTAR, Inc., Madison, WI) with an identity table and the following multiple alignment parameters: Gap penalty of 10 and gap length penalty of 10. Pairwise alignment parameters are Ktuple=1, gap penalty=3, windows=5, and diagonals=5.

Preferably, the recombination proteins comprise the amino acid sequence of SEQ ID NO:2, SEQ ID NO:4, or SEQ ID NO:6; or an allelic variant thereof; or a fragment thereof that has recombination activity. In a more preferred embodiment, the recombination protein comprises the amino acid sequence of SEQ ID NO:2, SEQ ID NO:4, or SEQ ID NO:6. In another preferred embodiment, the recombination protein consists of the amino acid sequence of SEQ ID NO:2, SEQ ID NO:4, or SEQ ID NO:6; or an allelic variant thereof; or a fragment thereof, wherein the recombination protein fragment has recombination activity.

The present invention also encompasses a recombination protein having the amino acid sequence of SEQ ID NO:2, SEQ ID NO:4, or SEQ ID NO:6.

A fragment of SEQ ID NO:2, SEQ ID NO:4 or SEQ ID NO:6 is a protein having one or more amino acids deleted from the amino and/or carboxy terminus of this amino acid sequence. Preferably, a fragment of SEQ ID NO:2 contains at least 300 amino acid residues, more preferably at least 315 amino acid residues, and most preferably at least 330 amino acid residues. Preferably, a fragment of SEQ ID NO:4 contains at least 500 amino acid residues, more preferably at least 520 amino acid residues, and most preferably at least 540 amino acid residues. Preferably, a fragment of SEQ ID NO:6 contains at least 720 amino acid residues, more preferably at least 750 amino acid residues, and most preferably at least 780 amino acid residues.

An allelic variant denotes any of two or more alternative forms of a gene occupying the same chromosomal locus. Allelic variation arises naturally through mutation, and may result in polymorphism within populations. Gene mutations can be silent (no change in the encoded recombination protein) or may encode recombination proteins having altered amino acid sequences. The allelic variant of a recombination protein is a recombination protein encoded by an allelic variant of a gene.

In a second embodiment, the genes encoding a recombination protein have a degree of identity to the recombination protein coding sequence of SEQ ID NO:1, SEQ ID NO:3 or SEQ ID NO:5 of at least about 70%, preferably at least about 75%, preferably at least about 80%, more preferably at least about 85%, even more preferably at least about 90%, most preferably at least about 95%, and even most preferably at least about 97% identity, which encode an active recombination protein; or allelic variants of SEQ ID NO:1, SEQ ID NO:3 or SEQ ID NO:5 which encode recombination protein fragments which have recombination activity. For purposes of the present invention, the degree of identity between two nucleic acid sequences is determined by the Wilbur-Lipman method (Wilbur and Lipman, 1983, Proceedings of the National Academy of Science USA 80: 726-730) using the LASERGENE™ MEGALIGN™ software (DNASTAR, Inc., Madison, WI) with an identity table and the following multiple alignment parameters: Gap penalty of 10 and gap length penalty of 10. Pairwise alignment parameters are Ktuple=3, gap penalty=3, and windows=20.

In a third embodiment, the genes encoding recombination proteins hybridize under very low stringency conditions, preferably low stringency conditions, more preferably medium stringency conditions, more preferably medium-high stringency conditions, even more preferably high stringency conditions, and most preferably very high stringency conditions with a nucleic acid probe which hybridizes under the same conditions with (i) SEQ ID NO:1, SEQ ID NO:3, or SEQ ID NO:5, (ii) the cDNA sequence contained in SEQ ID NO:1, SEQ ID NO:3, or SEQ ID NO:5, or a complementary strand of (i), or (ii), (J. Sambrook, E.F. Fritsch, and T. Maniatus, 1989, Molecular Cloning, A Laboratory Manual, 2d edition, Cold Spring Harbor, New York).

The nucleic acid sequence of SEQ ID NO:1, SEQ ID NO:3, or SEQ ID NO:5, as well as the amino acid sequence of SEQ ID NO:2, SEQ ID NO:4, or SEQ ID NO:6, may be used to design a nucleic acid probe to identify and clone DNA encoding recombination proteins having recombination activity from strains of different genera or species according to methods well known in the art. In particular, such probes can be used for hybridization with the genomic or cDNA of the genus or species of interest, following standard Southern blotting procedures, in order to identify and isolate the corresponding gene therein. Such probes can be considerably shorter than the entire sequence, but should be at least 15, preferably at least 25, and more preferably at least 35 nucleotides in length. Longer probes can also be used. Both DNA and RNA probes can be used. The probes are typically labeled for detecting the corresponding gene (for example, with ³²P, ³H, ³⁵S, biotin, or avidin). Such probes are encompassed by the present invention.

Thus, a genomic DNA or cDNA library prepared from such other organisms may be screened for DNA, which hybridizes with the probes described above and which encodes a recombination protein having recombination activity. Genomic or other DNA from such other organisms may be separated by agarose or polyacrylamide gel electrophoresis, or other separation techniques. DNA from the libraries or the separated DNA may be transferred to and immobilized on nitrocellulose or other suitable carrier material. In order to identify a clone or DNA which is homologous with SEQ ID NO:1, SEQ ID NO:3, or SEQ ID NO:5; or a subsequence thereof, the carrier material is used in a Southern blot. For purposes of the present invention, hybridization indicates that the nucleic acid sequence hybridizes to a labeled nucleic acid probe corresponding to the nucleic acid sequence shown in SEQ ID NO:1, SEQ ID NO:3, or SEQ ID NO:5, its complementary strand, or a subsequence thereof, under very low to very high stringency conditions. Molecules to which the nucleic acid probe hybridizes under these conditions are detected using X-ray film.

In a preferred embodiment, the nucleic acid probe is a nucleic acid sequence which encodes the recombination protein of SEQ ID NO:2, SEQ ID NO:4, or SEQ ID NO:6; or a subsequence thereof. In another preferred embodiment, the nucleic acid probe is SEQ ID NO:1, SEQ ID NO:3, or SEQ ID NO:5. In another preferred embodiment, the probe is the nucleic acid sequence encoding a recombination protein contained in plasmid pZL1rdhA13 that is contained in *Escherichia coli* NRRL B-30503. In another preferred embodiment, the probe is the nucleic acid sequence encoding the recombination protein contained in plasmid pZL1 rdhB6 that is contained in *Escherichia coli* NRRL B-30503. In another preferred embodiment, the probe is the nucleic acid sequence encoding a recombination protein contained in plasmid pZL1rdhD17 that is contained in *Escherichia coli* NRRL B-30505. In another preferred embodiment, the probe is the nucleic acid sequence encoding a recombination protein contained in plasmid pZL1rdhD10 that is contained in *Escherichia coli* NRRL B-30506.

For long probes of at least 100 nucleotides in length, very low to very high stringency conditions are defined as prehybridization and hybridization at 42°C in 5X SSPE, 0.3% SDS, 200 µg/ml sheared and denatured salmon sperm DNA, and either 25% formamide for very low and low stringencies, 35% formamide for medium and medium-high stringencies, or 50% formamide for high and very high stringencies, following standard Southern blotting procedures.

For long probes of at least 100 nucleotides in length, the carrier material is finally washed three times each for 15 minutes using 2 x SSC, 0.2% SDS preferably at least at 45°C (very low stringency), more preferably at least at 50°C (low stringency), more preferably at least at 55°C (medium stringency), more preferably at least at 60°C (medium-high stringency), even more preferably at least at 65°C (high stringency), and most preferably at least at 70°C (very high stringency).

For short probes which are about 15 nucleotides to about 70 nucleotides in length, stringency conditions are defined as prehybridization, hybridization, and washing post-hybridization at 5°C to 10°C below the calculated Tₘ using the calculation according to Bolton and McCarthy (1962, Proceedings of the National Academy of Sciences USA 48:1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml following standard Southern blotting procedures.

For short probes which are about 15 nucleotides to about 70 nucleotides in length, the carrier material is washed once in 6X SCC plus 0.1% SDS for 15 minutes and twice each for 15 minutes using 6X SSC at 5°C to 10°C below the calculated Tₘ.

In a fourth embodiment, the genes encode variants of the recombination protein having an amino acid sequence of SEQ ID NO:2, SEQ ID NO:4, or SEQ ID NO:6, comprising a substitution, deletion, and/or insertion of one or more amino acids.

The amino acid sequences of the variant recombination proteins may differ from the amino acid sequence of SEQ ID NO:2, SEQ ID NO:4, or SEQ ID NO:6, by an insertion or deletion of one or more amino acid residues and/or the substitution of one or more amino acid residues by different amino acid residues. Preferably, amino acid changes are of a minor nature, that is conservative amino acid substitutions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions which do not generally alter the specific activity are known in the art and are described, for example, by H. Neurath and R.L. Hill, 1979, In, The Proteins, Academic Press, New York. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly as well as these in reverse.

The genes encoding recombination proteins may be obtained from microorganisms of any genus. For purposes of the present invention, the term "obtained from" as used herein in connection with a given source shall mean that the recombination protein encoded by the nucleic acid sequence is produced by the source or by a cell in which the nucleic acid sequence from the source has been inserted.

The genes encoding recombination proteins may be obtained from any filamentous fungal source including, but not limited to, an *Acremonium, Aspergillus, Aureobasidium, Cryptococcus, Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium*, or *Trichoderma* strain.

In a preferred embodiment, the genes encoding recombination proteins are obtained from a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Trichoderma harzianum, Trichoderma koningii, Trichoderma longibrachiatum, Trichoderma reesei,* or *Trichoderma viride* strain.

In another preferred embodiment, the genes encoding recombination proteins are obtained from an *Aspergillus aculeatus, Aspergillus awamori, Aspergillus foetidus, Aspergillus japonicus, Aspergillus nidulans, Aspergillus niger*, or *Aspergillus oryzae* strain.

In a more preferred embodiment, the genes encoding recombination proteins are obtained from *Aspergillus oryzae*.

It will be understood that for the aforementioned species, the invention encompasses both the perfect and imperfect states, and other taxonomic equivalents, *e.g.*, anamorphs, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL).

Furthermore, such genes encoding recombination proteins may be identified and obtained from other sources including microorganisms isolated from nature (*e*.*g.*, soil, composts, water, etc.) using the above-mentioned probes. Techniques for isolating microorganisms from natural habitats are well known in the art. The gene may then be derived by similarly screening a genomic or cDNA library of another microorganism. Once a gene encoding a polypeptide has been detected with the probe(s), the sequence may be isolated or cloned by utilizing techniques which are known to those of ordinary skill in the art (see, *e.g.*, Sambrook *et al*., 1989, *supra).*

In a most preferred embodiment, the gene encoding the recombination protein is set forth in SEQ ID NO:1. In another most preferred embodiment, the gene is the sequence contained in plasmid pZL1rdhA13 that is contained in *Escherichia coli* NRRL B-30503. In another most preferred embodiment, the gene is set forth in SEQ ID NO:3. In another most preferred preferred embodiment, the gene is the sequence contained in plasmid pZL1 rdhB6 that is contained in *Escherichia coli* NRRL B-30503. In another most preferred embodiment, the gene is set forth in SEQ ID NO:5. In another most preferred preferred embodiment, the gene is the sequence contained in plasmid pZL1rdhD17 that is contained in *Escherichia coli* NRRL B-30505. In another most preferred embodiment, the gene is set forth in SEQ ID NO:7. In another most preferred embodiment, the gene is the sequence contained in plasmid pZL1rdhD10 that is contained in *Escherichia coli* NRRL B-30506.

The present invention also relates to mutant genes encoding recombination proteins comprising at least one mutation in the recombination protein coding sequence of SEQ ID NO:1, SEQ ID NO:3, or SEQ ID NO:5 in which the mutant gene encodes a polypeptide which consists of SEQ ID NO:2, SEQ ID NO:4, or SEQ ID NO:6, respectively.

The techniques used to isolate or clone a gene are known in the art and include isolation from genomic DNA, preparation from cDNA, or a combination thereof. The cloning of the genes from such genomic DNA can be effected, *e.g.*, by using the well-known polymerase chain reaction (PCR) or antibody screening of expression libraries to detect cloned DNA fragments with shared structural features. See, *e.g.*, Innis et al., 1990, PCR: A Guide to Methods and Application, Academic Press, New York. Other nucleic acid amplification procedures such as ligase chain reaction (LCR), ligated activated transcription (LAT) and nucleic acid sequence-based amplification (NASBA) may be used.

In the methods of the present invention, the genes encoding recombination proteins are preferably overexpressed. Overexpression of these genes can be accomplished by multiple insertions of the genes in the genome of the filamentous fungal host cell and/or by substituting heterologous control sequences for the native control sequences in the gene, *e.g.*, a strong promoter.

The present invention is further described by the following examples which should not be construed as limiting the scope of the invention.

### Examples

### Example 1: Aspergillus oryzae genomic DNA extraction

*Aspergillus oryzae* HowB101, *Aspergillus oryzae* HowB430, or *Aspergillus oryzae* HowB425 was grown in 25 ml of 0.5% yeast extract-2% glucose (YEG) medium for 24 hours at 37°C and 250 rpm. Mycelia were then collected by filtration through Miracloth (Calbiochem, La Jolla, CA) and washed once with 25 ml of 10 mM Tris-1 mM EDTA (TE) buffer. Excess buffer was drained from the mycelia preparation which was subsequently frozen in liquid nitrogen. The frozen mycelia preparation was ground to a fine powder in an electric coffee grinder, and the powder was added to a disposable plastic centrifuge tube containing 20 ml of TE buffer and 5 ml of 20% w/v sodium dodecylsulfate (SDS). The mixture was gently inverted several times to ensure mixing, and extracted twice with an equal volume of phenol:chloroform:isoamyl alcohol (25:24:1 v/v/v). Sodium acetate (3 M solution) was added to the extracted sample to a final concentration of 0.3 M followed by 2.5 volumes of ice cold ethanol to precipitate the DNA. The tube was centrifuged at 15,000 x g for 30 minutes to pellet the DNA. The DNA pellet was allowed to air-dry for 30 minutes before resuspension in 0.5 ml of TE buffer. DNase-free ribonuclease A was added to the resuspended DNA pellet to a concentration of 100 µg per ml and the mixture was then incubated at 37°C for 30 minutes. Proteinase K (200 µg/ml) was added and the tube was incubated an additional one hour at 37°C. Finally, the sample was extracted twice with phenol:chloroform:isoamyl alcohol and the DNA precipitated with ethanol. The precipitated DNA was washed with 70% ethanol, dried under vacuum, resuspended in TE buffer, and stored at 4°C.

### Example 2: PCR Amplification of a portion of the Aspergillus oryzae rdhA gene

A portion of the *Aspergillus oryzae rdhA* (rad51 homolog A) gene was amplified by hemi-nested degenerate PCR. The first amplification employed degenerate primers 971514 and 971515, shown below, coding for amino acids DNVAYAR and MFNPDPK. Primer 971514 (DNVAYAR): 5'-GAYAAYGTIGCITAYGCNMG-3' (SEQ ID NO:7) Primer 971515 (MFNPDPK): 5'-TTIGGRTCNGGRTTRAACAT-3' (SEQ ID NO:8)

The amplification reactions (30 µl) were prepared using *Aspergillus oryzae* HB101 genomic DNA as template with the following components: PCR buffer II (Perkin Elmer, Branchburg, NJ), 0.25 mM dNTPs, 0.8 µg of *Aspergillus oryzae* HowB101 genomic DNA, 6.4 µM primer 971514, 3.2 µM primer 971515, and 1.5 units of *Taq* DNA polymerase (Perkin Elmer, Branchburg, NJ). Before amplification, the template DNA was denatured in a boiling water bath for 5 minutes and quick-cooled on ice. The reaction was initiated by adding *Taq* DNA polymerase to the other reaction components at 72°C. The reactions were incubated in a Perkin-Elmer Model 480 Thermal Cycler programmed as follows: 35 cycles each for 20 seconds at 94°C, 30 seconds at 66°C, 60 seconds ramping from 66 to 50°C, and 60 seconds at 72°C (5 minute final extension). The reaction products were isolated on a 1.6% agarose gel using 40 mM Tris base-20 mM sodium acetate-1 mM disodium EDTA (TAE) buffer where a 300 bp product band was excised from the gel and purified using a QIAquick Gel Extraction Kit (QIAGEN, Chatsworth, CA) according to the manufacturer's instructions.

One-tenth of the isolated 300 bp product was amplified under the same conditions described above except that primer 971516, shown below, was used in place of primer 971515.
Primer 971516 (NQVVAQV): 5'-ACYTGIGCIACNACYTGRTT-3' (SEQ ID NO:9)
The products were fractionated as before and a band at approximately 260 bp was excised and purified as described for the 300 bp product.

The purified PCR product was subsequently subcloned using the TOPO TA Cloning kit (Invitrogen, Carlsbad, CA) according to the manufacturer's instructions and the DNA sequence was determined using M13 Forward (-20) Primer (Invitrogen, Carlsbad, CA). DNA sequence analysis of the 260 bp *rdhA* gene segment showed that the amplified gene segment encoded a portion of the corresponding *Aspergillus oryzae rdhA* gene.

### Example 3: Isolation of a full-length Aspergillus oryzae rdhA genomic clones

Genomic DNA libraries were constructed using the bacteriophage cloning vector λZipLox (Life Technologies, Gaithersburg, MD) with *E. coli* Y1090ZL cells (Life Technologies, Gaithersburg, MD) as a host for plating and purification of recombinant bacteriophage and *E. coli* DH10Bzip (Life Technologies, Gaithersburg, MD) for excision of individual pZL1 clones containing the *rdhA* gene.

*Aspergillus oryzae* HowB425 genomic DNA was partially digested with Tsp509I and size-fractionated on 1% agarose gels. DNA fragments migrating in the size range 3-7 kb were excised and eluted from the gel using Prep-a-Gene reagents (BioRad Laboratories, Hercules, CA). The eluted DNA fragments were ligated with *Eco*RI-cleaved and dephosphorylated AZipLox vector arms (Life Technologies, Gaithersburg, MD), and the ligation mixtures were packaged using commercial packaging extracts (Stratagene, La Jolla, CA). The packaged DNA libraries were plated and amplified in *Escherichia coli* Y1090ZL cells (Life Technologies, Gaithersburg, MD).

The *Aspergillus oryzae* HowB425 DNA library was plated on NZCYM agar plates. Plaque lifts (Maniatis et al., 1982, Molecular Cloning, A Laboratory Manual, Cold Spring Harbor Press, Cold Spring Harbor, New York) were performed on approximately 40,000 pfu and the DNA was fixed onto membranes by heating at 80°C for two hours. The membranes were soaked for 30 minutes at 65°C in a hybridization solution containing 6X SSPE and 7.0% SDS.

The subcloned *rdhA* product of the PCR amplification described in Example 2 was excised from the vector pCR2.1-TOPO by digestion with *Eco*RI. Approximately 28 ng was random-primer labeled using a Stratagene Prime-It II Kit (Stratagene, La Jolla, CA) according to the manufacturer's instructions and used to probe the approximately 40,000 pfu of the *Aspergillus oryzae* genomic library constructed from *Aspergillus oryzae* strain HowB425 in the vector λZipLox. The radiolabeled *rdhA* gene fragment was then denatured by adding sodium hydroxide to a final concentration of 0.5 M, and added to the hybridization solution at an activity of approximately 1 x 10⁶ cpm per ml of hybridization solution. The mixture was incubated overnight at 65°C in a shaking water bath. Following incubation, the membranes were washed two times in 0.2X SSC with 0.2% SDS at room temperature and an additional two times in the same solution at 65°C. The membranes were then sandwiched between sheets of plastic and exposed to X-ray film for 18 hours at -80°C with intensifying screens (Kodak, Rochester, NY).

Fourteen plaques produced strong hybridization signals with the probe. Twelve of the plaques were picked from the plates and eluted overnight in 1 ml of SM (5.8 g/l NaCl, 2 g/l MgSO₄·7H₂O, 50 mM Tris-Cl, 0.01% gelatin). For plaque purification, the eluates were diluted 1:100 and 2 µl of the dilution was plated on NZCYM plates together with Y1090ZL plating bacteria. Plaque lifts were prepared and screened as described above, and individual plaques were picked into 0.5 ml of SM. The pZL1 plasmids were excised from the purified phagemid clones according to the protocol suggested by Life Technologies (Gaithersburg, MD). Colonies were inoculated into three ml of LB plus 50 µg/ml ampicillin medium and grown overnight at 37°C. Miniprep DNA was prepared from each of these clones using the Qiagen Bio Robot 9600 according to the manufacturer's protocol. The plasmids were digested with *Eco*RI and *Xba*I and fractionated by agarose gel electrophoresis in order to determine if the clones were identical and to determine their sizes. The nine unique clones had insert sizes ranging from 3.15 to 6.4 kb.

### Example 4: Characterization of the Aspergillus oryzae genomic clone encoding RDHA

DNA sequencing of each clone was performed with an Applied Biosystems Prism 377 DNA Sequencer using the BigDye Terminator Cycle Sequencing Ready Reaction kit (ABI, Foster City, CA) according to the manufacturer's instructions. Oligonucleotide sequencing primers were designed to complementary sequences in the pZL1 plasmid vector and were synthesized by Operon Technologies Inc., Alameda, CA. Contig sequences were generated by sequencing from the ends of each pZL1 clone and by sequencing subclones obtained from *Sal*I, *Pst*I, or *Hind*III digests of Clone #3, Clone #7, Clone #12, or Clone 13.

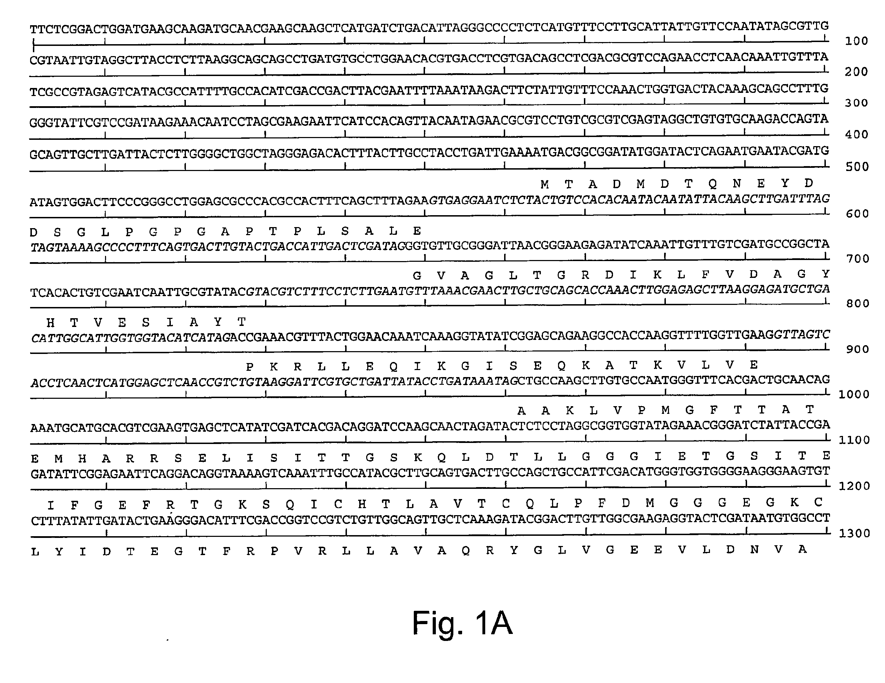

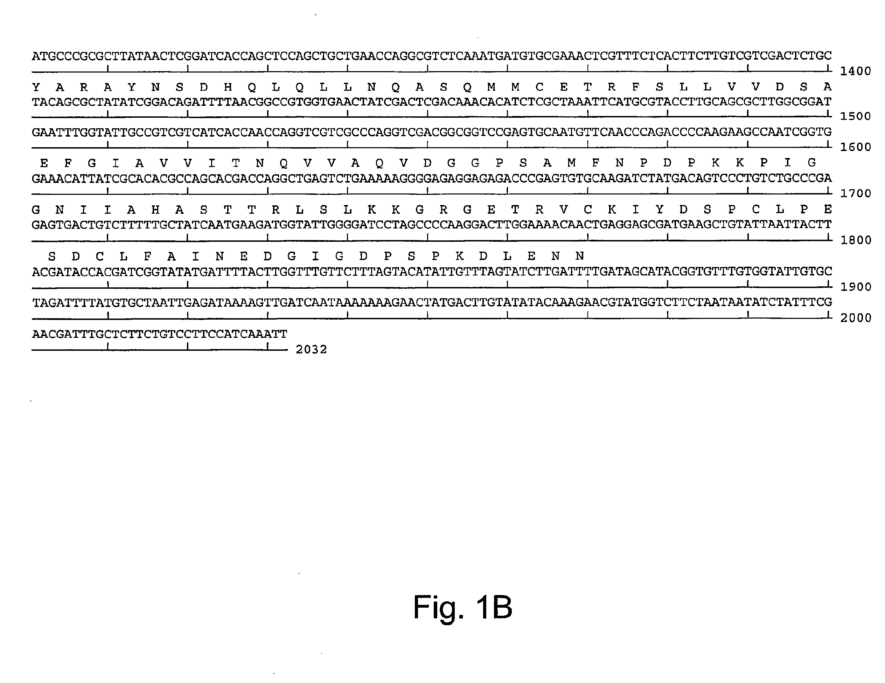

The 1.3 kb genomic region encompassing the coding sequence was sequenced to an average redundancy of 5.9. The nucleotide sequence and deduced amino acid sequence are shown in Figure 1 (SEQ ID NOs: 1 and 2). Sequence analysis of the cloned insert revealed a coding sequence of 1307 bp (excluding the stop codon) encoding a protein of 348 amino acids. The coding sequence is punctuated by three introns of 97 bp, 98 bp, and 68 bp. The G+C content of the coding sequence is 55.3%. The predicted RDHA polypeptide has a molecular mass of 37.6 kdal and an isoelectric point of 5.24. Using the Signal P software program (Nielsen et al., 1997, Protein Engineering 10: 1-6), no signal peptide was predicted (Y < 0.027).

A comparative alignment of the *Aspergillus oryzae* RDHA protein sequence with other sequences using the Clustal W algorithm in the Megalign program of DNASTAR, showed that the deduced amino acid sequence of the *Aspergillus oryzae* RDHA protein shares 98% identity to the deduced amino acid sequence of the UVSC protein of *Emericella nidulans* (accession number CAB02454).

Clone 13 was deposited as *E. coli* pZL1rdhA13 (NRRL B-30503) on July 27, 2001, with the Agricultural Research Service Patent Culture Collection, Northern Regional Research Center, 1815 University Street, Peoria, Illinois.

### Example 5: PCR Amplification of a portion of the Aspergillus oryzae rdhb gene

A portion of two *Aspergillus* oryzae genes homologous to the yeast rad52 gene were amplified by consensus/degenerate PCR (Rose et al., 1998, Nucleic Acids Res. 26: 1628-35). The amplification employed primers 980539 and 980540 shown below.
Primer 980539 (ANEVFGFNGW):
5'-CGAACGAAGTCTTCGGTTTYAAYGGNTGG-3' (SEQ ID NO:10)
Primer 980540 (KKEGTTDGMK):
5'-CTTCATGCCGTCGGTAGTNCCYTCYTTYTT-3' (SEQ ID NO:11)

The amplification reaction (30 µl) was prepared using *Aspergillus oryzae* HB425 genomic DNA as template with the following components: PCR buffer II (Perkin Elmer), 0.20 mM dNTPs, 0.4 µg of *Aspergillus oryzae* HowB425 genomic DNA, 5.0 µM primer 980539, 5.0 µM primer 980540, and 3.0 units of *Taq* DNA polymerase. Before amplification, the template DNA was denatured in a boiling water bath for 5 minutes and quick-cooled on ice. The reaction was initiated by adding *Taq* DNA polymerase to the other reaction components at 72°C. The reactions were incubated in a Stratagene Robocycler programmed for 35 cycles each for 30 seconds at 94°C, 60 seconds at 53°C, and 90 seconds at 72°C (7 minutes final extension).

The amplification products were fractionated as described above for the *rdhA* gene, and bands at about 350 and 300 bp were excised and cloned using the TOPO TA cloning kit according to the manufacturer's instructions and the DNA sequence was determined using T7 promoter primer. DNA sequence analysis of the 350 and 300 bp gene segments showed that the amplified gene segments encoded a portion of two closely related *Aspergillus oryzae* genes, hereafter designated as *rdhB* (rad52 homolog B) and *rdhC* (rad52 homolog C), respectively.

### Example 6: Isolation of a full-length Aspergillus oryzae rdhb genomic clone

Approximately 50 ng of the gel-purified ca. 300-bp product of the PCR amplification described in Example 3 was random-primer labeled using a Stratagene Prime-It II Kit according to the manufacturer's instructions and used to probe approximately 100,000 pfu of an *Aspergillus oryzae* genomic library constructed from *Aspergillus oryzae* strain HowB430 in the vector λZipLox using the same procedures described in Example 3.

Eleven hybridizing plaques were obtained, and four of these were purified, excised as pZL1 clones, and characterized as described in Example 3. The two unique clones obtained had insert sizes of approximately 3.9 kb and 6.3 kb. The larger clone was designated *E. coli* pZL1 clone #6 and submitted to sequence analysis (see Example 7).

### Example 7: Characterization of the Aspergillus oryzae genomic clone encoding RDHB

DNA sequencing of each clone was performed with an Applied Biosystems Prism 377 DNA Sequencer using the BigDye Terminator Cycle Sequencing Ready Reaction kit according to the manufacturer's instructions. Oligonucleotide sequencing primers were designed to complementary sequences in the pZL1 plasmid vector and were synthesized by Operon Technologies Inc., Alameda, CA. Contig sequences were generated using a transposon insertion strategy (Primer Island Transposition Kit, Perkin-Elmer/Applied Biosystems, Inc., Foster City, CA).

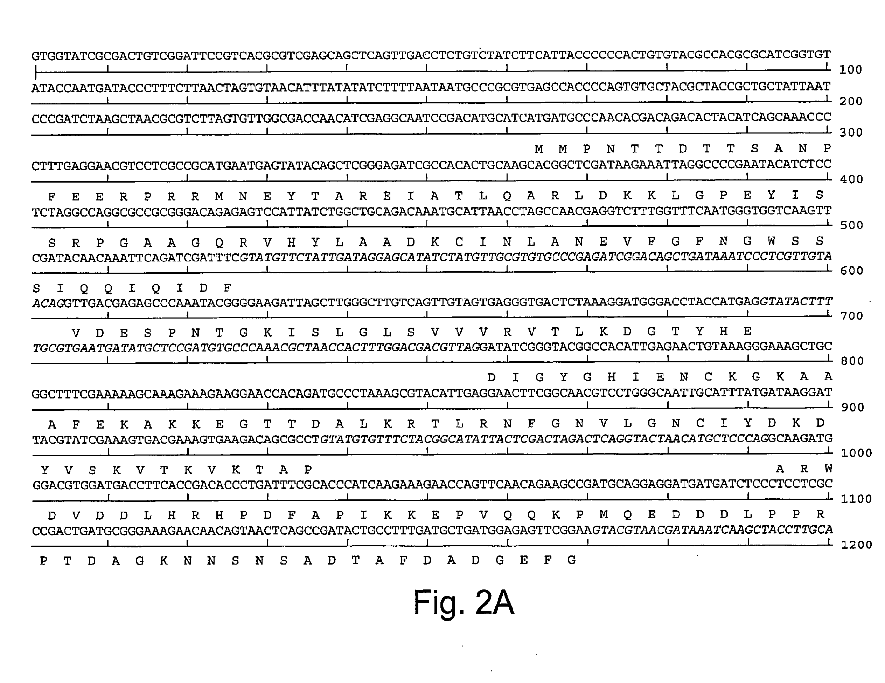

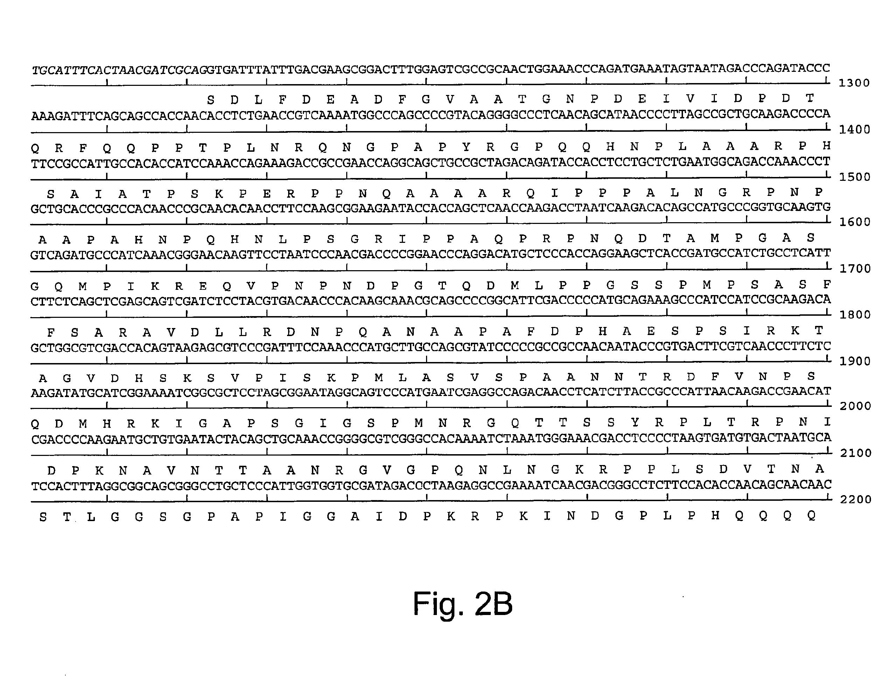

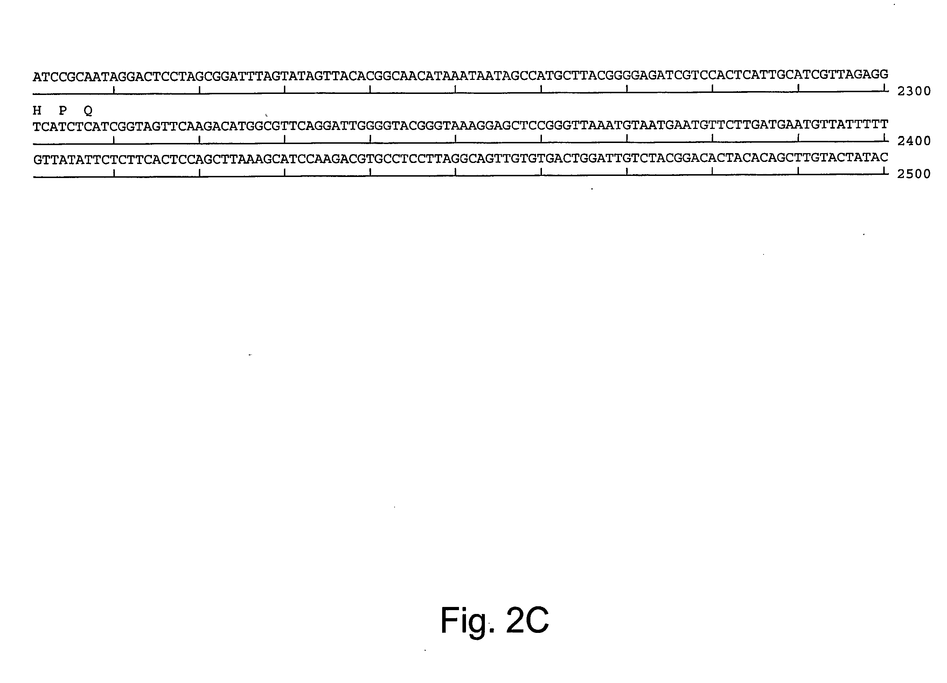

A 3257 bp genomic fragment was sequenced to an average redundancy of 4.7. The nucleotide sequence and deduced amino acid sequence are shown in Figure 2 (SEQ ID NOs:3 and 4). Sequence analysis of the cloned insert revealed a coding sequence of 1946 bp (excluding the stop codon) encoding a protein of 565 amino acids. The coding sequence is punctuated by four introns of 78 bp, 65 bp, 56, and 52 bp. The G+C content of the coding sequence is 51.8%. The predicted RDHB polypeptide has a molecular mass of 60.7 kdal and an isoelectric point of 8.64. Using the Signal P software program (Nielsen et al., 1997, Protein Engineering 10: 1-6), no signal peptide was predicted (Y < 0.043).

A comparative alignment of the *Aspergillus oryzae* RDHB protein sequence with other sequences using the Clustal W algorithm in the Megalign program of DNASTAR, showed that the deduced amino acid sequence of the *Aspergillus oryzae* RDHB protein shares 33% identity to the deduced amino acid sequence of the RAD22 protein of *Schizosaccharomyces pombe* (accession number P36592) and 33% identity to the RAD52 protein of *Saccharomyces cerevisiae* (accession number P06778).

Clone #6 was deposited as *E. coli* pZL1rdhB6 (NRRL B-30504) on July 27, 2001, with the Agricultural Research Service Patent Culture Collection, Northern Regional Research Center, 1815 University Street, Peoria, Illinois.

### Example 8. Construction of pRamB33

Intermediates pRaMB31 and pRaMB32 were constructed as follows: First, plasmid pUC19 was digested with *Nde*I plus *Pvu*II and the 2241 bp vector fragment, purfied by agarose gel electrophoresis, was ligated with the following synthetic linker which contains restriction sites for *Mun*I*, Pac*I, *Bam*HI, *Hin*dIII, *Pme*I, and *Mun*I while inactivating the Ndel cloning site:
5'-TATCAATTCTTAATTAAGGATCCAAGCTTGTTTAAACAATTC-3' (SEQ ID NO:12)
3'-AGTTAACAATTAATTCCTAGGTTCGAACAAATTTGTTAAC-5'- (SEQ ID NO:13)

The resulting pUC19-derivative was termed pRaMB31. Next, the *Aspergillus oryzae pgk* promoter and terminator regions (Genbank accession number D28484) as well as the bar gene from *Streptomyces hygroscopicus* (White et al. 1990, Nucleic Acids Res. 18: 1062) were amplified by PCR using the following primer pairs:
*Aspergillus oryzae pgk* promoter:
   5'-GATACATGTTATGGAGATGTTCTATCACACAAG-3' (contains *Afl*III site) (SEQ ID NO:14)
   5'-CAGGATCCTGCAGTATTGACTACTATGGT-3' (contains *Bam*HI site) (SEQ ID NO:15)
*Aspergillus oryzae pgk* terminator
   5'-CTGTTTAAACTGCAGGGAGGAACTGAAAAAGG-3' (contains *Pme*I site) (SEQ ID NO:16)
   5'-GTTAAGCTTGCGAAACGCAAATAATGTGTTG-3' (contains *Hind*III site) (SEQ ID NO:17)
*Streptomyces hygroscopicus bar* gene
   5'-GTTACATGTCTCCAGAACGACGCCCGGCGGACATC-3' (contains *Afl*III site) (SEQ ID NO:18)
   5'-TGAAGCTTCAGATCTCGGTGACGGGCAG-3' (contains *Hin*dIII site) (SEQ ID NO:19)

The amplification reactions (100 µl) was prepared using pMT1612 (which harbors the bar gene from *Streptomyces hygroscopicus -* EMBL accession number X05822) as template with the following components: 1X *Pwo* buffer (Roche Molecular Biochemicals, Indianapolis, IN), 0.25 mM dNTPs, 1.0 µM of each primer, and 5 units of *Pwo* DNA polymerase. The reactions were incubated in an Applied Biosystems thermocycler programmed for 1 cycle at 95°C for 3 minutes, 45°C for 2 minutes, and 67°C for 5 minutes followed by 30 cycles each at 95°C for 2 minutes; 45°C for 2 minutes; and 67°C for 2 minutes.

The PCR-amplified *pgk* terminator was digested with *Hin*dIII plus *Pme*I and the 635 bp product was purified by agarose gel electrophoresis, then ligated with pRaMB31 that had been cleaved with the same enzymes. The resulting intermediate plasmid was designated as pRaMB31.1. Next, the *pgk* promoter and bargene segments were digested with *Bam*HI plus *Afl*III and *Hin*dIII plus *Afl*III, respectively, and purified by electrophoresis. These two fragments were combined in a three-part ligation with the intermediate pRaMB31.1 that had been digested with *Bam*HI plus *Hin*dIII. The product of this ligation, pRaMB32 contained the *Sfreptomyces hygroscopicus* bargene under transcriptional control of the *Aspergillus oryzae* pgk promoter and terminator regions.

Next, the *Aspergillus oryzae niaA* promoter and alkaline protease (*alp*) terminator regions were amplified by PCR using high-fidelity Pwo polymerase (Boehringer-Mannheim) as above with the following primer pairs:
*Aspergillus oryzae niaA* promoter
   5'-GGTTAATTAACCGGCAGGGAAGGCCAATGAAAG-3'(contains *Afl*III site) (SEQ ID NO:20)
   5'-CCACGCGTATTTAAATGTCCGGGATGGATAGCACTGTGG-3' (contains *Pac*I site) (SEQ ID NO:21)
*Aspergillus oryzae alp* terminator
   5'-GGACGCGTGCGGCCGCGTACCAGGAGTACGTCGCAGG-3'(contains *Mlu*I site) (SEQ ID NO:22)
   5'-GGAGATCTGCAGCTGTGTACCAATAGAC-3' (contains *Bgl*II site) (SEQ ID NO:23)

The amplified *niaA* promoter segment was cloned directly into pUC118 (Yanisch-Perron et al., 1985, Gene 33: 103-119), which had been digested with *Sma*I and dephosphorylated. Similarly, the *alp* terminator region was subcloned into pCR-blunt (Invitrogen, Carlsbad, CA). The nucleotide sequences of both products were determined to ensure accuracy. The *niaA* promoter fragment was isolated by gel electrophoresis following cleavage with *Pac*I plus *Mlu*I, and the *alp* terminator segment was purified after digestion with *Mlu*I plus *Bgl*II. These purified fragments were mixed in a three-part ligation with pRaMB32 which had been previously cut with *Bam*HI plus *Pac*I. The resulting vector, designated as pRaMB33, contained (a) a selectable *bar* gene under the transcriptional control of the *pgk* promoter and terminator, and (b) unique *Not*I and Swal restriction sites located between the *niaA* promoter and *alp* terminator for directional cloning of cDNA or other coding regions of interest.

### Example 9: Construction of expression vector with niaA promoter

Plasmid pRaMB33 was digested with *Xba*I and *Nru*I to remove the Basta-resistance cassette. The remaining vector was isolated on a 0.8% agarose gel using TAE buffer where a 4.4 kb band was excised from the gel and purified using a QIAquick Gel Extraction Kit (QIAGEN, Chatsworth, CA) according to the manufacturer's instructions.

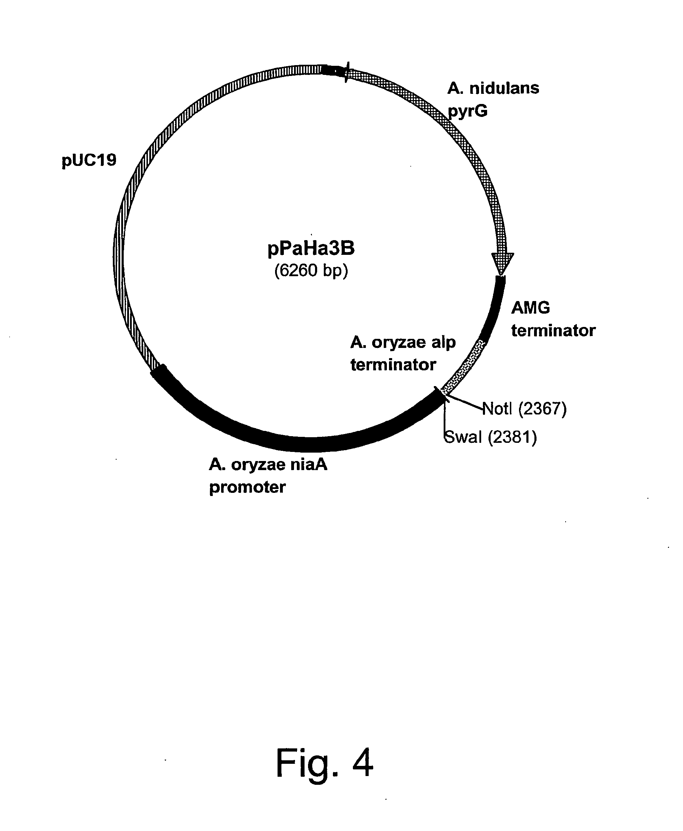

Plasmid pBANe13 (WO 97/47746) was digested with *Pme*I and *Nhe*I, and the fragment containing the *pyrG* gene and AMG terminator was similarly gel isolated and purified. The fragments were mixed together, blunt-ended using Klenow polymerase, ligated, and transformed into *E. coli* DH5α. Plasmid DNA was prepared from ten of the resulting transformants, and one displaying the correct restriction digest pattern was designated pPaHa3B (Figure 4). The *niaA* promoter is induced by nitrate.

### Example 10: Plasmids for inter-plasmid recombination assay

Plasmid pSMO122 (U.S. Patent No. 5,958,727) was digested with *Hin*dIII and treated with bacterial alkaline phosphatase. Plasmid Arp1 (Gems et al., 1991, Gene 98:61-67) was digested with *Hind*III and the digest fractionated on a 1.0% agarose gel in TAE buffer. A 5.8 kb fragment was excised from the gel and purified using a QIAquick Gel Extraction Kit (QIAGEN, Chatsworth, CA) according to the manufacturer's instructions. This fragment was ligated to the linearized pSMO122 plasmid and transformed into *Escherichia coli* DH5α. Plasmid DNA was prepared from transformants, and one, showing the correct fragment sizes after digestion with *Hin*dIII, was designated pHB217. The fragment contains the AMA1 replication region from *Emericella nidulans* and the *pyrG* gene from *Aspergillus oryzae.*

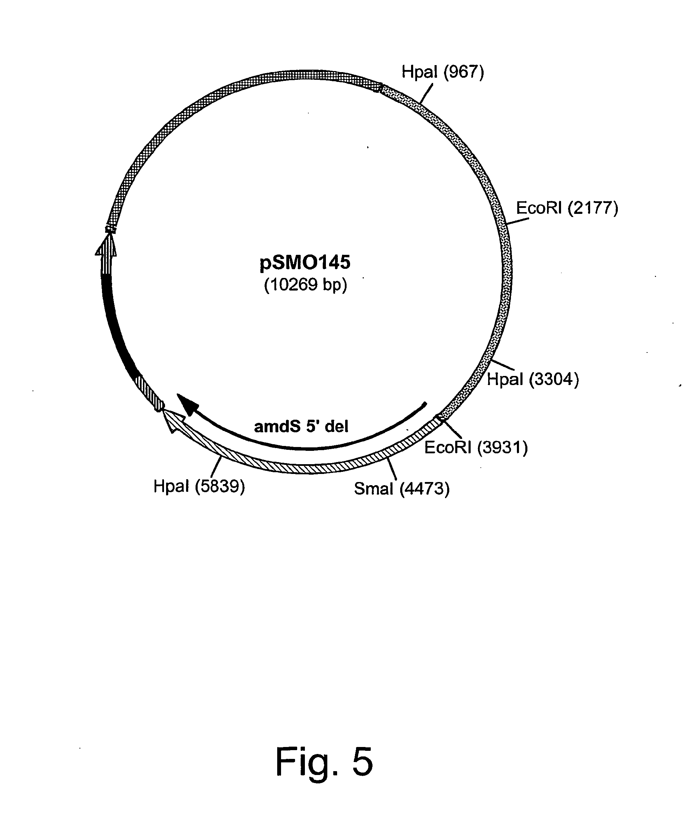

Plasmid pPaHa1-1 was digested *Nsi*I and the ends were made blunt using T4 DNA polymerase. The products were fractionated on a 0.8% agarose gel using TAE buffer and a 2 kb band was excised from the gel and purified using a QIAEX Gel Extraction Kit (QIAGEN, Chatsworth, CA) according to the manufacturer's instructions. The fragment was then inserted into the *Sma*I site of pHB217. The plasmid was designated pSMO145 (Figure 5). The plasmid carries a 220 bp deletion of the *Emericella nidulans amds* gene encompassing a portion of that gene's promoter, all of the 5'-untranslated region, and 132 bp of the coding region.

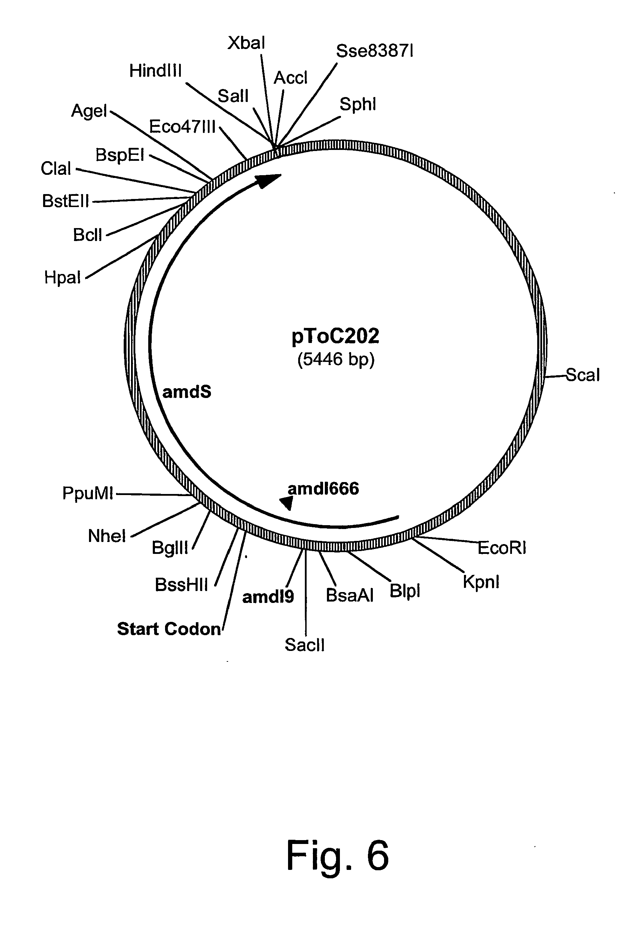

Plasmid pToC202 (Figure 6) was constructed to contain three up promoter mutations have identified within the *Aspergillus nidulans amds* gene: The 1666 and I66 up mutations have been described by Katz et al., 1990, Mol. Gen. Genet. 220: 373-376. The 19 mutation has been described by Davis and Hynes, 1989, TIG 5: 14 - 19 and by Todd, 1998, EMBO 17: 2042-2054. Plasmid pI66PI9 contains the *Aspergillus nidulans amds* with the two up promoter mutations I66 and I9. The *amds* allele of this plasmid was subcloned into pUC19 as a 2,7 kb Xbal fragment to form the plasmid pToC186C. (Yanisch-Perron et al., 1985, Gene 33 103-119).

Plasmid pMSX-6B1 contains the *Aspergillus nidulans amds* gene with the up promoter mutation I666. The *amds* allele of this plasmid was subcloned into pUC19 as a 2.7 kb *Xba*I fragment to form the plasmid pToC196. The I9 and I666 mutations were combined by inserting a 544 bp *Xma*I fragment from pToC186 harboring the I9 mutation into the 4903 bp *Xma*I fragment of pToC196 to form the plasmid pToC202 (Figure 6).

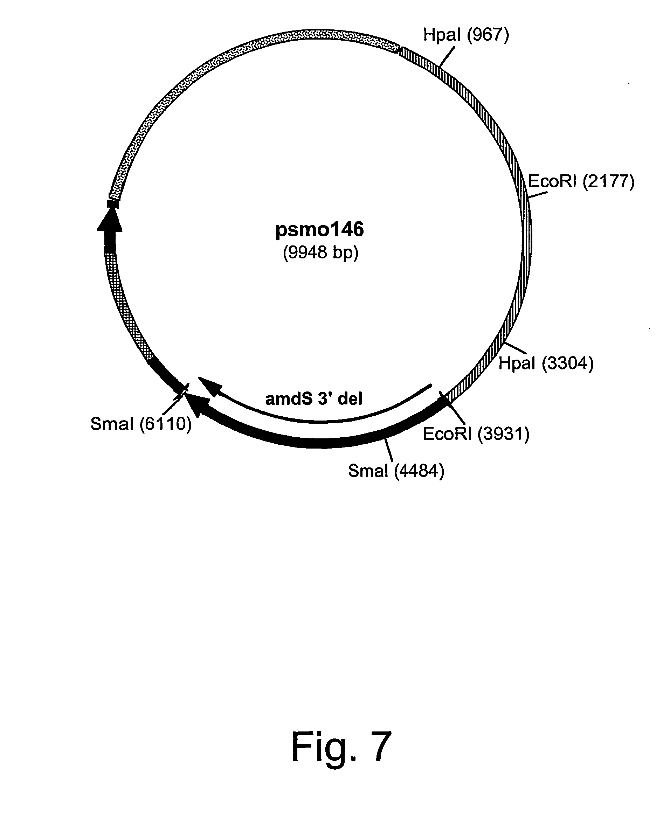

A 3' truncation of the *Emericella nidulans amds* gene was produced by digesting plasmid pToC202 with *Eco*RI and *Hpa*I, blunting with Klenow fragment, gel and purified using a QIAEX Gel Extraction Kit according to the manufacturer's instructions. The fragment was then inserted into the Smal site of pHB217. The resulting plasmid was designated pSMO146 (Figure 7). The promoter region of *amds* in this construct contained mutations that enhance promoter strength, allowing good growth on acetamide as the sole nitrogen source with a single copy of the gene.

### Example 11: Construction of Aspergillus oryzae rdhA and rdhB overexpression vectors

Plasmid pRaMB32 (described in Example 8) was digested with Pstl and Scal and fractionated on a 1 % agarose gel. The 2.8 kb band containing the *pgk* promoter, *bar* gene, and *pgk* terminator was excised and purifed with the Qiagen QIAEX II kit (QIAGEN, Chatsworth, CA) according to the manufacturer's instructions. Plasmid pBANe8 (U.S. Patent No. 5,958,727) was digested with *Nsi*I and dephosphorylated using 150 units of bacterial alkaline phosphatase followed by heat inactivation at 65°C for 1 hour. The digest was fractionated on a 1% agarose gel and the 5.0 kb band was excised and purified as above. The two fragments were ligated together and transformed into *E. coli* XL10 Gold cells (Stratagene, La Jolla, CA) according to the manufacturer's instructions. Plasmid DNA was prepared from transformants and screened for correctness by digesting with *Stu*I. One plasmid showing the correct digestion pattern was named pBANe44.

The 1.3 kb coding region of the *Aspergillus oryzae rdhA* gene was amplified by PCR from *E. coli* pZL1 clone #13. Primers incorporated *Swa*I, *Pac*I, or *Not*I sites for subsequent cloning and had the following sequence:
Sense Swa primer (980442):
   5'-CATTTAAATGATGACGGCGGATATG-3' (SEQ ID NO:24)
Antisense Pac primer (980359):
   5'-GTTAATTAATCAGTTGTTTTCCAAGTC-3' (SEQ ID NO:25)
Antisense *Not* primer (980451):
   5'-AGCGGCCGCTCAGTTGTTTTCCAAGTC-3' (SEQ ID NO:26)

The amplification reaction (50 µl) was composed of the following components: 1 X Pwo buffer (Roche Molecular Biochemicals, Indianapolis, IN), 0.2 mM dNTPs, 1.0 µM of each primer, 5 units of Pwo DNA polymerase, and approximately 60 ng of heat-denatured clone #13. The reactions were incubated in a Perkin-Elmer Model 480 Thermal Cycler programmed as follows: 22 cycles each at 94°C for 45 seconds; 55°C (52°C for first two cycles) for 45 seconds; 72°C for 90 seconds, and a final extension at 72°C for 7 minutes.

The products were fractionated on a 0.8% agarose gel using TAE buffer, and the predominant band at 1.3 kb was excised and purified using the QIAquick Gel Extraction Kit. The products were cloned into pCR^{®}2.1-TOPO (Invitrogen, Carlsbad, CA) after addition of 3' A-overhangs according to the manufacturer's suggested protocol.

The 1.3 kb insert from one randomly selected clone was removed by sequential digestion with *Swa*I and *Pac*I (TAKA promoter construct) or *Not*I (*niaA* promoter construct), gel purified, and ligated into similarly digested pBANe13, pBANe44, or pPaHa3B. The ligation mixtures were transformed into *E. coli* DH5α, and clones were screened for the correct inserts by digestion with *Swa*I and *Pac*I or *Swa*I and *Not*I. Miniprep DNA was sequenced from the ends of both inserts and shown to contain the full *rdhA* coding sequence. The constructs were designated pBANe13rad51, pSMO143, and pPaHa3Brad51.

The 1.96 kb coding region of *rdhB* was amplified essentially as described above using pZL1 clone #6 and the following primers:
Sense Swal primer (980924):
   5'-ATTTAAATGATGCCCAACACGACAGACA-3' (SEQ ID NO:27)
Antisense *Pac*I primer (980925):
   5'-TTAATTAACTATTGCGGATGTTGTTGCT-3' (SEQ ID NO:28)
Antisense *Not*I primer (980826):
   5'-GCGGCCGCCTATTGCGGATGTTGTTGC-3' (SEQ ID NO:29)

The annealing temperature for the PCR was 60°C (58°C for first two cycles). The DNA was subcloned into pCR-Blunt (Invitrogen, Carlsbad, CA), and miniprep DNA from clones containing the correct inserts was cloned into pBANe13, pBANe44, pRaMB33, or pPaHa3B as described above. The resulting constructs were named pBANe13rad52, pSMO145, pSMO155 and pPaHa3Brad52, respectively.

### Example 12: Construction of Aspergillus oryzae PaHa29

*Aspergillus oryzae hemA* 5'-deletion strain SE29-70 (Elrod et al., 2000, Current Genetics 38:291-298) was cultured on PDA plates containing 5-aminolevulinic acid and uridine to allow for loss of the *pyrG* gene. Spores from this plate were then plated on minimal plates containing fluoroorotic acid (FOA), uridine, and 5-aminolevulinic acid. Eight FOA-resistant colonies were spore purified on minimal plates containing 5-aminolevulinic acid and uridine. One of the FOA-resistant colonies was verified as having a *pyrG* deletion phenotype by lack of growth on minimal medium containing 5-aminolevulinic acid and by recovery of prototrophy after transformation of protoplasts (prepared as in Example 13) with an autonomously-replicating plasmid carrying the *pyrG* gene (pHB217). This strain was designated *Aspergillus oryzae* PaHa29.

### Example 13: Construction of Aspergillus oryzae HowB423 and HowB425

Protoplasts of *Aspergillus oryzae* HowB101 were transformed with pSMO143 or pSMO145 and plated on Basta transformation plates.

Protoplasts of *Aspergillus oryzae* strain HowB101were prepared according to the method of Christensen et al., 1988, BiolTechnology 6: 1419-1422. The transformation was conducted with protoplasts at a concentration of ca. 2x10⁷ protoplasts per ml. One hundred µl of protoplasts were placed on ice for 5 minutes with ca. 2 µg of the pSMO143 or pSMO145; 250 µl of 60% polyethylene glycol 4000, 10 mM Tris-HCl, pH 7.5, 10 mM CaCl₂ was added, and the protoplasts were incubated at 37°C for 30 minutes. Three mls of STC (1.2 M sorbitol, 10 mM Tris-HCl, pH 7.5, and 10 mM CaCl₂) was added. The solution was mixed gently and poured onto 150 mm Basta transformation plates (per liter: 0.52 g of KCI, 0.52 g of MgSO₄·7H₂O, 1.52 g of KH₂PO₄, 1 ml of trace metals described below, 342.3 g of sucrose, 25 g of Noble agar, 10 ml of 1 M urea, 10 ml of 5mg/ml Basta). The trace metals solution (1000X) was comprised of 22 g of ZnSO₄·7H₂O, 11 g of H₃BO₃, 5 g of MnCl₂·4H₂O, 5 g of FeSO₄·7H₂O, 1.6 g of CoCl₂·5H₂O, 1.6 g of (NH₄)₆Mo₇O₂₄, and 50 g of Na₄EDTA per liter. Plates were incubated 5-7 days at 34°C until colonies appeared. Putative transformants were spore purified twice on the same medium.

### Example 14: Construction of hemA 3'-deletion

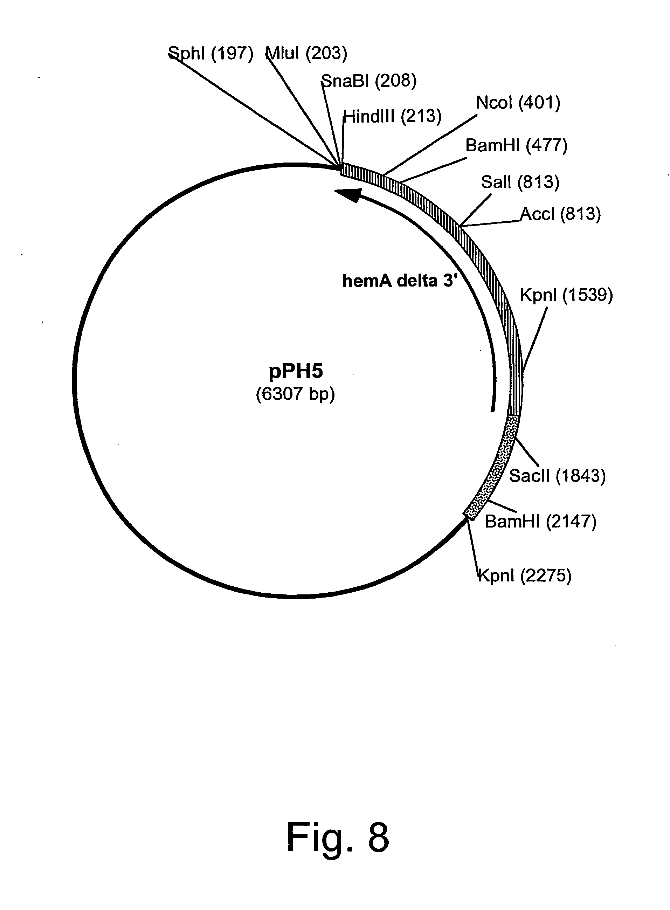

Plasmid pSE17 (WO 97/47746) was digested with *Hind*III to remove a portion of the *hemA* coding sequence and all of the 3' flanking sequence to produce a 6.3 kb fragment. The 6.3 kb fragment was run on a 0.8% agarose gel in TAE buffer, excised, and purifed using a QIAEX II Gel Extraction Kit (QIAGEN, Chatsworth, CA) according to the manufacturer's instructions. The fragment was recircularized by ligation and transformed into *E. coli* XL1-Blue cells to yield plasmid pPH5 (Figure 8).

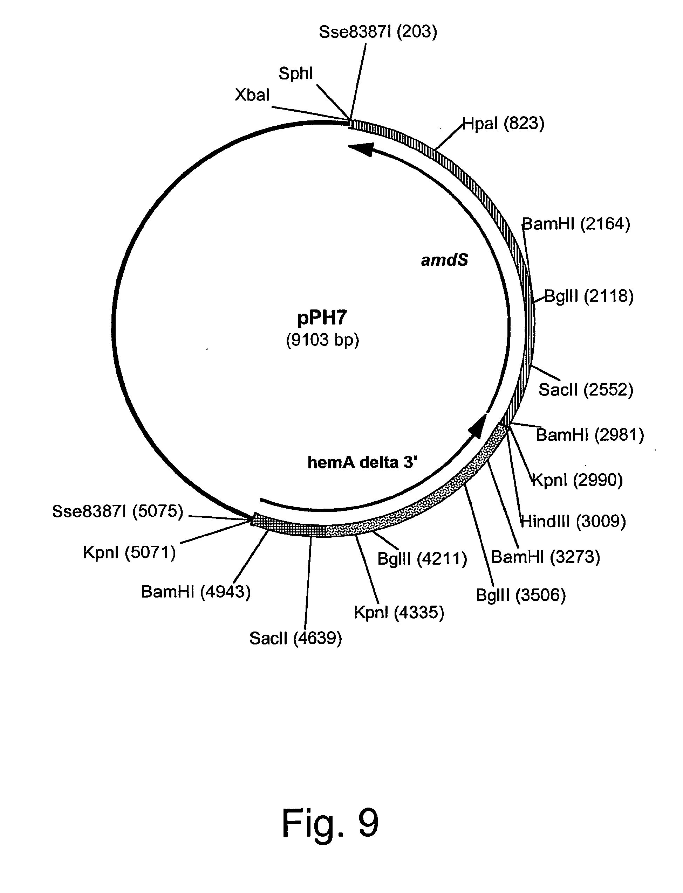

The *amds* gene from *Emericella nidulans* was isolated from pToC202 by digestion with *Eco*RI, Klenow fill-in, digestion with *Sph*I, and gel purification as above. The *amds* gene fragment was ligated into pPH5 digested with *Sph*I and *Sna*BI and similarly gel purified. The ligation mixture was transformed into *E. coli* XL1-Blue cells and plasmid DNA was prepared from twenty-four transformants. One plasmid DNA preparation showing the correct size fragments upon digestion with *Sac*I, *Kpn*I, or *Bam*HI was designated pPH7 (Figure 9).

### Example 15: Construction of hemAΔ strains overexpressing rdhA or rdhB

Protoplasts of *Aspergillus oryzae* PaHa29 were prepared as described in Example 13 and transformed with several µg of supercoiled pBANe13rad51, pBANe13rad52, pPaHa3Brad51, or pPaHa3Brad52, and plated on minimal medium containing 30 µg/ml 5-aminolevulinic acid. Individual transformants were spore purified on MMGAS (per liter: 0.5 g of NaCl, 0.5 g of MgSO₄·7H₂O, 2.0 g of KH₂PO₄, 1.2 g of K₂HPO₄, 1 ml of trace metals described below, 218 g of sorbitol, 20 g of Noble agar, 3.7 g of NH₄Cl, 0.1 ml of 1.0 M CaCl₂, and 10 ml of glycerol) plus 5-aminolevulinic acid (pBANe13 transformants) or MMASM (per liter: 0.5 g of NaCl, 0.5 g of MgSO₄·7H₂O, 2.0 g of KH₂PO₄, 1.2 g of K₂HPO₄, ml of trace metals described below, 20 g of sucrose, 20 g of Noble agar, 3.7 g of NH₄Cl, and 0.1 ml of 1.0 M CaCl₂) plus 5-aminolevulinic acid (pPaHa3B transformants). The trace metals solution (1000X) was comprised of 10 g of ZnSO₄·7H₂O, 0.4 g of CuSO₄·5H₂O, 0.04 g of Na₂B₄O₇·10H₂O, 0.7 g of MnSO₄·H₂O, 1.2 g of FeSO₄·7H₂O, 1.6 g of CoCl₂·5H₂O, and 0.8 g of Na₂MoO₂·2H₂O per liter. Respective transformants from the indicated plasmids were designated PaHa30, PaHa31, PaHa32, and PaHa33. Multiple transformants of each were generated and are designated by appending a number, e.g., PaHa31-2.

### Example 16: Effect of rdhA or rdhB overexpression on interplasmid recombination

*Aspergillus oryzae* grows very poorly using acetamide as the sole nitrogen source. Growth can be greatly enhanced by introduction of one or more copies of the *amds* gene from *Emericella nidulans.* This characteristic was used to monitor inter-plasmid recombination by co-transforming *Aspergillus oryzae* protoplasts with two autonomously-replicating plasmids, one carrying a deletion in the 5' region of *amds* (pSMO145), and the other carrying a deletion in the 3' region (pSMO146). Vigorous growth of transformants on acetamide can only be achieve following homologous recombination between the different plasmids to reconstitute at least one complete *amds* gene. Both plasmids also carry the *pyrG* gene in order to assess relative transformation efficiency.

The frequency of recombination in parental (*Aspergillus oryzae* HowB101) and *rdhA* (*Aspergillus oryzae* HowB443) or *rdhB* (*Aspergillus oryzae* HowB445) over-expression strains was assessed by co-transforming with both plasmids and plating on minimal medium with either nitrate or acetamide as the sole nitrogen sources (Table 1). The sucrose in these plates partially induces the TAKA promoter. Protoplasts of the indicated strains were prepared as described in Example 13 and co-transformed with 1.5 µg each of pSMO145 and pSMO146. A portion of the protoplasts was plated on minimal medium with either nitrate or acetamide as the sole nitrogen source, and the number of colonies was counted after six days of incubation at 37°C. Minimal nitrate plates contained, per liter, 6 g NaNO₃, 0.52 g KCl, 6.08 g KH₂PO₄, 0.5 g MgSO₄·7H₂O, 342.3 g sucrose, 10 g glucose, 0.004 g biotin, 20 g noble agar, and 1 ml of the trace metals described in Example 15. The medium was adjusted to pH 6.5 with NaOH. Minimal acetamide plates (COVE) contained, per liter, 10 mM acetamide, 15 mM CsCl, 0.52 g KCl, 1.52 g KH₂PO₄, 0.52 g MgSO₄·7H₂O, 342.3 g sucrose, 25 g noble agar, and 1 ml of trace metals. Transformation with either plasmid alone yielded no transformants on acetamide. Overall transformation efficiency of the over-expressing strains was somewhat reduced compared to the parental strain, however, inter-plasmid recombination frequencies were elevated by 14 and 26-fold in the *rdhA* and *rdhB* over-expression strains, respectively. In *Aspergillus oryzae* HowB445, plasmids in almost half of the total transformants presumably underwent at least one homologous recombination event that reconstituted a functional *amds* gene.

**Table 1.**

| Stimulation of interplasmid recombination in *rdhA* or *rdhB* overexpressing strains. | | | |
|---|---|---|---|
| | HowB101 | HowB443 | HowB445 |
| Transformants per ng, nitrate (*pyrG* selection) | 3.43 | 1.83 | 1.33 |
| Transformants per ng, acetamide (*amds* and *pyrG* selection) | 0.06 | 0.46 | 0.61 |
| Recombination frequency | 0.018 | 0.251 | 0.456 |
| Fold stimulation | 1.0 | 14.4 | 26.1 |

### Example 17: Effect of rdhA or rdhB overexpression on interchromosomal recombination

The *hemA* gene of *Aspergillus oryzae* codes for 5-aminolevulinate synthase, the first enzyme in heme biosynthesis. Mutants lacking this enzyme are unable to grow unless the medium is supplemented with 5-aminolevulinic acid. The native *hemA* gene in the *rdhB* overexpressing *Aspergillus oryzae* strain PaHa31-2 has been replaced by *hemA* carrying a 445-bp deletion in the 5' region of the coding sequence according to the procedure described in U.S. Patent No. 6,100,057, and thus this strain will not grow on minimal medium. Protoplasts of *Aspergillus oryzae* PaHa31-2 were transformed with 5 µg of plasmid pPH7 (Example 14) using the protocol described in Example 13. This plasmid carries the *hemA* gene with a deletion of all of the 3'-untranslated region and the last 382 bp of the coding region. The plasmid also contains the *E. nidulans amds* gene, and transformants were therefore initially selected on COVE plates (Example 16) containing 20 µg/ml of 5-aminolevulinic acid. One specific transformant that grew on COVE but still required 5-aminolevulinic acid for growth was spore purified twice and designated *Aspergillus oryzae* PaHa31-2.2.

Spores from transformant *Aspergillus oryzae* PaHa31-2.2 were plated on MMGU medium (MMGAS (Example 15) without sorbitol and with 10 mM urea in place of NH₄Cl) containing increasing concentrations of maltose in order to induce expression of *rdhB* in a controlled fashion. Growth on this medium can only occur if homologous recombination occurs between the single-copy chromosomal *hemA*Δ5'-gene and the chromosomally-integrated plasmid carrying the *hemA*Δ3' gene.

The results demonstrated that induction of *rdhB* expression greatly increased the frequency of homologous recombination. Concentrations of maltose as low as 0.02% had an obvious stimulatory effect. Most of the colonies were very slow to first appear and also grew very slowly, even when transferred to new plates not containing maltose. However, these colonies grew fairly normally when the medium was supplemented with 5-aminolevulinic acid, indicating that the complementation for *hemA* deficiency was only partial. Most likely this resulted from a gene conversion event that restored the coding region of *hemA* in one of the *hemA3'* gene copies, but failed to restore the 3'-untranslated region. This could result in relatively low-level expression and incomplete complementation.

The low concentrations of maltose required to achieve marked stimulation of *hemA*⁺ colony formation suggested that relatively mild induction of *rdhB* transcription was sufficient to maximally promote homologous recombination. Also, transcription from the TAKA promoter was not completely suppressed in glycerol, and thus the background levels of recombination seen on glycerol may at least partially reflect this lack of complete suppression. To overcome this, strains were created wherein *rdhA* (PaHa32) or *rdhB* (PaHa33) was expressed under control of the weaker *niaA* promoter. The 3'-deleted copy of *hemA* carried on plasmid pPH7 was introduced into these strains in a manner identical to that described above for creation of PaHa31-2.2. The specific transformants selected for testing were designated *Aspergillus oryzae* PaHa32-4.6 and PaHa33-5.1.

Approximately 2 x 10⁷ spores of PaHa32-4.6 or PaHa33-5.1 were plated on either MMASM (Example 15) or MMNSM (MMASM with 10 mM NaNO₃ in place of NH₄Cl). The former medium keeps the *niaA* promoter turned off and the latter medium induces the *niaA* promoter and hence stimulates transcription of the *rdhA* or *rdhB* gene. The appearance of colonies was monitored for 7 days. The results demonstrated that interchromosomal recombination is stimulated by an elevation in transcription of either *rdhA* or *rdhB.*

### Example 18: PCR Amplification of a portion of the Aspergillus oryzae rdhd gene

A portion of the *Aspergillus oryzae rdhD (rad54* homolog D) gene was amplified by nested degenerate PCR. The amplification employed primers 980057, 980058, 980059 and 980060 shown below.
Primer 980057:
5'- GAYCCIGAYTGGAAYCCNG -3' (SEQ ID NO:30)
Primer 980058:
5'- TTYTTYTGICCRTCNCKCCA -3' (SEQ ID NO:31)
Primer 980059:
5'- AAYTAYACICARACNYTNGA -3' (SEQ ID NO:32)
Primer 980060:
5'- ATITTYTCYTCDATNGTNC -3' (SEQ ID NO:33)

The first amplification reaction (30 µl) was prepared using *Aspergillus oryzae* HB101 genomic DNA as template with the following components: PCR buffer II (Perkin Elmer), 0.20 mM dNTPs, 0.4 µg of *Aspergillus oryzae* HowB101 genomic DNA, 5.0 µM primer 980059, 5.0 µM primer 980060, and 3.0 units of *Taq* DNA polymerase. Before amplification, the template DNA was denatured in a boiling water bath for 5 minutes and quick-cooled on ice. The reaction was initiated by adding *Taq* DNA polymerase to the other reaction components at 72°C. The reactions were incubated in a Stratagene Robocycler programmed as follows: 35 cycles each for 45 seconds at 94°C, 45 seconds at 39, 41, or 43°C, and 60 seconds at 72°C (7 minutes final extension). Reaction products were pooled, precipitated with 2 volumes of ethanol, dried, and dissolved in 10 µl of TE. The second amplification reaction (30 µl) was prepared using the product of the first amplification as template with the following components: PCR buffer II (Perkin Elmer), 0.20 mM dNTPs, 0.2 µl of template DNA, 5.0 µM primer 980057, 5.0 µM primer 980058, and 3.0 units of *Taq* DNA polymerase. Before amplification, the template DNA was denatured in a boiling water bath for 5 minutes and quick-cooled on ice. The reaction was initiated by adding *Taq* DNA polymerase to the other reaction components at 72°C. The reactions were incubated in a Stratagene Robocycler programmed as follows: 35 cycles each for 45 seconds at 94°C, 45 seconds at 46, 48, 50, or 52°C, and 60 seconds at 72°C (7 minutes final extension).

A portion of the reaction products was fractionated on a 3% agarose gel, and bands at about 70 bp were excised and purified using QIAquick with a final elution volume of 30 µl. Approximately 2 µl of this product was reamplified under the same PCR conditions and fractionated and purified in the same manner. The ca. 70 bp fragment was cloned using the TOPO TA cloning kit according to the manufacturer's instructions and the DNA sequence was determined using T7 promoter primer. DNA sequence analysis of the 68 bp gene segment showed that the amplified gene encoded a portion of the *Aspergillus oryzae rdhD* gene. The sequence from this clone was used to design a non-degenerate primer to be used for amplification of a larger region of the *rdhD* gene. The employed primer is shown below.
Primer 980866:
5'- AATGCTTGTTGATCAGCAG -3' (SEQ ID NO:34)

The amplification reaction (120 µl) was prepared using *Aspergillus oryzae* HB425 genomic DNA as template with the following components: PCR buffer II (Perkin Elmer), 0.25 mM dNTPs, 2.0 µg template DNA, 4.2 µM primer 980059, 0.4 µM primer 980866, and 5.0 units of *Taq* DNA polymerase. Before amplification, the template DNA was denatured in a boiling water bath for 5 minutes and quick-cooled on ice. The reaction was initiated by adding *Taq* DNA polymerase to the other reaction components at 72°C. The reactions were incubated in a Stratagene Robocycler programmed as follows: 30 cycles each for 45 seconds at 94°C, 45 seconds at 39, 41, 43, or 45°C, and 60 seconds at 72°C (7 minutes final extension). The ca. 250 bp product was fractionated on an agarose gel, excised, and purified using the QIAquick system. Three µl of the purified fragment was reamplified under the same PCR conditions for 25 cycles at an annealing temperature of 40°C, and the product was gel purified in the same manner. Direct sequencing of the PCR product using primer 980866 demonstrated that the gene fragment encoded a portion of the *rdhD* gene.

### Example 19: Isolation of partial-length Aspergillus oryzae rdhD genomic clones

Genomic libraries were prepared and plated as in Example 3. The PCR product of 232 bp described in Example 18 was radioactively labeled using the Stratagene Prime-It II kit according to the manufacturer's protocol with the exception that the random primers were replaced by 0.6 µM of primer 866. The labeled product was used to probe approximately 100,000 pfu of an *Aspergillus oryzae* genomic library constructed from *Aspergillus oryzae* strain HowB430 in the vector λZipLox using the same procedures described in Example 3.

Eleven hybridizing plaques were obtained, and four of these were purified, excised as pZL1 clones, and characterized as described in Example 3.

### Example 20: Characterization of the Aspergillus oryzae genomic clone encoding RDHD

DNA sequencing of each clone was performed with an Applied Biosystems Prism 377 DNA Sequencer using the BigDye Terminator Cycle Sequencing Ready Reaction kit according to the manufacturer's instructions. Oligonucleotide sequencing primers were designed to complementary sequences in the pZL1 plasmid vector and were synthesized by Operon Technologies Inc., Alameda, CA. Contig sequences were generated using a transposon insertion strategy (Primer Island Transposition Kit, Perkin-Elmer/Applied Biosystems, Inc., Foster City, CA).

A 5514 bp genomic fragment was sequenced to an average redundancy of 6.0, and includes sequences from all of the genomic clones. No single clone contained the entire gene, but overlapping pZL1 clones #10 and #17 together encompassed the entire gene. The nucleotide sequence and deduced amino acid sequence are shown in Figure 2. Sequence analysis of the cloned insert revealed a coding sequence of 2645 bp (excluding the stop codon) encoding a protein of 811 amino acids. Clone 10 contained nucleotides 390-2906 of SEQ ID NO:5 encoding amino acids 59-811 of SEQ ID NO:6, while clone 17 contained nucleotides 161-1749 of SEQ ID NO:5 encoding amino acids 1-459 of SEQ ID NO:6. The coding sequence is punctuated by four introns of 54 bp, 63 bp, 49, and 46 bp. The G+C content of the coding sequence (including introns) is 47.3%. The predicted RDHD polypeptide has a molecular mass of 99.2 kDa and an isoelectric point of 8.90. Using the Signal P software program (Nielsen et al., 1997, Protein Engineering 10: 1-6), no signal peptide was predicted (Y < 0.037).

A comparative alignment of the *Aspergillus oryzae* RDHD protein sequence with other sequences using the Clustal W algorithm in the Megalign program of DNASTAR, showed that the deduced amino acid sequence of the *Aspergillus oryzae* RDHD protein shares 74% identity to the deduced amino acid sequence of the MUS-25 protein of *Neurospora crassa* (accession number Q9P978).

Clones 10 and 17 were deposited as *E. coli* pZL1 rdhD17 (NRRL B-30505) and *E. coli* pZL1rdhD10 (NRRL B-30506) on July 27, 2001, with the Agricultural Research Service Patent Culture Collection, Northern Regional Research Center, 1815 University Street, Peoria, Illinois.

### Example 21: Construction of pHB241 and pHB242

pToC202 was digested with *Hind*III and then shrimp alkaline phosphatase (Roche, Indianapolis, IN) was added and incubated according to the manufacturer's instructions. The 5.4 kb fragment was agarose gel purified using Qiex II (QIAGEN, Chatsworth, CA.

pHB217 (Example 10) was digested with *Hind*III endonuclease. The 5.8 kb fragment containing the *Aspergillus oryzae* AMAI region was gel-isolated using Qiex II.

The 5.4 kb and 5.8 kb fragments were ligated for two hours and used to transform One Shot competent *E. coli* (Invitrogen, Carlsbad, CA) according to the manufacturer's instructions. The plasmid was designated pHB241.

Plasmid pHB241 was digested with both Nhel and *Bst*EII and the ends were made blunt using the Klenow fragment of DNA Polymerase I. The plasmid was closed by ligation and designated pHB242.

### Example 22: Construction of pPAHA1 Step 1

Plasmid pBANe6 (U.S. Patent No. 5,958,727) was digested with *Bam*HI and *Bse*RI and the ends were filled in with T4 DNA polymerase. A 6.75 kb fragment was gel-purified and isolated using the Qiaquick system. The fragment was ligated and transformed into *E. coli* Sure Cells (Strategene, La Jolla, CA) following manufactures instructions. The resulting plasmid was named pPAHA1 Step1, which contains a 222 bp deletion of the *amds* gene.

### Example 23: Gap Repair in Aspergillus oryzae

The following experiments were performed to determine whether *Aspergillus oryzae* functions as a host for gap repair and DNA shuffling.

Two *amds* deletion fragments were derived from *Hpa*I/*Eco*RI digestion of pToc202 and *Nsi*I digestion of pPaHa1 to yield 813 bp of 5' and 604 bp of 3' overlap with the gapped plasmid pHB241, respectively, and resolved using 1% agarose gels. The fragments were cut from the gel and the DNA was purified using the Qiaex II Gel-Extraction Kit as recommended by manufacturer (Qiagen, Chatsworth, CA). These two fragments were co-transformed with the *Nhe*I/*Bst*EII digested plasmid pHB241 or pHB242, a re-ligated gapped pHB241 linearized with *Bss*HII (Table 1) into *Aspergillus oryzae* host cells described in Table 1 using the same protoplast techniques described in Example 13. Transformants were selected on Cove + uridine for growth on acetamide (Example 16).

The results are shown below in Table 1.

**Table 1. Co-transformation of amds deletion fragments and linear gapped pHB241 and pHB242**

| **Experiment 1** | | |
|---|---|---|
| **Strain Transformants** | **DNA** | |
| *A. oryzae* HowB101 | pHB241 circular (100 ng) | 500 |
| | PHB242 linear (300 ng) | 0 |
| | PToC202 *Eco*RI/ *Hpa*I (E/H) (400 ng) + pPaHa1 *Nsi*I (N) (400 ng) | 0 |
| | pHB242 linear (300 ng) + pToC202 E/H, pToC202 N (400 ng each) | 55 |
| *A. oryzae* HowB445 | | |
| | pHB241 circular (100ng) | 75 |
| | pHB242 linear (300ng) + pToC202 E/H, pToC202 N (400ng each) | 25 |
| **Experiment 2** | | |
| *A. oryzae* HowB101 | | |
| | pHB241 circular (10 ng) | 77 |
| | pHB241 linear(150 ng) | 5 |
| | pToC202 E/H (200 ng) + pPaHa1 *Nsi*I(200 ng) | 0 |
| | pHB241 linear (150 ng) + pToC202 E/H, pToC202 N (200 ng each) | 89 |
| *Aspergillus oryzae* HowB445 | | |
| | pHB241 circular (10 ng) | 5 |
| | pHB241 linear(150 ng) | 0 |
| | pHB241 linear (150 ng) + pToC202 E/H, pToC202 N (200 ng each) | 45 |

The results of experiments 1 and 2 demonstrate that recombination occurred between the two deletion bearing fragments and the gapped plasmid, restoring a functional *amds* gene. Because of the different transformation frequencies exhibited between the wild type and *rdhB* strains, recombination efficiencies were calculated as the percentage of *amds* recombinants per ng normalized to the strains transformation efficiency. In experiment 1, a three-fold increase in recombination was achieved. In experiment 2, recombination in the *rdhB* over-expressing strain is increased approximately seven-fold. In all experiments, no *amds* recombinants were ever observed in transformations using the two *amds* deletion fragments alone. These results confirm that, as in yeast, DNA shuffling is a process that occurs in *Aspergillus oryzae.*

The total number of transformants was consistently higher in *Aspergillus oryzae* HowB101. This is most likely due to the higher transformation frequency of this strain. Increasing the amount of either the gapped plasmid or linear DNA's resulted in greater numbers of transformants. In several experiments, up to 500 recombinants were obtained. These high numbers suggest that sufficient yields of DNA shuffled recombinants could be obtained directly in *Aspergillus oryzae* for screening as opposed to heterologous systems such as yeast.

Also apparent is the higher proportion of *amds* recombinants obtained in the *rdhB* over-expression *Aspergillus oryzae* strain HowB 445. With transformation frequencies approximately 10-fold lower, the *rdhB* over-expression strain generated *amds* recombinants equal or greater than that obtained in a wildtype strain. From this it can be concluded that increased expression of recombination-associated genes stimulate homologous recombination in *Aspergillus oryzae.*

### Example 24: Construction of Plasmid pENi2229

Plasmid pENi2229 was constructed to incorporate additional restriction sites using several plasmids as described below. The final pENi2229 plasmid contains the AMA1 sequence for autonomous replication in *Aspergillus* species, a *pyrG* selectable marker for selection in filamentous fungi, a strong TAKA-npi promoter for the expression of proteins, a number of useful restriction sites downstream of the promoter, a termination sequence, an *E. coli* ori sequence for replication in bacteria, and a beta-lactamase expression cassette for selection in bacteria.

Using pENI2151 as template and PWO polymerase (as recommended by the manufacture), a PCR-reaction was made using primer 2120201J1 and 1288-taka.
1298-TAKA:
gcaagcgcgcgcaatacatggtgttttgatcat (SEQ ID NO:35)
210201J:

The PCR fragment (650 bp) and pENI2207 were digested with restriction endonucleases *Bss*HII and *Bgl*II. The vector and the PCR fragment were purified from a 1% agarose gel using Qiagen spin columns (Qiagen, Valencia, CA) following the manufacturer's instructions.

The PCR fragment and the vector were ligated, and transformed into the *E.coli* strain DH10B. Plasmid from one of the transformants was isolated (Qiagen, Valencia, CA) following the manufacturer's instructions, verified by DNA sequencing, and named pEN12229.

Plasmid pEN12151: Plasmids pENI1902 and pENI1861 were both digested with restriction endonuclease *Hind*III, and.pENI1902 was treated with phosphatase. Both a 2408 bp fragment from pENI1861 and digested vector pENI1902 were purified from 1% gel using Qiagen spin columns (Qiagen, Valencia, CA) following the manufacturer's instructions.

The fragment and the vector were ligated, and transformed into the *E.coli* strain DH10B. Plasmid from one of the transformants was isolated and named pENI2151.

Plasmid pENI2207: Plasmids pENI2151 and pENI2155 were digested with restriction endonucleases *Stu*I and *Sph*I. Both the 2004 bp fragment from pENI2155 and digested vector PENI2151 were purified from 1% gel using Qiagen spin columns (Qiagen, Valencia, CA) following the manufacturer's instructions. The fragment and the vector were ligated, and transformed into the *E.coli* strain DH10B. Plasmid from one of the transformants was isolated and named pENI2207.

Plasmid pENI1902 was made in order to have a promoter that works in both *E.coli* and *Aspergillus.* This was done by unique site elimination using the "Chameleon double stranded site-directed mutagenesis kit" as recommended by Stratagene^{®}.

Plasmid pENI1861 was used as template and the following primers with 5' phosphorylation were used as selection primers: 177996, 135640, and 135638. The 080399J 19 primer with 5' phosphorylation was used as mutagenic primer to introduce a -35 and -10 promoter consensus sequence (from *E.coli*) in the *Aspergillus* expression promoter. Introduction of the mutations was verified by sequencing.
177996: gaatgacttg gttgacgcgt caccagtcac (SEQ ID NO:37)
135640: cttattagta ggttggtact tcgag (SEQ ID NO:38)
135638: gtccccagag tagtgtcact atgtcgaggc agttaag (SEQ ID NO:39)
080399J 19: gtatgtccct tgacaatgcg atgtatcaca tgatataatt actagcaagg gaagccgtgcttgg (SEQ ID NO:40)

Plasmid pENI1861 was made in order to have the state of the art *Aspergillus* promoter in the expression plasmid, as well as a number of unique restriction sites for cloning. A PCR fragment (Approx. 620 bp) was made using plasmid pMT2188 (the construction of pMT2188 is described below) as template and the following primers:
051199J1: cctctagatctcgagctcggtcaccggtggcctccgcggccgctggatccccagttgtg (SEQ ID NO:41)
1298TAKA: gcaagcgcgcgcaatacatggtgttttgatcat (SEQ ID NO:42)

The fragment was cut with *BssH*II and *Bgl*II, and cloned into pENI1849 which was also cut with *Bss*HII and *Bgl*II. The cloning was verified by sequencing.

Plasmid pMT2188 was based on the *Aspergillus* expression plasmid pCaHj 483 (described in WO 98/00529), which consists of an expression cassette based on the *Aspergillus niger* neutral amylase II promoter fused to the *Aspergillus nidulans* triose phosphate isomerase non translated leader sequence (Pna2/tpi) and the *Aspergillus niger* amyloglycosidase terminater (Tamg). Also present on the pCaHj483 is the *Aspergillus* selective marker *amds* from *A. nidulans* enabling growth on acetamide as sole nitrogen source. These elements are cloned into the *E*. *coli* vector pUC19 (New England Biolabs). The ampicillin resistance marker enabling selection in *E. coli* of pUC19 was replaced with the URA3 marker of *Saccharomyces cerevisiae* that can complement a *pyrF* mutation in *E. coli,* the replacement was done in the following way:
The pUC19 origin of replication was PCR amplified from pCaHj483 with the primers:
   142779: 5' ttgaattgaaaatagattgatttaaaacttc-3' (SEQ ID NO:43)
   142780: 5' ttgcatgcgtaatcatggtcatagc-3' (SEQ ID NO:44)
Primer 142780 introduces a *Bbu*I site in the PCR fragment. The Expand™ PCR system (Roche Molecular Biochemicals, Basel, Switzerland) was used for the amplification following the manufacturers instructions for this and the subsequent PCR amplifications.
   The URA3 gene was amplified from the general *S. cerevisiae* cloning vector pYES2 (Invitrogen, Carlsbad, Ca, USA) using the primers:
   140288: 5' ttgaattcatgggtaataactgatat-3' (SEQ ID NO:45)
   142778: 5' aaatcaatctattttcaattcaattcatcatt-3' (SEQ I D NO:46)
Primer 140288 introduces an *Eco*RI site in the PCR fragment. The two PCR fragments were fused by mixing them and amplifying using the primers 142780 and 140288 in the splicing by overlap method (Horton et al., 1989, Gene 77: 61-68).

The resulting fragment was digested with *Eco*RI and *Bbu*I and ligated to the largest fragment of pCaHj 483 digested with the same enzymes. The ligation mixture was used to transform the *pyrF E.coli* strain DB6507 (ATCC 35673) made competent by the method of Mandel and Higa (Mandel and Higa, 1970. J. Mol. Biol. 45: 154). Transformants were selected on solid M9 medium (Sambrook et. al., 1989, Molecular cloning, a laboratory manual, 2nd edition, Cold Spring Harbor Laboratory Press) supplemented with 1 g/l casaminoacids, 500 microgram/l thiamine and 10 mg/l kanamycin.

A plasmid from a selected transformant was termed pCaHj527. ThePna2/tpi promoter present on pCaHj527 was subjected to site directed mutagenises by a simple PCR approach. Nucleotides 134-144 were altered from GTACTAAAACC to CCGTTAAATTT using the mutagenic primer 141223. Nucleotides 423-436 were altered from ATGCAATTTAAACT to CGGCAATTTAACGG using the mutagenic primer 141222. The resulting plasmid was designated pMT2188.
Primer 141223:
5' ggatgctgttgactccggaaatttaacggtttggtcttgcatccc-3' (SEQ ID NO:47)
Primer 141222:
5'-ggtattgtcctgcagacggcaatttaacggcttctgcgaatcgc-3' (SEQ ID NO:48)

Plasmid pENI1849 was made in order to truncate the *pyrG* gene to the essential sequences for *pyrG* expression, in order to decrease the size of the plasmid, thus improving transformation frequency. A PCR fragment (approx. 1800 bp) was made using pENI1299 (described in WO 00/24883, Example 1) as template and the following primers:
270999J8: tctgtgaggcctatggatctcagaac (SEQ ID NO:49)
270999J9: gatgctgcatgcacaactgcacctcag (SEQ ID NO:50)

The PCR-fragment was digested with *Stu*I and *Sph*I, and cloned into pENI1298 (described in WO 00/24883, Example 1), and also digested with *Stu*I and *Sph*I; the cloning was verified by sequencing.

Plasmid pENI2155 comprises a bad kozak region upstream of the *pyrG* gene, and is constructed as follows:
Using plasmid pENI1861 as template, and PWO polymerase (conditions as recommended by manufacturer), two PCR-reactions were made using primer 141200J1 and 270999J9 in the one PCR-reaction and primers 141200J2 and 290999J8 in another PCR-reaction:
141200J1: 5'-atcggttttatgtcttccaagtcgcaattg-3' (SEQ ID NO: 51)
141200J2: 5'-cttggaagacataaaaccgatggaggggtagcg-3' (SEQ ID NO:52)
270999J8: 5'-tctgtgaggcctatggatctcagaac-3' (SEQ ID NO:53)
270999J9: 5'-gatgctgcatgcacaactgcacctcag-3' (SEQ ID NO:54)

The PCR fragments were purified from a 1% agarose gel using QIAGEN™ spin columns. A second PCR-reaction was run using the two fragments as template along with the primers 270999J8 and 270999J9. The PCR-fragment from this reaction was purified from a 1% agarose gel as described; the fragment and the vector pENI1849 (containing a lipase gene as expression reporter) were cut with the restriction enzymes *Stu*I and *Sph*I*,* the resulting fragments were purified from a 1% agarose gel as described previously.

The purified fragments were ligated and transformed into the *E.coli* strain DH10B. Plasmid DNA from one of the transformants was isolated and sequenced to confirm the introduction of a mutated Kozak region: ggtttt**atg** (rather than the wildtype: gccaac**atg**). This plasmid was denoted: pENI2155.

### Example 25: Construction of pCW013

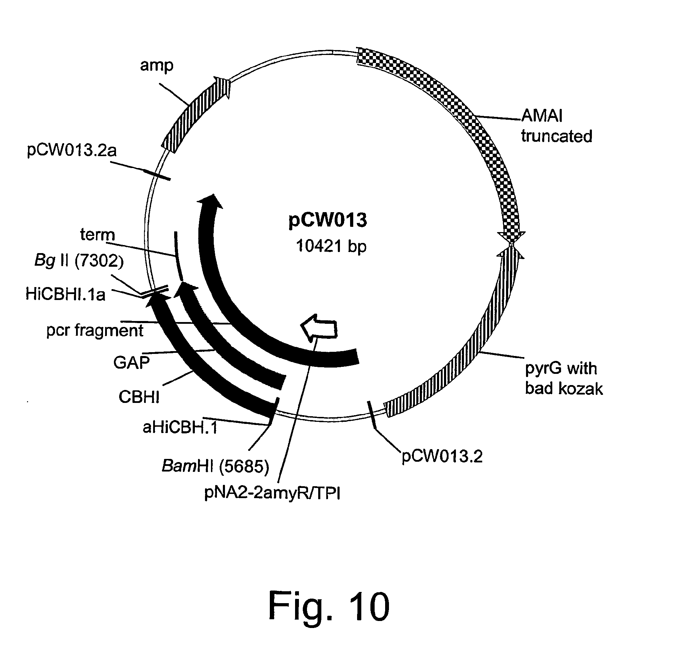

Plasmid pCW013 (Figure 10) was constructed from pENi2229 to obtain expression of a *Humicola insolens* cellobiohydrolase (CBHI) in *Aspergillus oryzae.* The coding sequence for *Humicola insolens* CBHI was amplified by PCR from pHD459b, which was created as described by Dalboge and Heldt-Hansen, 1994, Mol. Gen. Gene 243: 253-260, utilizing the screening procedure for glucanase detection. The PCR fragment containing the full-length cbh1 gene was subcloned into pENi2229 as a *Bam*HI/*Xma*I fragment. Construction of the pCW013 plasmid was accomplished as described below.

PCR fragments were extended with a *Bam*HI site on the 5' end of CBHI and an *Xma*I site on the 3' end using the following primers.
Primer 1: 5'-CGCGGATCCACCATGCGTACCGCCAAGTTCGCC-3' (SEQ ID NO:55)
Primer 2: 5'-GCCCCGGGTTACAGGCACTGAGAGTACCAG-3' (SEQ ID NO:56)

The amplification reactions (50 µl) contained the following components: 0.3 µg of pHD459b1 unit of PWO polymerase, 1X PWO polymerase buffer, 0.2 mM dNTPs, 50 pmol of primer 1, and 50 pmol of primer 2. The reactions were incubated in a Eppendorf Mastercycler (Eppendorf, Westbury, New York) programmed for 30 cycles each at 95°C for 30 seconds, 55°C for 30 seconds and 72°C for 1 minute.

The reaction products were then resolved on a 0.8% agarose gel and a 1605 bp product band was excised from the gel and purified using Amicon's Ultrafree DA Centrifugal Unit (Millipore, Bedford, MA) according to manufacturer's instructions. The purified product was then ligated and transformed using pCR4 Blunt TOPO Vector Kit (Invitrogen, Carlsbad, CA) following the manufacturer's instructions. The transformation was plated on 2XYT/ampicillin agar medium and grown overnight at 37°C. The 2XYT/ampicillin agar medium was composed per liter of per liter 16 g of tryptone, 10 g of yeast extract, 5 g of sodium chloride, and 15 g of Bacto agar supplemented with 100 µg of ampicillin per liter.

White colonies were picked into 3 ml of 2XYT/ampicillin medium and grown overnight at 37°C. Plasmid DNA was isolated from the cultures using Qiagen Qiabot Miniprep Station (Qiagen, Valencia, CA) following the manufacturer's instructions. The plasmid DNA was analyzed by restriction mapping to identify clones positive for CBHI insertion using restriction endonucleases *Bam*HI and *Xma*I. Once a clone was validated that there was successful insertion of the CBHI gene, the clone was sequenced for fidelity using BigDye Terminator Version 3 and analyzed using ABI 3700 DNA Analyzer (Foster City, CA) according the manufacturer's instructions.

The TOPO clone DNA containing confirmed CBHI sequence was digested with *Bam*HI and *Xma*I and the reaction product was then resolved on a 0.8% agarose gel and a 1605 bp product was excised and purified using Amicon's Ultrafree DA Centrifugal Unit (Amicon, Beverly, MA) following the manufacturer's instructions.

Plasmid pENi2229 was digested in the same manner with *Bam*HI and Xmal to create compatible ends with CBHI. The digestion product was resolved on a 0.8% agarose gel and an 8810 bp product was excised and purified using Amicon's Ultrafree DA Centrifugal Unit according to the manufacturer's instructions.

The *Bam*HI/X*ma*I CBHI gene fragment was ligated into the *Bam*HI/*Xma*I digested pENi2229 using Rapid DNA Ligation Kit (Roche, Indianapolis, IN) following the manufacturer's instructions. This ligation was then used to transform *E. coli* Sure Cells following the manufacturer's instructions. Colonies were selected, cultured, and plasmid was prepared as described above. The plasmid DNA was analyzed by restriction mapping to identify clones positive for CBHI insertion using *Bam*HI and *Xma*I. The positive colonies were designated pCW013.

### Example 26: Generation of gap repair fragments

To examine whether *Aspergillus oryzae* functions as a host for gap repair, the following experiments were performed. The DNA fragment containing the *cbh1* gene was derived by PCR using plasmid pCW013 as the template and the following primers that anneal 900 bp upstream and 1063 bp downstream of the gap generated from *Bam*HI/B*gl*II digestion of pCW013:
Primer 3: 5'-cgatctcgcagtcccgattcgcc-3' (SEQ ID NO:57)
Primer 4: 5'-tccgggagctgcatgtgtcagag-3' (SEQ ID NO:58)

Amplification reactions (50 µl) were composed of 0.5 µg of pCW013, 50 pmol of primer 3, 50 pmol of primer 4, 0.2 mM dNTP's, 1X *Taq* DNA polymerase buffer, and 2.5 Units of *Taq* DNA polymerase. The reactions were incubated in an Eppendorf Mastercycler programmed for 20 cycles each 94°C for 30 seconds, 55°C for 30 seconds, and 72°C for 3 minutes. The reaction products were purified using Qiaquick PCR Purification Kit (Qiagen, Valencia, CA) following the manufacturer's instructions.

Gapped pCW013 was prepared using *Bam*HI and *Bgl*II as follows: 37 µg of pCW013, 50 units of *Bam*HI, 50 units of *Bgl*II (Roche, Indianapolis, IN), and 1X Buffer A (Roche, Indianapolis, IN) were incubated for 3 hours at 37°C. The reaction product was then resolved on a 0.8% agarose gel where a 8816 bp product band was excised from the gel and purified using Amicon's Ultrafree DA Centrifugal Unit according to manufacturer's instructions.

### Example 27: Preparation and transformation of Aspergillus oryzae:

Protoplasts of *Aspergillus oryzae* Jal250 (WO 99/61651, Example 9) were prepared similarly as described in Example 13. Frozen protoplasts of *Aspergillus oryzae* Jal250 were thawed on ice. Gapped pCW013 and the cbh1 PCR fragment, at approximately a 1:3 molar ratio, respectively, were added to a 15 ml sterile polypropylene tube. One hundred µl of protoplasts were added and mixed gently. Two-hundred-fifty µl of PEG solution was then added to the DNA, mixed gently, and incubated at 37°C for 20 minutes. Three ml of STC was then added and mixed gently. Aliquots of 300 µl were removed and added to 20 ml of 50°C *pyrG* overlay (per liter: 0.5 g of NaCl, 0.5 g of MgSO₄·7H₂0, 2.0 g of KH₂PO₄, 1.2 g of K₂HPO₄, 1 ml of trace metals described below, 20 g of sucrose, 20 g of Noble agar, 3.7 g of NH₄Cl, and 0.1 ml of 1.0 M CaCl₂; the trace metals solution (1000X) was composed per liter of 10 g of ZnSO₄·7H₂O, 0.4 g of CuSO₄·5H₂O, 0.04 g of Na₂B₄O₇·10H₂O, 0.7 g of MnSO₄·H₂O, 1.2 g of FeSO₄·7H₂O, 1.6 g of CoCl₂·5H₂O, and 0.8 g of Na₂MoO₂·2H₂O) which was then poured on room temperature plates. These plates were allowed to solidify at room temperature and then incubated at 34°C for 3 days. The results are shown in Table 2.

**Table 2**

| | |
|---|---|
| Gapped pCW013 | 0 colonies |
| Gapped pCW013 + PCR fragment | 72 colonies |

The results indicated that reconstitution of an autonomously replicating plasmid required the presence of both the gapped pCW013 and the cbh1 PCR fragment.

Activity assays were performed to validate the fidelity of the repair. The transformants should contain an expression cassette encoding CBHI that was contained on the PCR fragment. These transformants were isolated and grown in 24 well plates containing 1 ml of ¼ strength MDU2BP medium containing maltose to induce the production of CBHI. The plates were incubated at 34°C for 4 days. Positive controls were six transformants containing intact pCW013, which are positive for CBHI production. The negative controls were six transformants containing pENi2229, which is negative for CBHI. The controls were obtained following the protoplasting and tranformation procedure described above, substituting 1 µg of plasmid DNA for the gapped plasmid/PCR fragment DNA mix. At 4 days, samples of the culture broth were assayed for CBHI activity.

CBHI activity was determined as follows. Broth samples were diluted in assay buffer to a final concentration of 50 mM succinate, pH 5.0 and 0.01% Tween-20. The substrate phosphoric acid swollen cellulose (PASC) was added at 0.5% (v/v). Following a 20 hour incubation at room temperature, reducing sugars were measured using the p-hydroxybenzoicacid hydrazide (PHBAH) method according to Lever, 1972, Anal. Biochem. 24: 273-279). The final concentration of reagents was 1.5% PHBAH, 2% NaOH, and 5% potassium sodium tartrate tetrahydrate. The reactions were heated at 100°C for 10 minutes, and sample absorbance measured at 405 nm. To determine reducing sugar release due to CBHI activity, control samples lacking PASC were subtracted from samples containing PASC. Final enzyme activity levels were compared to that obtained from culturing pCW13 (non-gap repaired) transformants. Relative absorbance values were indicative of enzyme activity, relating directly to the moles of reducing sugars released by CBHI degradation of PASC.

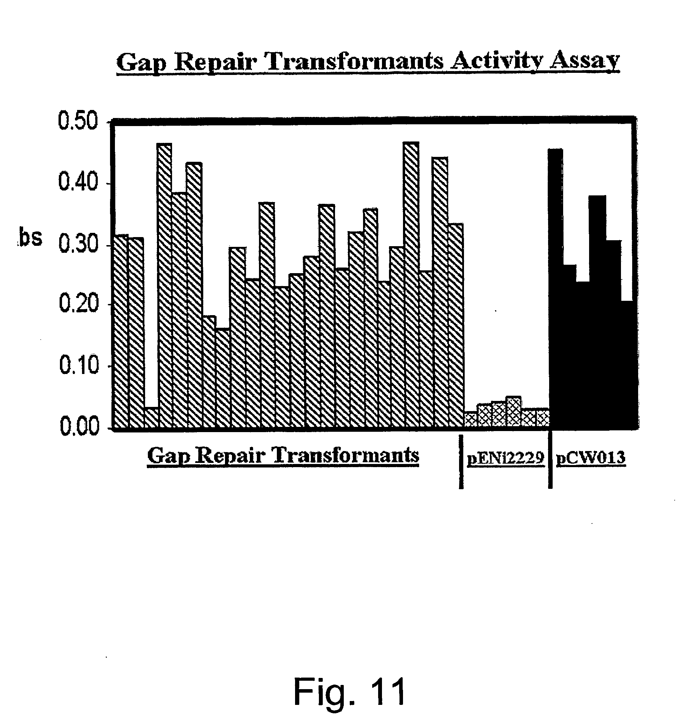

The assay results shown in Figure 11 demonstrated that repair of the gap was complete and that gap repaired plasmids expressed CBHI at levels comparable to transformants harboring the circular plasmid pCW013.

### Deposit of Biological Material

The following biological material has been deposited under the terms of the Budapest Treaty with the Agricultural Research Service Patent Culture Collection, Northern Regional Research Center, 1815 University Street, Peoria, Illinois, 61604, and given the following accession numbers:

| Deposit | Accession Number | Date of Deposit |
|---|---|---|
| *E. coli* pZL1 rdhA13 | NRRL B-30503 | July 27, 2001 |
| *E. coli* pZL1rdhB6 | NRRL B-30504 | July 27, 2001 |
| *E. coli* pZL1rdhD17 | NRRL B-30505 | July27, 2001 |
| *E. coli* pZL1rdhD10 | NRRL B-30506 | July 27, 2001 |

The strains have been deposited under conditions that assure that access to the culture will be available during the pendency of this patent application to one determined by the Commissioner of Patents and Trademarks to be entitled thereto under 37 C.F.R. §1.14 and 35 U.S.C. §122. The deposits represent a substantially pure cultures of the deposited strains. The deposits are available as required by foreign patent laws in countries wherein counterparts of the subject application, or its progeny are filed. However, it should be understood that the availability of a deposit does not constitute a license to practice the subject invention in derogation of patent rights granted by governmental action.

The invention described and claimed herein is not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

## Claims

1. A method for preparing variants of a nucleotide sequence in a filamentous fungal host, comprising:
(a) introducing into a population of filamentous fungal host cells:
(1) one or more circular plasmids comprising a DNA sequence and a plasmid replicator mediating autonomous replication, wherein the one or more circularized plasmids are linearized by digestion of the DNA sequence and removal of a portion of the DNA sequence; and
(2) a library of DNA fragments comprising one or more mutations of the DNA sequence, wherein the fragments comprise at least two regions, one or more first regions which are homologous to the 5' region or the 3' region of the gap in the linearized DNA sequence and/or plasmid sequence and one or more second regions which are homologous to the 5' region or the 3' region of the DNA fragments of the library;
wherein the linearized plasmids and the DNA fragments recombine by *in vivo* homologous recombination to produce a plurality of autonomously replicating plasmids comprising one or more variants of the DNA sequence; and
wherein the population of filamentous fungal host cells comprise a heterologous nucleic acid sequence encoding a recombination protein, which participates in the *in vivo* homologous recombination, selected from the group consisting of: (A) a nucleic acid sequence having at least 70% identity with SEQ ID NO:1, SEQ ID NO:3 or SEQ ID NO:5; and (B) a nucleic acid sequence which hybridizes under medium stringency conditions with (i) SEQ ID NO:1, SEQ ID NO:3 or SEQ ID NO:5, (ii) the cDNA sequence contained in SEQ ID NO:1, SEQ ID NO:3 or SEQ ID NO:5, or (iii) a complementary strand of (i) or (ii);
(b) cultivating the population of recombinant filamentous fungal cells in a medium suitable for growth; and
(c) screening the population of recombinant filamentous fungal cells for variants of the DNA sequence contained on one or more autonomously replicating circularized plasmids.

2. The method of claim 1, wherein more than one cycle of steps (a) to (c) is performed.

3. The method of claim 1, wherein two or more linearized plasmids are recombined by *in vivo* recombination with two or more homologous DNA fragments in the same cycle.

4. The method of claim 1, wherein the ratio between the linearized plasmids and the homologous DNA fragments are in the range from 20:1 to 1:50 mol plasmid:mol fragments with specific concentrations in the range of 1 pM to 10 M of the DNA.

5. The method of claim 1, wherein at least 2 of the DNA fragments have partially overlapping regions; preferably 2 to 50 of the DNA fragments have partially overlapping regions; and more preferably 2 to 10 of the DNA fragments have partially overlapping regions.

6. The method of claim 1, wherein the overlapping regions of the DNA fragments are in the range from 30 to 5000 bp; preferably the overlapping regions of the DNA fragments are in the range from 30 bp to 500 bp; and more preferably the overlapping regions of the DNA fragments are in the range from 30 bp to 100 bp.

7. The method of claim 1, wherein at least one cycle of step (a) to (c) is backcrossing with the initially used DNA fragments.

8. The method of claim 1, wherein the DNA fragments are prepared under conditions suitable for high, medium or low mutagenesis.

9. The method of claim 1, wherein the DNA sequence is selected from the group consisting of (a) a gene that encodes a polypeptide or an RNA; (b) a disrupted gene; (c) a partially deleted gene; (d) a regulatory control sequence; (e) a recombinantly manipulated version of a gene native or foreign to the filamentous fungal host cell; (f) a transposon; (g) a ribozyme; or (h) a portion of (a), (b), (c), (d), (e), (f) or (g).

10. The method of claim 9, wherein the polypeptide is an antibody, hormone, enzyme, receptor, reporter, selectable marker, or a protein with biological activity.

11. The method of claim 10, wherein the enzyme is an oxidoreductase, transferase, hydrolase, lyase, isomerase, or ligase.

12. The method of claim 9, wherein the regulatory control sequence is selected from the group consisting of a promoter, signal sequence, leader, polyadenylation sequence, propeptide sequence, consensus translational initiator sequence, signal peptide sequence, and transcription terminator.

13. The method of claim 9, wherein the disrupted gene is disrupted with a selectable marker gene selected from the group consisting of *amds* (acetamidase), *argB* (ornithine carbamoyltransferase), *bar* (phosphinothricin acetyltransferase), *hygB* (hygromycin phosphotransferase), *niaD* (nitrate reductase), *pyrG* (orotidine-5'-phosphate decarboxylase), sC (sulfate adenyltransferase), and *trpC* (anthranilate synthase); and equivalents thereof.

14. The method of claim 9, wherein the transposon is selected from the group consisting of P elements, LINES, SINES, Ty1, gypsy, Fot1, hAT, Restless, Guest, elements, tn10, Tad-1, Afut-1, and the retrotransposons MAGGY Ty3 and Ty5.

15. The method of claim 1, wherein the *in vivo* recombination occurs by homologous recombination.

16. The method of claim 1, wherein *the in vivo* recombination occurs by non-homologous recombination.

17. The method of claim 1, wherein the one or more regions of the DNA fragments that are homologous to the DNA sequence are a 5' region and/or a 3' region that flank (a) a gene that encodes a polypeptide or an RNA; (b) a gene disrupted with a third nucleic acid sequence; (c) a partially deleted gene; (d) a regulatory control sequence; (e) a recombinantly manipulated version of a gene native or foreign to the filamentous fungal host cell; (f) a transposon; (g) a ribozyme; or (h) a portion of (a), (b), (c), (d), (e), (f) or (g).

18. The method of claim 1, wherein the one or more regions of the DNA fragments that are homologous to the DNA sequence are a 5' region and/or a 3' region of (a) a gene that encodes a polypeptide or an RNA; (b) a gene disrupted with a third nucleic acid sequence; (c) a partially deleted gene; (d) a regulatory control sequence; (e) a recombinantly manipulated version of a gene native or foreign to the filamentous fungal host cell; (f) a transposon; (g) a ribozyme; or (h) a portion of (a), (b), (c), (d), (e), (f) or (g).

19. The method of claim 1, wherein the one or more regions of the DNA fragments that are homologous to the DNA sequence are part of a gene native or foreign to the filamentous fungal host cell.

20. The method of claim 9, wherein the hormone or protein with biological activity are selected from the group consisting of insulin, ACTH, glucagon, somatostatin, somatotropin, thymosin, parathyroid hormone, pigmentary hormones, somatomedin, erythropoietin, luteinizing hormone, chorionic gonadotropin, hypothalamic releasing factors, antidiuretic hormones, thyroid stimulating hormone, relaxin, interferon, thrombopoietin, and prolactin.

21. The method of claim 1, wherein at least one of the DNA sequences is a wild-type DNA sequence.

22. The method of claim 1, wherein the regions homologous to the DNA sequence or vector sequence are at least 60% homologous; preferably at least 70% homologous; more preferably at least 80% homologous; and most preferably at least 90% homologous.

23. The method of claim 1, wherein the filamentous fungal cell is an *Acremonium, Aspergillus, Aureobasidium, Cryptococcus, Filibasidium, Fusarium, Gibberella, Humicola, Magnaporthe, Mucor, Myceliophthora, Myrothecium, Neocallimastix, Neurospora, Paecilomyces, Penicillium, Piromyces, Schizophyllum, Talaromyces, Thermoascus, Thielavia, Tolypocladium,* or *Trichoderma* strain; preferably the filamentous fungal cell is an *Aspergillus* strain; more preferably the filamentous fungal cell is an *Aspergillus oryzae* or *Aspergillus niger.*

24. The method of claim 1, wherein the replicator sequence is AMA1 or ANS1.

25. The method of claim 1, wherein the recombination protein has at least 70% identity with SEQ ID NO: 2, SEQ ID NO:4 or SEQ ID NO:6; preferably at least 75% identity; more preferably at least 80% identity; even more preferably at least 85% identity; still more preferably at least 90% identity; yet more preferably at least 95% identity with SEQ ID NO: 2, SEQ ID NO:4 or SEQ ID NO:6.

26. The method of claim 1, wherein the recombination protein comprises the amino acid sequence of SEQ ID NO:2, SEQ ID NO:4 or SEQ ID NO:6.

27. The method of claim 1, wherein the recombination protein consists of the amino acid sequence of SEQ ID NO:2, SEQ ID NO:4 or SEQ ID NO:6; or a fragment thereof which has recombination activity.

28. The method of claim 27, wherein SEQ ID NO:2 is encoded by SEQ ID NO:1, SEQ ID NO:4 is encoded by SEQ ID NO:3, and SEQ ID NO:6 is encoded by SEQ ID NO:5.

29. The method of claim 1, wherein the nucleic acid sequence encoding the recombination protein has at least 70% identity with SEQ ID NO:1, SEQ ID NO:3 or SEQ ID NO:5; preferably at least 75% identity; more preferably at least 80% identity; still more preferably at least 85% identity; yet more preferably at least 90% identity; and most preferably at least 95% identity with SEQ ID NO:1, SEQ ID NO:3 or SEQ ID NO:5.

30. The method of claim 1, wherein the nucleic acid sequence encoding the recombination protein hybridizes under medium stringency, medium-high stringency, or high stringency conditions with (i) SEQ ID NO:1, SEQ ID NO:3 or SEQ ID NO:5, (ii) the cDNA sequence contained in SEQ ID NO:1, SEQ ID NO:3 or SEQ ID NO:5, or (iii) a complementary strand of (i) or (ii).

31. The method of claim 1, wherein the nucleic acid sequence is contained in plasmid pZL1rdhA13 which is contained in *Escherichia coli* NRRL B-30503; plasmid pZL1rdhB6 which is contained in *Escherichia coli* NRRL B-30504; or plasmid pZL1 rdhD17 which is contained in *Escherichia coli* NRRL B-30505 and plasmid pZL1rdhD10 which is contained in *Escherichia coli* NRRL B-30506.

32. The method of claim 1, further comprising isolating from the population of recombinant filamentous fungal cells an autonomously replicating plasmid comprising a variant DNA sequence.

33. The method of claim 32, wherein the variant DNA sequence encodes a product with an improved property of interest.

34. The method of claim 33, wherein the improved characteristic is selected from the group consisting of thermostability, thermolability, protease-resistance, pH optimum, pH stability, altered substrate specificity, and increased promoter activity.

## Patentansprüche

1. Verfahren zum Herstellen von Varianten einer Nukleotidsequenz in einem filamentöser-Pilz-Wirt, umfassend:
(a) Einführen in eine Population von filamentöser-Pilz-Wirtszellen:
(1) ein oder mehrere zirkuläre Plasmide, umfassend eine DNA-Sequenz und einen Plasmidreplikator, der autonome Replikation vermittelt, wobei die ein oder mehreren zirkularisierten Plasmide durch Verdau der DNA-Sequenz und Entfernen eines Teils der DNA-Sequenz linearisiert werden; und
(2) eine Bibliothek von DNA-Fragmenten, umfassend ein oder mehrere Mutationen der DNA-Sequenz, wobei die Fragmente mindestens zwei Regionen umfassen, ein oder mehrere erste Regionen, die zu der 5'-Region oder der 3'-Region der Lücke in der linearisierten DNA-Sequenz und/oder Plasmidsequenz homolog sind, und ein oder mehrere zweite Regionen, die zu der 5'-Region oder der 3'-Region der DNA-Fragmente der Bibliothek homolog sind;
wobei die linearisierten Plasmide und die DNA-Fragmente durch homologe *in vivo-*Rekombination rekombinieren, um eine Vielzahl von autonom replizierenden Plasmiden zu erzeugen, die ein oder mehrere Varianten der DNA-Sequenz umfassen; und
wobei die Population von filamentöser-Pilz-Wirtszellen eine heterologe Nukleinsäuresequenz umfassen, die ein Rekombinationsprotein kodiert, das bei der homologen *in vivo*-Rekombination mitwirkt, ausgewählt aus der Gruppe, bestehend aus: (A) einer Nukleinsäuresequenz, die mindestens 70% Identität mit SEQ ID NO: 1, SEQ ID NO: 3 oder SEQ ID NO: 5 aufweist; und (B) einer Nukleinsäuresequenz, die unter Bedingungen mittlerer Stringenz hybridisiert mit (i) SEQ ID NO: 1, SEQ ID NO: 3 oder SEQ ID NO: 5, (ii) der in SEQ ID NO: 1, SEQ ID NO: 3 oder SEQ ID NO: 5 enthaltenen cDNA-Sequenz, oder (iii) einem komplementären Strang von (i) oder (ii);
(b) Kultivieren der Population rekombinanter filamentöser-Pilz-Zellen in einem für Wachstum geeigneten Medium; und
(c) Durchmustern der Population rekombinanter filamentöser-Pilz-Zellen auf Varianten der DNA-Sequenz, enthalten auf ein oder mehreren autonom replizierenden zirkularisierten Plasmiden.

2. Verfahren nach Anspruch 1, wobei mehr als ein Zyklus von Schritten (a) bis (c) durchgeführt wird.

3. Verfahren nach Anspruch 1, wobei zwei oder mehrere linearisierte Plasmide durch *in vivo*-Rekombination mit zwei oder mehreren homologen DNA-Fragmenten in dem gleichen Zyklus rekombiniert werden.

4. Verfahren nach Anspruch 1, wobei das Verhältnis zwischen den linearisierten Plasmiden und den homologen DNA-Fragmenten in dem Bereich von 20:1 bis 1:50 Mol Plasmid : Mol Fragmente mit spezifischen Konzentrationen in dem Bereich von 1 pM bis 10 M der DNA sind.

5. Verfahren nach Anspruch 1, wobei mindestens 2 der DNA-Fragmente teilüberlappende Regionen aufweisen; vorzugsweise 2 bis 50 der DNA-Fragmente teilüberlappende Regionen aufweisen; und stärker bevorzugt 2 bis 10 der DNA-Fragmente teilüberlappende Regionen aufweisen.

6. Verfahren nach Anspruch 1, wobei die überlappenden Regionen der DNA-Fragmente in dem Bereich von 30 bis 5000 bp sind; die überlappenden Regionen der DNA-Fragmente vorzugsweise in dem Bereich von 30 bp bis 500 bp sind; und die überlappenden Bereiche der DNA-Fragmente stärker bevorzugt in dem Bereich von 30 bp bis 100 bp sind.

7. Verfahren nach Anspruch 1, wobei mindestens ein Zyklus von Schritt (a) bis (c) Rückkreuzen mit den ursprünglich verwendeten DNA-Fragmenten ist.

8. Verfahren nach Anspruch 1, wobei die DNA-Fragmente unter für hohe, mittlere oder geringe Mutagenese geeigneten Bedingungen hergestellt werden.

9. Verfahren nach Anspruch 1, wobei die DNA-Sequenz ausgewählt wird aus der Gruppe, bestehend aus (a) einem Gen, das ein Polypeptid oder eine RNA kodiert; (b) einem unterbrochenen Gen; (c) einem teilweise deletierten Gen; (d) einer regulatorischen Kontrollsequenz; (e) einer rekombinant manipulierten Version eines Gens, das gegenüber der filamentöser-Pilz-Wirtszelle nativ oder fremd ist; (f) einem Transposon; (g) einem Ribozym; oder (h) einem Teil von (a), (b), (c), (d), (e), (f) oder (g).

10. Verfahren nach Anspruch 9, wobei das Polypeptid ein Antikörper, Hormon, Enzym, Rezeptor, Reporter, selektierbarer Marker oder ein Protein mit biologischer Aktivität ist.

11. Verfahren nach Anspruch 10, wobei das Enzym eine Oxidoreduktase, Transferase, Hydrolase, Lyase, Isomerase oder Ligase ist.

12. Verfahren nach Anspruch 9, wobei die regulatorische Kontrollsequenz ausgewählt ist aus der Gruppe, bestehend aus einem Promotor, einer Signalsequenz, einem Leader, einer Polyadenylierungssequenz, einer Propeptidsequenz, einer Translationsinitiatorkonsensussequenz, einer Signalpeptidsequenz und einem Transkriptionsterminator.

13. Verfahren nach Anspruch 9, wobei das unterbrochene Gen mit einem selektierbaren Markergen unterbrochen wird, ausgewählt aus der Gruppe, bestehend aus *amds* (Acetamidase), *argB* (Ornithincarbamoyltransferase), *bar* (Phosphinothricinacetyltransferase), *hygB* (Hygromycinphosphotransferase), *niaD* (Nitratreduktase), *pyrG* (Orotidin-5'-Phosphat-Decarboxylase), *sC* (Sulfatadenyltransferase), und *trpC* (Anthranilatsynthase); und Äquivalenten davon.

14. Verfahren nach Anspruch 9, wobei das Transposon ausgewählt wird aus der Gruppe bestehend aus P-Elementen, LINES, SINES, Ty1, gypsy, Fot1, hAT, Restless, Guest, Elementen, tn10, Tau-1, Afut-1 und den Retrotransposons MAGGY Ty3 und Ty5.

15. Verfahren nach Anspruch 1, wobei die *in vivo*-Rekombination durch homologe Rekombination erfolgt.

16. Verfahren nach Anspruch 1, wobei die *in vivo*-Rekombination durch nicht homologe Rekombination erfolgt.

17. Verfahren nach Anspruch 1, wobei die ein oder mehreren Regionen der DNA-Fragmente, die homolog zu der DNA-Sequenz sind, eine 5'-Region und/oder eine 3'-Region sind, die flankieren (a) ein Gen, das ein Polypeptid oder eine RNA kodiert; (b) ein Gen, das mit einer dritten Nukleinsäuresequenz unterbrochen ist; (c) ein teilweise deletiertes Gen; (d) eine regulatorische Kontrollsequenz; (e) eine rekombinant manipulierte Version eines Gens, das gegenüber der filamentöser-Pilz-Wirtszelle nativ oder fremd ist; (f) ein Transposon; (g) ein Ribozym; oder (h) einen Teil von (a), (b), (c), (d), (e), (f) oder (g).

18. Verfahren nach Anspruch 1, wobei die ein oder mehreren Regionen der DNA-Fragmente, die homolog zu der DNA-Sequenz sind, eine 5'-Region und/oder eine 3'-Region sind von (a) einem Gen, das ein Polypeptid oder eine RNA kodiert; (b) einem Gen, das mit einer dritten Nukleinsäuresequenz unterbrochen ist; (c) einem teilweise deletierten Gen; (d) einer regulatorischen Kontrollsequenz; (e) einer rekombinanten manipulierten Version eines Gens, das gegenüber der filamentöser-Pilz-Wirtszelle nativ oder fremd ist; (f) einem Transposon; (g) einem Ribozym; oder (h) einem Teil von (a), (b), (c), (d), (e), (f) oder (g).

19. Verfahren nach Anspruch 1, wobei die ein oder mehreren Regionen der DNA-Fragmente, die homolog zu der DNA-Sequenz sind, ein Teil eines Gens sind, das gegenüber der filamentöser-Pilz-Wirtszelle nativ oder fremd ist.

20. Verfahren nach Anspruch 9, wobei das Hormon oder Protein mit biologischer Aktivität ausgewählt werden aus der Gruppe, bestehend aus Insulin, ACTH, Glukagon, Somatostatin, Somatotropin, Thymosin, Nebenschilddrüsenhormon, Pigmenthormonen, Somatomedin, Erythropoietin, luteinisierendem Hormon, Choriongonadotropin, hypothalamischen Freisetzungsfaktoren, antidiuretischen Hormonen, Schilddrüsenstimulierendem Hormon, Relaxin, Interferon, Thrombopoietin und Prolaktin.

21. Verfahren nach Anspruch 1, wobei mindestens eine der DNA-Sequenzen eine Wildtyp-DNA-Sequenz ist.

22. Verfahren nach Anspruch 1, wobei die zu der DNA-Sequenz oder Vektor-Sequenz homologen Regionen mindestens 60% homolog; vorzugsweise mindestens 70% homolog; stärker bevorzugt mindestens 80% homolog; und am stärksten bevorzugt mindestens 90% homolog sind.

23. Verfahren nach Anspruch 1, wobei die filamentöser-Pilz-Zelle ein *Acremonium-, Aspergillus-, Aureobasidium-, Cryptococcus-, Filibasidium-, Fusarium-, Gibberella-, Humicola-, Magnaporthe-, Mucor-, Myceliophthora-, Myrothecium-, Neocallimastix-, Neurospora-, Paecilomyces-, Penicillium-, Piromyces-, Schizophyllum-, Talaromyces-, Thermoascus-, Thielavia-, Tolypocladium-* oder *Trichoderma-Stamm* ist; die filamentöser-Pilz-Zelle vorzugsweise ein *Aspergillus-Stamm* ist; die filamentöser-Pilz-Zelle stärker bevorzugt ein *Aspergillus Oryzae* oder *Aspergillus Niger* ist.

24. Verfahren nach Anspruch 1, wobei die Replikatorsequenz AMA1 oder ANS1 ist.

25. Verfahren nach Anspruch 1, wobei das Rekombinationsprotein mindestens 70% Identität mit SEQ ID NO: 2, SEQ ID NO: 4 oder SEQ ID NO: 6; vorzugsweise mindestens 75% Identität; stärker bevorzugt mindestens 80% Identität; noch stärker bevorzugt mindestens 85% Identität; noch stärker bevorzugt mindestens 90% Identität; noch stärker bevorzugt mindestens 95% Identität mit SEQ ID NO: 2, SEQ ID NO: 4 oder SEQ ID NO: 6 aufweist.

26. Verfahren nach Anspruch 1, wobei das Rekombinationsprotein die Aminosäuresequenz von SEQ ID NO: 2, SEQ ID NO: 4 oder SEQ ID NO: 6 umfasst.

27. Verfahren nach Anspruch 1, wobei das Rekombinationsprotein aus der Aminosäuresequenz von SEQ ID NO: 2, SEQ ID NO: 4 oder SEQ ID NO: 6; oder einem Fragment davon, das Rekombinationsaktivität aufweist, besteht.

28. Verfahren nach Anspruch 27, wobei SEQ ID NO: 2 von SEQ ID NO: 1 kodiert wird, SEQ ID NO: 4 von SEQ ID NO: 3 kodiert wird, und SEQ ID NO: 6 von SEQ ID NO: 5 kodiert wird.

29. Verfahren nach Anspruch 1, wobei die das Rekombinationsprotein kodierende Nukleinsäuresequenz mindestens 70% Identität mit SEQ ID NO: 1, SEQ ID NO: 3 oder SEQ ID NO: 5; vorzugsweise mindestens 75% Identität; stärker bevorzugt mindestens 80% Identität; noch stärker bevorzugt mindestens 85% Identität; noch stärker bevorzugt mindestens 90% Identität; und am stärksten bevorzugt mindestens 95% Identität mit SEQ ID NO: 1, SEQ ID NO: 3 oder SEQ ID NO: 5 aufweist.

30. Verfahren nach Anspruch 1, wobei die das Rekombinationsprotein kodierende Nukleinsäuresequenz unter Bedingungen mittlerer Stringenz, mittelhoher Stringenz oder hoher Stringenz hybridisiert mit (i) SEQ ID NO: 1, SEQ ID NO: 3 oder SEQ ID NO: 5, (ii) der cDNA-Sequenz, enthalten in SEQ ID NO: 1, SEQ ID NO: 3 oder SEQ ID NO: 5, oder (iii) einem komplementären Strang von (i) oder (ii).

31. Verfahren nach Anspruch 1, wobei die Nukleinsäuresequenz enthalten ist in Plasmid pZL1rdhA13, das in *Escherichia coli* NRRL B-30503 enthalten ist; Plasmid pZL1rdhB6, das in *Escherichia coli* NRRL B-30504 enthalten ist; oder Plasmid pZL1rdhD17, das in *Escherichia coli* NRRL B-30505 enthalten ist und Plasmid pZL1rdhD10, das in *Escherichia coli* NRRL B-30506 enthalten ist.

32. Verfahren nach Anspruch 1, weiterhin umfassend Isolieren eines autonom replizierenden Plasmids, umfassend eine DNA-Sequenzvariante, aus der Population von rekombinanten filamentöser-Pilz-Zellen umfasst.

33. Verfahren nach Anspruch 32, wobei die DNA-Sequenzvariante ein Produkt mit einer verbesserten Eigenschaft von Interesse kodiert.

34. Verfahren nach Anspruch 33, wobei das verbesserte Charakteristikum ausgewählt ist aus der Gruppe, bestehend aus Thermostabilität, Thermolabilität, Proteaseresistenz, pH-Optimum, pH-Stabilität, veränderter Substratspezifität und erhöhter Promoteraktivität.

## Revendications

1. Procédé pour préparer des variants d'une séquence de nucléotides dans un hôte fongique filamenteux, comprenant :
(a) l'introduction, dans une population de cellules hôtes fongiques filamenteuses :
(1) d'un ou plusieurs plasmides circulaires comprenant une séquence d'ADN et un réplicateur plasmidique capable de médier la réplication autonome, où le ou les plasmides circularisés sont linéarisés par digestion de la séquence d'ADN et élimination d'une portion de la séquence d'ADN ; et
(2) d'une bibliothèque de fragments d'ADN comprenant une ou plusieurs mutations de la séquence d'ADN, où les fragments comprennent au moins deux régions, une ou plusieurs premières régions qui sont homologues à la région 5' ou à la région 3' de la brèche dans la séquence d'ADN linéarisés et/ou à la séquence plasmidique et une ou plusieurs deuxièmes régions qui sont homologues à la région 5' ou à la région 3' des fragments d'ADN de la bibliothèque ;
où les plasmides linéarisés et les fragments d'ADN se recombinent par recombinaison homologue *in vivo* pour produire une pluralité de plasmides à réplication autonome comprenant un ou plusieurs variants de la séquence d'ADN ; et
où la population de cellules hôtes fongiques filamenteuses comprend une séquence d'acide nucléique hétérologue codant une protéine de recombinaison, qui participe à la recombinaison homologue *in vivo,* choisie dans le groupe constitué par : (A) une séquence d'acide nucléique ayant au moins 70 % d'identité avec SEQ ID NO : 1, SEQ ID NO : 3 ou SEQ ID NO : 5 ; et (B) une séquence d'acide nucléique qui s'hybride, dans des conditions de stringence moyenne, avec (i) SEQ ID NO : 1, SEQ ID NO : 3 ou SEQ ID NO : 5, (ii) la séquence d'ADNc contenue dans SEQ ID NO : 1, SEQ ID NO : 3 ou SEQ ID NO : 5, ou (iii) un brin complémentaire de (i) ou (ii) ;
(b) la culture de la population de cellules fongiques filamenteuses recombinées dans un milieu adapté pour la croissance ; et
(c) le criblage de la population de cellules fongiques filamenteuses recombinées pour des variants de la séquence d'ADN contenus dans un ou plusieurs plasmides circularisés à réplication autonome.

2. Procédé selon la revendication 1, dans lequel plusieurs cycles des étapes (a) à (c) sont effectués.

3. Procédé selon la revendication 1, dans lequel deux ou plus plasmides linéarisés sont recombinés par recombinaison *in vivo* avec deux ou plus fragments d'ADN homologues dans le même cycle.

4. Procédé selon la revendication 1, dans lequel le rapport entre les plasmides linéarisés et les fragments d'ADN homologues est situé dans la plage allant de 20/1 à 1/50 moles de plasmide/moles de fragments avec des concentrations spécifiques situées dans la plage allant de 1 pM à 10 M de l'ADN.

5. Procédé selon la revendication 1, dans lequel au moins 2 des fragments d'ADN ont des régions se chevauchant partiellement ; de préférence 2 à 50 des fragments d'ADN ont des régions se chevauchant partiellement ; et de manière encore plus préférée 2 à 10 des fragments d'ADN ont des régions se chevauchant partiellement.

6. Procédé selon la revendication 1, dans lequel les régions chevauchantes des fragments d'ADN sont situées dans la plage allant de 30 à 5 000 pb ; de préférence les régions chevauchantes des fragments d'ADN sont situées dans la plage allant de 30 pb à 500 pb ; et de manière encore plus préférée les régions chevauchantes des fragments d'ADN sont situées dans la plage allant de 30 pb à 100 pb.

7. Procédé selon la revendication 1, dans lequel au moins un cycle de l'étape (a) à l'étape (c) est réalisé en rétrocroisement avec les fragments d'ADN utilisés initialement.

8. Procédé selon la revendication 1, dans lequel les fragments d'ADN sont préparés dans des conditions adaptées pour une mutagenèse forte, moyenne ou faible.

9. Procédé selon la revendication 1, dans lequel la séquence d'ADN est choisie dans le groupe constitué par (a) un gène qui code un polypeptide ou un ARN ; (b) un gène interrompu ; (c) un gène ayant subi une délétion partielle ; (d) une séquence de contrôle de la régulation ; (e) une version manipulée par recombinaison d'un gène natif ou étranger à la cellule hôte fongique filamenteuse ; (f) un transposon ; (g) un ribozyme ; ou (h) une portion de (a), (b), (c), (d), (e), (f) ou (g).

10. Procédé selon la revendication 9, dans lequel le polypeptide est un anticorps, une hormone, une enzyme, un récepteur, un rapporteur, un marqueur de sélection ou une protéine ayant une activité biologique.

11. Procédé selon la revendication 10, dans lequel l'enzyme est une oxydoréductase, une transférase, une hydrolase, une lyase, une isomérase ou une ligase.

12. Procédé selon la revendication 9, dans lequel la séquence de contrôle de la régulation est choisie dans le groupe constitué par un promoteur, une séquence signal, une séquence de tête, une séquence de polyadénylation, une séquence propeptidique, une séquence consensus initiatrice de la traduction, une séquence de peptide signal et un terminateur de transcription.

13. Procédé selon la revendication 9, dans lequel le gène interrompu est interrompu par un gène marqueur de sélection choisi dans le groupe constitué par *amds* (acétamidase), *argB* (ornithine carbamoyltransférase), *bar* (phosphinothricine acétyltransférase), *hygB* (hygromycine phosphotransférase), *niaD* (nitrate réductase), *pyrG* (oritidine-5'-phosphate décarboxylase), sC (sulfate adényltransférase) et *trpC* (anthranilate synthase) et leurs équivalents.

14. Procédé selon la revendication 9, dans lequel le transposon est choisi dans le groupe constitué par les éléments P, LINES, SINES, Ty1, gypsy, Fot1, hAT, Restless, les éléments Guest, tn10, Tad-1, Afut-1 et les rétrotransposons MAGGY Ty3 et Ty5.

15. Procédé selon la revendication 1, dans lequel la recombinaison *in vivo* se produit par recombinaison homologue.

16. Procédé selon la revendication 1, dans lequel la recombinaison *in vivo* se produit par recombinaison non homologue.

17. Procédé selon la revendication 1, dans lequel la ou les régions des fragments d'ADN qui sont homologues à la séquence d'ADN sont une région 5' et/ou une région 3' qui flanquent (a) un gène qui code un polypeptide ou un ARN ; (b) un gène interrompu par une troisième séquence d'acide nucléique ; (c) un gène ayant subi une délétion partielle ; (d) une séquence de contrôle de la régulation ; (e) une version manipulée par recombinaison d'un gène natif ou étranger à la cellule hôte fongique filamenteuse ; (f) un transposon ; (g) un ribozyme ; ou (h) une portion de (a), (b), (c), (d), (e), (f) ou (g).

18. Procédé selon la revendication 1, dans lequel la ou les régions des fragments d'ADN qui sont homologues à la séquence d'ADN sont une région 5' et/ou une région 3' de (a) un gène qui code un polypeptide ou un ARN ; (b) un gène interrompu par une troisième séquence d'acide nucléique ; (c) un gène ayant subi une délétion partielle ; (d) une séquence de contrôle de la régulation ; (e) une version manipulée par recombinaison d'un gène natif ou étranger à la cellule hôte fongique filamenteuse ; (f) un transposon ; (g) un ribozyme ; ou (h) une portion de (a), (b), (c), (d), (e), (f) ou (g).

19. Procédé selon la revendication 1, dans lequel la ou les régions des fragments d'ADN qui sont homologues à la séquence d'ADN font partie d'un gène natif ou étranger à la cellule hôte fongique filamenteuse.

20. Procédé selon la revendication 9, dans lequel l'hormone ou la protéine ayant une activité biologique est choisie dans le groupe constitué par l'insuline, l'ACTH, le glucagon, la somatostatine, la somatotrophine, la thymosine, l'hormone parathyroïdienne, les hormones pigmentaires, la somatomédine, l'érythropoïétine, l'hormone lutéinisante, la gonadotrophine chorionique, les facteurs de libération hypothalamiques, les hormones antidiurétiques, la thyréostimuline, la relaxine, l'interféron, la thrombopoïétine et la prolactine.

21. Procédé selon la revendication 1, dans lequel au moins l'une des séquences d'ADN est une séquence d'ADN de type sauvage.

22. Procédé selon la revendication 1, dans lequel les régions homologues à la séquence d'ADN ou à la séquence du vecteur sont homologues à au moins 60 % ; de préférence homologues à au moins 70 % ; de manière préférée homologues à au moins 80 % ; et de manière encore plus préférée homologues à au moins 90 %.

23. Procédé selon la revendication 1, dans lequel la cellule fongique filamenteuse est une souche *d'Acremonium, Aspergillus, Aureobasidium, Cryotococcus, Filibasidium, Fusarium, Gibberella, Humicola, Magnaporthe, Mucor, Myceliophthora, Myrothecium, Neocallimastix, Neurospora, Paecilomyces, Penicillium, Piromyces, Schizophyllum, Talaromyces, Thermoascus, Thielavia, Tolypocladium* ou *Trichoderma* ; de préférence la cellule fongique filamenteuse est une souche *d'Aspergillus* ; de manière encore plus préférée la cellule fongique filamenteuse est *Aspergillus oryzae* ou *Aspergillus niger.*

24. Procédé selon la revendication 1, dans lequel la séquence du réplicateur est AMA1 ou ANS1.

25. Procédé selon la revendication 1, dans lequel la protéine de recombinaison a au moins 70 % d'identité avec SEQ ID NO : 2, SEQ ID NO : 4 ou SEQ ID NO : 6 ; de préférence au moins 75 % d'identité ; de préférence encore au moins 80 % d'identité ; de manière encore plus préférée au moins 85 % d'identité ; de manière encore plus préférée au moins 90 % d'identité ; et de manière encore plus préférée au moins 95 % d'identité avec SEQ ID NO : 2, SEQ ID NO : 4 ou SEQ ID NO : 6.

26. Procédé selon la revendication 1, dans lequel la protéine de recombinaison comprend la séquence d'acides aminés de SEQ ID NO : 2, SEQ ID NO : 4 ou SEQ ID NO : 6.

27. Procédé selon la revendication 1, dans lequel la protéine de recombinaison est constituée de la séquence d'acides aminés de SEQ ID NO : 2, SEQ ID NO : 4 ou SEQ ID NO : 6 ; ou d'un fragment de celles-ci qui a une activité de recombinaison.

28. Procédé selon la revendication 27, dans lequel SEQ ID NO : 2 est codée par SEQ ID NO : 1, SEQ ID NO : 4 est codée par SEQ ID NO : 3, et SEQ ID NO : 6 est codée par SEQ ID NO : 5.

29. Procédé selon la revendication 1, dans lequel la séquence d'acide nucléique codant la protéine de recombinaison a au moins 70 % d'identité avec SEQ ID NO : 1, SEQ ID NO : 3 ou SEQ ID NO : 5 ; de préférence au moins 75 % d'identité ; de préférence encore au moins 80 % d'identité ; de manière encore plus préférée au moins 85 % d'identité ; de manière encore plus préférée au moins 90 % d'identité ; et de manière encore plus préférée au moins 95 % d'identité avec SEQ ID NO : 1, SEQ ID NO : 3 ou SEQ ID NO : 5.

30. Procédé selon la revendication 1, dans lequel la séquence d'acide nucléique codant la protéine de recombinaison s'hybride dans des conditions de stringence moyenne, de stringence moyenne-forte ou de stringence forte avec (i) SEQ ID NO : 1, SEQ ID NO : 3 ou SEQ ID NO : 5, (ii) la séquence d'ADNc contenue dans SEQ ID NO : 1, SEQ ID NO : 3 ou SEQ ID NO : 5, ou (iii) un brin complémentaire de (i) ou (ii).

31. Procédé selon la revendication 1, dans lequel la séquence d'acide nucléique est contenue dans le plasmide pZL 1rdhA13 qui est contenu dans *Escherichia coli* NRRL B-30503 ; le plasmide pZL 1rdhB6 qui est contenu dans *Escherichia coli* NRRL B-30504 ; ou le plasmide pZL rdhD17 qui est contenu dans *Escherichia coli* NRRL B-30505 et le plasmide pZL 1rdhD10 qui est contenu dans *Escherichia coli* NRRL B-30506.

32. Procédé selon la revendication 1, comprenant en outre l'isolation, à partir de la population de cellules fongiques filamenteuses recombinées, d'un plasmide à réplication autonome comprenant une séquence d'ADN variante.

33. Procédé selon la revendication 32, dans lequel la séquence d'ADN variante code un produit ayant une propriété d'intérêt qui est améliorée.

34. Procédé selon la revendication 33, dans lequel la caractéristique améliorée est choisie dans le groupe constitué par la thermostabilité, la thermolabilité, la résistance aux protéases, le pH optimum, la stabilité au pH, une spécificité de substrat altérée et une activité promotrice accrue.
